# EUROPEAN PATENT SPECIFICATION

(11) **EP 4 441 057 B1**
(45) Date of publication and mention of the grant of the patent: **12.08.2026**
(21) Application number: 22823380.5
(22) Date of filing: 29.11.2022
(51) Int. Cl.: C07F 7/08, C07F 7/10, C08C 19/22, C08C 19/24, C08C 19/42, C08C 19/44, C08F 236/04, C08F 236/10, C07D 207/08, C07D 241/04, C07D 265/30, C07C 211/02, B60C 1/00, C08K 3/36, C08F 236/06, C08C 19/25

(54) **AMINE-CONTAINING VINYLDISILOXANES IN THE MANUFACTURE OF ELASTOMERIC POLYMERS**
AMINHALTIGE VINYLDISILOXANE IN DER HERSTELLUNG VON ELASTOMERPOLYMEREN
VINYLDISILOXANES CONTENANT DES AMINES DANS LA FABRICATION DE POLYMÈRES ÉLASTOMÈRES

(30) Priority: 30.11.2021 EP 21211476
(43) Date of publication of application: 09.10.2024
(73) Proprietor: Synthos Dwory 7 spolka z ograniczona odpowiedzialnoscia, 32-600 Oswiecim (PL)
(72) Inventor: RÖSSLE, Michael, 06217 Merseburg (DE); THIELE, Sven, 06120 Halle (DE)
(74) Representative: Hoffmann Eitle
(86) International application number: PCT/EP2022/083651
(87) International publication number: WO 2023/099462

(56) References cited:
- WO-A1-2015/055252
- WO-A1-2017/216344
- CHO HYEON MO ET AL: "Generation of (dimethylaminomethyl)phenylvinylsilanone from 1,3-dihydroxy-1,3-disiloxane", JOURNAL OF ORGANOMETALLIC CHEMISTRY, vol. 706-707, 1 June 2012 (2012-06-01), AMSTERDAM, NL, pages 1 - 3, XP055919921, ISSN: 0022-328X, DOI: 10.1016/j.jorganchem.2012.02.002
- JUNG HYO-JIN ET AL: "The Potentiometric Studies on the Effects of Various Functional Groups in Disiloxane as an Anion-Selective Ionophore", BULLETIN OF THE KOREAN CHEMICAL SOCIETY, vol. 26, no. 1, 20 January 2005 (2005-01-20), KR, pages 57 - 62, XP055919932, ISSN: 0253-2964
- YUKIO NAGASAKI ET AL: "Reactivity of Lithium alkylamide toward Vinylsilane Derivatives", BULLETIN OF THE CHEMICAL SOCIETY OF JAPAN, CHEMICAL SOCIETY OF JAPAN,NIPPON KAGAKUKAI, JP, vol. 65, no. 4, 1 April 1992 (1992-04-01), pages 949 - 953, XP007916066, ISSN: 0009-2673, DOI: 10.1246/BCSJ.65.949

## Description

### FIELD OF THE INVENTION

The present invention relates to novel amine-containing vinyldisiloxane compounds which are useful as modifying monomers in the manufacture of elastomeric polymers that can be used in rubber articles such as tires. The present invention further relates to a process for preparing an elastomeric polymer in the presence of vinyldisiloxane compounds, an elastomeric polymer obtainable by this process, non-vulcanized and vulcanized polymer compositions comprising the elastomeric polymer and articles comprising one or more components formed from the vulcanized polymer composition.

### BACKGROUND OF THE INVENTION

Amine-containing vinylsilanes are used in the rubber field as modifying monomers in the polymerization of diene monomers such as butadiene, optionally together with aromatic vinyl monomers such as styrene, to produce rubber which can favorably be used in the manufacture of fuel-efficient rubbers.

One approach for obtaining fuel-efficient tires lies in the production of tire formulations having reduced hysteresis loss. The hysteresis loss of a cross-linked elastomeric polymer composition is related to its tan δ value at 60°C (see ISO 4664-1:2005; Rubber, Vulcanized or thermoplastic; Determination of dynamic properties - part 1: General guidance). In general, vulcanized elastomeric polymer compositions having relatively low tan δ values at 60°C are preferred for having lower hysteresis loss. In the final tire product, this translates into lower rolling resistance and better fuel economy. In contrast, lower tan δ values at 0°C correspond to a deteriorated wet grip of the tire product, and it is generally accepted that a lower rolling resistance tire can be obtained at the expense of deteriorated wet grip.

For example, if, in a random solution styrene-butadiene rubber (random SSBR), the polystyrene unit concentration is reduced with respect to the total polybutadiene unit concentration, the SSBR glass transition temperature is reduced and, as a result, both tan δ at 60°C and tan δ at 0°C are reduced, corresponding to low rolling resistance and deteriorated wet grip performance of the tire. Accordingly, when assessing the rubber vulcanizate performance correctly, both tan δ at 60°C and tan δ at 0°C should be monitored along with the tire heat build-up.

US 8,304,488 relates to a conjugated diene polymer obtained by polymerizing a conjugated diene monomer and an aminosilane-containing monomer which can be used in rubber articles such as tires.

DE 10 2011 109 823 describes a conjugated diene-based polymer comprising a diene-based monomer unit, a vinylsilane monomer unit and a vinylamine monomer unit for use in a rubber composition for tires. WO 2017/216344 A1 discloses compounds used for preparing elastomeric polymers.

The present invention aims the provision of modifying monomers which are useful in the manufacture of elastomeric polymers, and to the corresponding elastomeric polymers and compositions comprising the same that lead to an improved performance balance of rolling resistance and wet grip characteristics paired with satisfactory processing in terms of Mooney viscosity (CML) and maintenance of mechanical properties such as tensile strength (TS), elongation at break (EB) and abrasion resistance.

### SUMMARY OF THE INVENTION

The present invention is based on the finding that the above objects can be solved by carrying out the polymerization of conjugated diene monomers in the presence of a specific amine-containing vinyldisiloxane compound.

Thus, in a first aspect, the present invention provides a vinyldisiloxane compound of the following Formula 2: 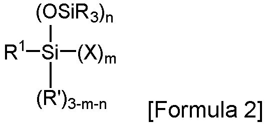 wherein
R and R' are independently selected from C₁₋₁₈ hydrocarbyl;
R¹ is optionally substituted vinyl, the one or more optional substituents being selected from methyl, ethyl and vinyl;
X is independently selected from one of the following moieties (X-1 to X-5): 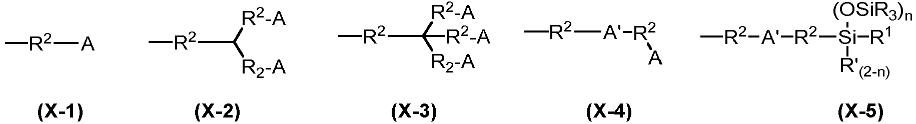
R² is a C₁₋₁₈ hydrocarbylene;
A is NR³R⁴, R³ and R⁴ being independently selected from linear, branched and cyclic C₁₋₈ alkyl, C₆₋₁₂ aryl, C₇-C₁₅ aralkyl, SiR"₃ or linear, branched and cyclic C₂₋₈ alkyl-O-SiR"₃, wherein each R" is independently selected from C₁₋₆ alkyl, C₆₋₁₂ aryl and C₇₋₁₈ alkylaryl, and R³ may be connected with R² or R⁴ to form a ring and the ring may contain, further to the R²-bonded nitrogen atom, one or more selected from an oxygen atom, a nitrogen atom and a sulfur atom, with the proviso that, in case R³ and R⁴ are both SiR"₃, two R" may be connected with each other to form a ring together with the Si-atoms to which they are bonded;
A' is NR⁵ with R⁵ being selected from linear, branched and cyclic C₁₋₈ alkylene, C₆₋₁₂ arylene, C₇-C₁₅ aralkylene SiR"₃ or R²A; or A' is a cyclic diaminediyl group such as piperazinyl;
n is 1 or 2; m is 1 or 2, and m + n is 2 or 3.

In a second aspect, the present invention provides a process for preparing an elastomeric polymer, wherein the process comprises the polymerization of at least one conjugated diene monomer and optionally one or more aromatic vinyl monomers in the presence of one or more initiator compounds and one or more compounds of Formula 2 as defined in the first aspect of the invention. In a preferred embodiment of the second aspect of the present invention, the polymerization is carried out in the presence of one or more compounds of Formula 2 as defined in the first aspect of the invention.

In a third aspect, the present invention provides an elastomeric polymer obtainable by the process as defined in the second aspect of the invention.

In a fourth aspect, the present invention provides a non-vulcanized polymer composition comprising the elastomeric polymer of the third aspect of the present invention and one or more additives.

In a fifth aspect, the present invention provides a vulcanized polymer composition which is obtainable by vulcanizing the polymer composition of the fourth aspect of the present invention and one or more vulcanizing agents.

In a sixth aspect, the present invention provides a method of preparing a vulcanized polymer composition, wherein the method comprises the step of vulcanizing the polymer composition of the fourth aspect of the present invention which comprises one or more vulcanizing agents.

In a seventh aspect, the present invention provides an article comprising at least one component formed from the vulcanized polymer composition of the fourth aspect of the present invention.

The present invention is based on the finding that specific amine-containing vinyldisiloxanes may be used as modifiers ("modifying monomers") and allow the preparation of elastomeric polymers and compositions that exhibit beneficial properties especially when used in the manufacture of tires. Specifically, it was surprisingly found that vulcanized polymer compositions ("vulcanized rubber compounds") produced from the elastomeric polymer of the invention exhibit an improved balance of low rolling resistance and high wet grip paired with good processing and mechanical properties. The beneficial properties were in particular found when using a vulcanized polymer composition of the invention containing silica or carbon black as fillers.

It is known that aminosilanes as modifying agents lead to the generation of covalent Si-O-Si bonds between the modified polymer and, e.g., silica as the filler. Yet, it has been observed that aminosilane modified polymer compositions show low processing characteristics and unfavourable mechanical properties.

Without being bound by theory, the present inventors assume that, due to the presence of both an amine group and a disiloxane structure, the amine-containing vinyldisiloxane of the present invention leads to a more favourable interaction with fillers, such as silica or carbon black, in a polymer composition, because non-covalent bonds in the form of coordinative bonds may be additionally created. The elastomeric polymers of the present invention, modified by means of one or more amine-containing vinyldisiloxane compounds of the present invention, lead to an improved performance balance in terms of rolling resistance and wet grip, as well as satisfactory processing properties and satisfactory or even improved mechanical properties.

As a result, the present invention can address the requirements described herein, fulfilling them by a modifying monomer not considered in the present field before, namely an amine-containing vinyldisiloxane. By this way, the present invention has been achieved.

### BRIEF DESCRIPTION OF THE FIGURES

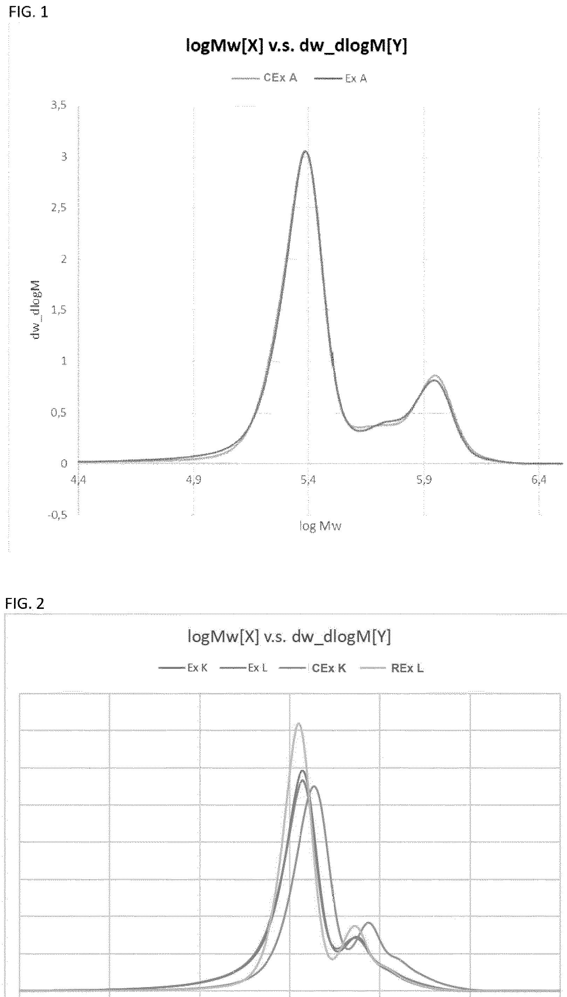

FIG. 1 shows the molecular weight distribution for Polymer Example A (Ex. A) and Polymer Comparative Example A (CEx. A) as measured by size exclusion chromatography (SEC).
FIG. 2 shows the molecular weight distribution for Polymer Example K (Ex. K), Polymer Example L (Ex. L), Polymer Comparative Example K (CEx. K) and Polymer Reference Example L (REx. L) as measured by size exclusion chromatography (SEC).

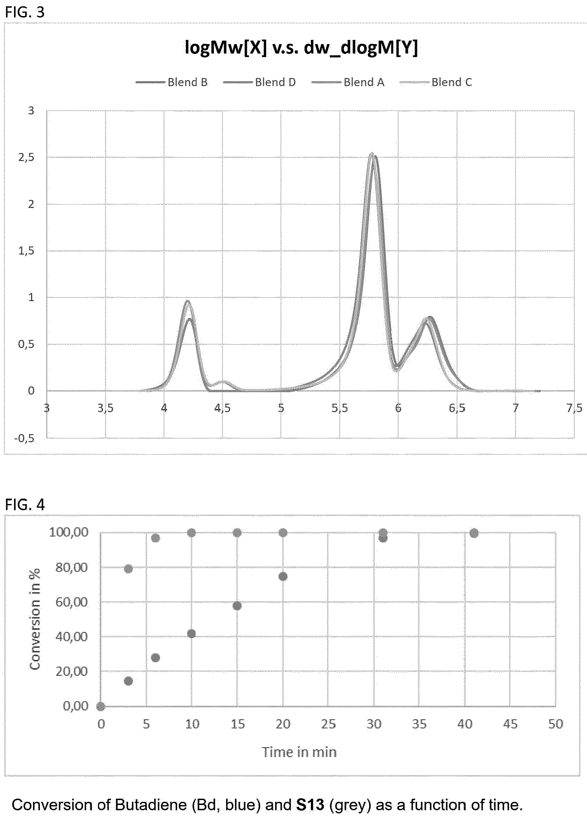

FIG. 3 shows the molecular weight distribution for SP/LP blends A, B, C and D as measured by size exclusion chromatography (SEC).
FIG. 4 is a monomer incorporation plot showing the conversion of butadiene (Bd) and the modifying monomer **S13** as a function of time for Polymer Example A (Ex. A) as measured by gas chromatography (GC).

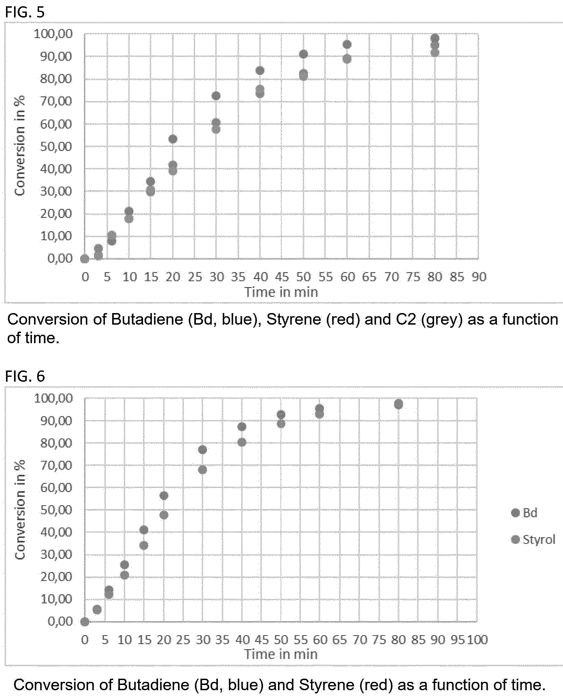

FIG. 5 is a monomer incorporation plot showing the conversion of butadiene (Bd), styrene and the modifying monomer **C2** as a function of time for Polymer Comparative Example K (CEx. K) as measured by gas chromatography (GC).
FIG. 6 is a monomer incorporation plot showing the conversion of butadiene (Bd) and styrene as a function of time for Polymer Reference Example L (REx. L) as measured by gas chromatography (GC).

### DETAILED DESCRIPTION

### Amine-containing vinyldisiloxane compound

The amine-containing vinyldisiloxane compound according to the first aspect of the present invention is characterized by having in combination both a siloxy group attached to the silicon atom of the vinylsilane moiety, thereby representing a vinyldisiloxane, as well as an organic group which is bonded to a Si-atom of the vinyldisiloxane moiety and has at least one tertiary amino group.

Specifically, the vinyldisiloxane compound of the first aspect of the present invention corresponds to the following Formula 2: 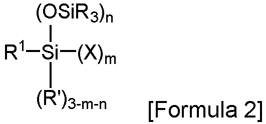
wherein R and R' are independently selected from C₁₋₁₈ hydrocarbyl; R¹ is optionally substituted vinyl, wherein the one or more optional substituents is selected from methyl, ethyl and vinyl; X is independently selected from one of the following moieties (X-1 to X-5): 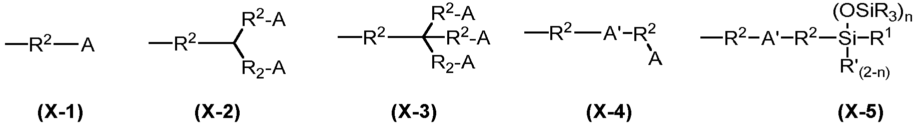
R² is a C₁₋₁₈ hydrocarbylene;
A is NR³R⁴, wherein R³ and R⁴ are independently selected from linear, branched and cyclic C₁₋₈ alkyl, C₆₋₁₂ aryl, C₇₋₁₅ aralkyl, SiR"₃ or linear, branched and cyclic C₂₋₈ alkyl-O-SiR"₃, wherein each R" is independently selected from C₁₋₆ alkyl, C₆₋₁₂ aryl and C₇₋₁₈ alkylaryl, and R³ may be connected with R² or R⁴ to form a ring and the ring may contain, further to the R²-bonded nitrogen atom, one or more selected from an oxygen atom, a nitrogen atom and a sulfur atom, with the proviso that, in case R³ and R⁴ are both SiR"₃, two R" may be connected with each other to form a ring together with the Si-atoms to which they are bonded; A' is NR⁵ with R⁵ being selected from linear, branched and cyclic C₁₋₈ alkylene, C₆₋₁₂ arylene, C₇-C₁₅ aralkylene, SiR"₃ or R²A; or A' is a cyclic diaminediyl group such as piperazinyl; n is 1 or 2; m is 1 or 2, and m + n is 2 or 3.

In one preferred embodiment, R² in Formula 2 is linear or branched C₁₋₈ alkylene, C₆₋₁₂ arylene or C₇-C₁₅ aralkylene. More preferably, R² is independently phenylene or linear C₁₋₈ alkylene, preferably linear C₃-₆ alkylene, more preferably C₃ alkylene.

In another preferred embodiment, R and R' in Formula 2 are independently selected from methyl, ethyl, n-propyl, i-propyl, t-butyl, phenyl and benzyl. R¹ is optionally substituted vinyl, wherein the one or more optional substituents is selected from methyl, ethyl and vinyl. X is independently selected from one of the moieties X-1 to X-5. R² in Formula 2 is a C₁₋₁₈ hydrocarbylene, preferably linear or branched C₁₋₈ alkylene, C₆₋₁₂ arylene or C₇-C₁₅ aralkylene, more preferably, R² is independently phenylene or linear C₁₋₈ alkylene, preferably linear C₃-₆ alkylene, more preferably C₃ alkylene. A is NR³R⁴, wherein R³ and R⁴ are independently selected from linear, branched and cyclic C₁₋₈ alkyl, C₆₋₁₂ aryl, C₇₋₁₅ aralkyl, SiR"₃ or linear, branched and cyclic C₂₋₈ alkyl-O-SiR"₃, wherein each R" is independently selected from C₁₋₆ alkyl, C₆₋₁₂ aryl and C₇₋₁₈ alkylaryl, and R³ may be connected with R² or R⁴ to form a ring and the ring may contain, further to the R²-bonded nitrogen atom, one or more selected from an oxygen atom, a nitrogen atom and a sulfur atom, with the proviso that, in case R³ and R⁴ are both SiR"₃, two R" may be connected with each other to form a ring together with the Si-atoms to which they are bonded. A' is NR⁵ with R⁵ being selected from linear, branched and cyclic C₁₋₈ alkylene, C₆₋₁₂ arylene, C₇-C₁₅ aralkylene; or A' is a cyclic diaminediyl group such as piperazinyl. In Formula 2 n is 1 or 2; m is 1 or 2, and m + n is 2 or 3.

In accordance with the present invention, a vinyl group is defined as a group C(R^{a}R^{b})=C(R^{c})-, wherein R^{a}, R^{b}and R^{c} are independently selected from hydrogen, methyl, ethyl or vinyl. In one particularly preferred embodiment of any of the vinyldisiloxane compounds described above, the vinyl group of R¹ represents unsubstituted vinyl, that is R^{a}, R^{b} and R^{c} are all hydrogen and R¹ conforms to CH₂=CH-. In another preferred embodiment, R¹ is substituted vinyl, wherein one of R^{a} or R^{b} is unsubstituted vinyl and R^{c} is hydrogen an R¹ conforms to 1,3-butadienyl. More preferably, R¹ is unsubstituted vinyl.

In any of the above vinyldisiloxane compounds, the amine-moiety A may for example be represented by a cyclic amine, including 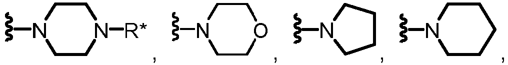 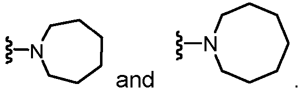 Preferably, A is represented by 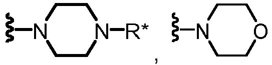 or 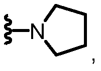 wherein R* may be selected from C₁₋₆ alkyl, C₆₋₁₂ aryl and C₇₋₁₈ alkylaryl, SiR"₃ or linear, branched and cyclic C₂₋₈ alkyl-O-SiR"₃, R" being defined as above. More preferably, A is represented by the piperazinyl moiety

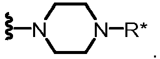

Preferably, when A is represented by the piperazinyl moiety, R* is methyl, ethyl, butyl, phenyl, benzyl; or A may be represented by piperazinyl which is substituted with SiR"₃ or C₂₋₈ alkyl-O-SiR"₃, and R" is independently selected from methyl, ethyl, n-propyl, i-propyl, t-butyl, phenyl and benzyl.

In another embodiment, in A, R³ and R⁴ may be independently selected from linear, branched and cyclic C₁₋₈ alkyl, C₆₋₁₂ aryl and C₇₋₁₅ aralkyl. Preferably, R³ and R⁴ are independently selected from methyl, ethyl, phenyl and benzyl.

In any of the vinyldisiloxane compounds of the first aspect of the present invention corresponding to Formula 2 each R may be independently selected from methyl, ethyl, i-propyl, t-butyl and phenyl. Preferably, the three Rs may be selected from the following combination of substituents: (methyl, methyl, methyl), (ethyl, ethyl, ethyl), (i-propyl, i-propyl, i-propyl), (phenyl, phenyl, phenyl), (methyl, methyl, t-butyl), (methyl, methyl, i-propyl), (ethyl, ethyl, i-propyl) and (phenyl, phenyl, t-butyl). More preferably the Rs are selected from (ethyl, ethyl, ethyl), (i-propyl, i-propyl, i-propyl) and (methyl, methyl, t-butyl). R", if present, may be equally selected from the substituents for R.

In any of the vinyldisiloxane compounds described above corresponding to Formula 2, n is 1 and m is 2, or n is 2 and m is 1, or n is 1 and m is 1, preferably n is 1 and m is 2 or 1.

As discussed above, in one embodiment of the vinyldisiloxane compound of the first aspect of the present invention, X in Formula 2 corresponds to (X-1): 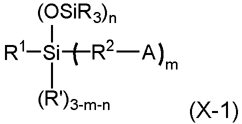 wherein R, R', R¹, R² and A are defined as above. Preferably, R¹ is an unsubstituted vinyl group or R² represents C₁₋₈ alkylene, preferably linear C₁₋₈ alkylene, for example methylene, ethylene, n-propylene and n-butylene or R¹ is an unsubstituted vinyl group and R² represents C₁₋₈ alkylene, preferably linear C₁₋₈ alkylene, for example methylene, ethylene, n-propylene and n-butylene. In another preferred embodiment, in case n is 1 and m is 1, R' is selected from methyl, ethyl, n-propyl, i-propyl, t-butyl, phenyl and benzyl and is preferably methyl.

In another exemplary embodiment, R' is unsubstituted vinyl, R' is methyl, R² is selected from C₁₋₈ alkylene and is preferably n-propylene and each R is independently selected from methyl, ethyl, i-propyl, t-butyl and phenyl. In this exemplary embodiment, A is represented by NR³R⁴, wherein R³ and R⁴ form a ring which may further contain a heteroatom, selected from oxygen, nitrogen and sulfur, preferably nitrogen. For example, R³ and R⁴ are connected such as to form a piperazinyl 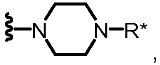 wherein R* is preferably methyl, phenyl, benzyl, SiR"₃ or linear, branched and cyclic C₂₋₈ alkyl-O-SiR"₃, wherein the C₂₋₈ alkyl may preferably linear alkyl and R" is preferably selected from methyl, ethyl, n-propyl, i-propyl, t-butyl and phenyl. In a preferred embodiment, R* is methyl, phenyl or benzyl.

Alternatively, R³ and R⁴ may each independently represent SiR"₃, wherein R" may be preferably selected from methyl, ethyl, n-propyl, i-propyl, t-butyl and phenyl. In one embodiment, each R" represents methyl. In another alternative embodiment, R³ and R⁴ are both SiR"₃ and two of the R" that are bonded to different Si-atoms form a ring together. For example, the two R" may form an alkylene group, such as ethylene, and the remaining R" may each independently be selected from C₁₋₆ alkyl and are preferably methyl.

In any of the above described vinyldisiloxane compounds corresponding to Formula 2, m is preferably 1 and n is preferably 1.

In addition, in any of the above described vinyldisiloxane compounds corresponding to Formula 2, R³ may be also be connected to R² to form a ring. In that case, A may be connected to the Si-atom via a cyclic ring structure and preferably A is incorporated in the cyclic ring structure. For example, -R²-A may be represented by 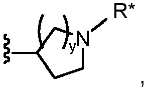 wherein R* is defined as above and may be preferably methyl, and y is 1 or more, preferably 1 or 2 and more preferably 2.

In another embodiment of the above described vinyldisiloxane compounds corresponding to Formula 2, wherein X is (X-1), m is 2. In this embodiment, R² may preferably be a C₁₋₈ alkylene, such as methylene, ethylene, n-propylene or n-butylene, more preferably n-propylene. In addition, for A which is represented by NR³R⁴, R³ and R⁴ may independently be selected from C₁₋₈ alkyl and are preferably methyl and/or benzyl. Alternatively, R³ and R⁴ may be connected to form a ring together with the nitrogen-atom they are bonded to in order to represent a pyrrolidine or a piperidine moiety.

In one embodiment of the above described vinyldisiloxane compounds in which X is (X-1) and m is 2, each set of R³ and R⁴ may be different to each other or be the same.

In another embodiment of any of the vinyldisiloxane compounds described above, X in Formula 2 may correspond to (X-2) and the compound is represented by the following formula: 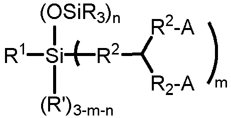 wherein each R² is each independently selected from the definitions above and A is as defined for (X-1). Preferably, one or more of R² are selected from C₁₋₈alkylene and preferably are selected from methylene, ethylene and n-propylene.

In another embodiment, X in Formula 2 may correspond to (X-3) and the compound is represented by the following formula: 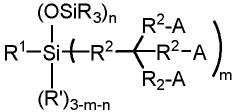 wherein each R² is each independently selected from the definitions above and A is as defined for (X-1). Preferably, one or more of R² are selected from C₁₋₈alkylene and preferably are selected from methylene, ethylene and n-propylene.

In a further embodiment, X in Formula 2 may correspond to (X-4) and the compound is represented by the following formula:

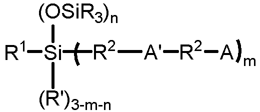

In this embodiment, R² each preferably represents a linear or branched C₁₋₈ alkylene. Preferably, A' represents a cyclic diaminediyl structure such as, for example, a piperazinyl as shown above. As a non-limiting example, a compound of Formula 2 in which X represents (X-4) may be as follows: 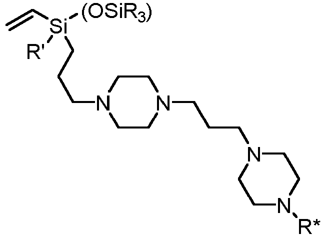 wherein R' and R³ are defined as above.

In another embodiment, X in Formula 2 may correspond to (X-5). That is, the vinyldisiloxane compound may represent a structure, wherein X is represented by the following structure: 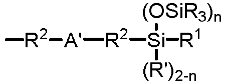 wherein R, R¹, R², R' as well as n are defined as above.

In the present invention, X preferably conforms to (X-1) as defined above.

### Preparation of the amine-containing vinyldisiloxane compound

The synthesis of an amine-containing vinyldisiloxanes of the present invention constitutes part of the common general knowledge of a person skilled in the art. For example, a chloro-vinyldisiloxan precursor may be reacted with a Grignard reagent prepared from a halogenated derivative of the amine-containing moiety X as defined for Formula 2.

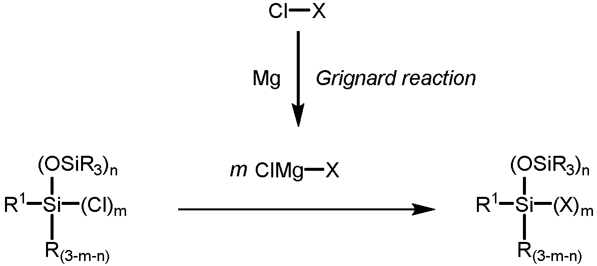

The conversion of chloro-vinyldisiloxanes may be performed in analogous manner to the reaction disclosed for chloro-vinylalkylsilanes with Grignard reagents described in, e.g., R. A. Benkeser et al. J. Org. Chem. 1979, 44, pp. 1370-1376; or J. Kazmierczak at al. J. of Catalysis 2018, 367, pp. 95-103.

The chloro-vinyldisiloxan may be prepared from a corresponding dichloro-vinylsilane (in case m = 1) or a corresponding trichloro-vinylsilane (in case m = 2) and a silanol R₃SiOH in the presence of a base, such as triethylamine (TEA). A second approach to alkylamine-containing vinylsilanes is described in US20100317852A1 (Shin Etsu). Here, allyl amines are converted to chlorosilanes in the presence of transition metal catalysts such as Pt-complexes. However, for the inventive vinyldisiloxanes, this approach needs to be slightly adjusted.

### Polymerization

The process for preparing the elastomeric polymer according to the second aspect of the present invention comprises the polymerization of at least one conjugated diene monomer and optionally one or more aromatic vinyl monomers in the presence of an initiator compound and one or more of the modifying monomers of the present invention.

The elastomeric polymer can generally be prepared via anionic, radical or transition metal-catalyzed polymerization, but is preferably prepared by anionic polymerization. Two or more vinyldisiloxane compounds of Formula 2 may be used in combination as modifying monomer(s). The polymerization may be conducted in a solvent and may be carried out with one or more of chain end-modifying agents, coupling agents incl. modified coupling agents, randomizer compounds and polymerization accelerator compounds.

Further to the following specific disclosure, generally applicable directions on polymerization technologies including polymerization initiator compounds, polar coordinator compounds and accelerators (for increasing/changing the reactivity of the initiator, for randomly arranging aromatic vinyl monomers and/or for randomly arranging and/or changing the concentration of 1,2-polybutadiene or 1,2-polyisoprene or 3,4-polyisoprene units introduced in the polymer); the amounts of each compound; monomer(s); and suitable process conditions are described in WO 2009/148932.

### a) Conjugated dienes (conjugated diene monomers)

Exemplary conjugated diene monomers useful in the present invention include 1,3-butadiene, 2-(C₁₋₅-alkyl)-1,3-butadiene such as isoprene (2-methyl-1,3-butadiene), 2,3-dimethyl-1,3-butadiene, 1,3-pentadiene, 2,4-hexadiene, 1,3-hexadiene, 1,3-heptadiene, 1,3-octadiene, 2-methyl-2,4-pentadiene, cyclopentadiene, 2,4-hexadiene, 1,3-cyclooctadiene, β-myrcene, terpinene, α-farnesene. A mixture of two or more conjugated dienes may be used. Preferred conjugated dienes include 1,3-butadiene and isoprene. In a preferred embodiment, the conjugated diene is 1,3-butadiene. The conjugated dienes may be used in a total amount of up to 99.99 wt.%, preferably 30 to 99.99 wt.%, based on the total weight of monomers used in the polymerization reaction.

### b) Aromatic vinyl monomers

The optional aromatic vinyl monomers are compounds having only one vinyl group attached to an aromatic group. Exemplary aromatic vinyl monomers include styrene, C₁₋₄ alkylsubstituted styrene such as 2-methylstyrene, 3-methylstyrene, 4-methylstyrene, 2,4-dimethylstyrene, 2,4,6-trimethylstyrene, α-methylstyrene, 2,4-diisopropylstyrene and 4-tert-butylstyrene, stilbene, vinyl benzyl dimethylamine, (4-vinylbenzyl)dimethyl aminoethyl ether, N,N-dimethylaminoethyl styrene, tert-butoxystyrene and vinylpyridine. Two or more aromatic vinyl monomers may be used in combination. A preferred aromatic monovinyl monomer is styrene. The aromatic vinyl monomer(s) may be used, depending on the application, in a total amount of up to 70 wt.%, especially 40-70 wt.% or 0-40 wt.%, for example 15-40 wt.% or 2-15 wt.%, based on the total weight of monomers used in the polymerization reaction.

In a preferred embodiment, the conjugated diene is 1,3-butadiene and the aromatic vinyl monomer is styrene.

### c) Other monomers

Comonomers other than the vinyldisiloxane monomer of Formula 2, the conjugated diene monomer and the aromatic vinyl monomer, which may be used in preparing the elastomeric polymer of the invention, include acrylic monomers such as acrylonitrile, acrylates, e.g., acrylic acid, methyl acrylate, ethyl acrylate, propyl acrylate and butyl acrylate, and methacrylates, e.g., methyl methacrylate, ethyl methacrylate, propyl methacrylate and butyl methacrylate.

Comonomers also include aromatic di- or higher vinyl compounds which have two or more vinyl groups attached to an aromatic group, such as divinylbenzene, including 1,2-divinylbenzene, 1,3-divinylbenzene and 1,4-divinylbenzene (which may be used as an isomeric mixture of 1,2-/1,3-/1,4-divinylbenzene herein referred to as "DVB"). They may be used in total amounts of 1 wt.% or less (based on the total molar weight of the monomers used to make the polymer). In one preferred embodiment, 1,2-divinylbenzene is used in combination with styrene and butadiene or isoprene. Further monomers include, for example, vinyl aminosilanes as described in WO2015/055252, WO2016/131590, WO2017/216344 and WO2019/020752 or monomers as disclosed in WO2008108377A or WO2019216645A1.

### d) Initiator compounds

The polymerization process of the present invention is carried out in the presence of an initiator compound, and two or more initiator compounds may be used in combination. The initiator compound may be a monovalent or multivalent (divalent, trivalent, etc.) initiator compound, including dianionic initiators. Suitable initiator compounds include alkali metals, organoalkali metal compounds, a complex between an alkali metal and a polar compound, an oligomer containing an alkali metal, and Lewis acid-base complexes. Exemplary alkali metals include lithium, sodium, potassium, rubidium and cesium. Exemplary organoalkali metal compounds include ethyllithium, n-butyllithium, s-butyllithium, t-octyllithium, isopropyllithium, phenyllithium, cyclohexyllithium, 2-butyllithium, 4-phenylbutyllithium, t-butyldimethylsilyloxypropyllithium, dialkylaminopropyllithium, N-morpholinopropyllithium, lithiumdiisopropylamide, lithium piperidide, lithium pyrrolidide, dilithiated diphenylethylene compounds, multi-lithiated trivinyl benzene compounds, sodium biphenylide, sodium naphthalenide and potassium naphthalenide. Exemplary complexes between an alkali metal and a polar compound include a lithium-tetramethylethylenediamine complex, a lithium-tetrahydrofuran complex, a lithium-ditetrahydrofuranepropane complex, and the sodium and potassium analogues thereof. More preferably, the initiator compound is a mono- or dilithium alkyl, alkylaryl or aryl compound. Further useful initiators include the amino silane polymerization initiators described in WO 2014/040640 and the polymerization initiators described in WO 2015/010710. The total amount of the initiator(s), in particular the organolithium initiator(s), will be adjusted depending on the monomer and target molecular weight. The total amount is typically from 0.05 to 5 mmol, preferably from 0.2 to 3 mmol per 100 grams of monomer.

### e) Solvent

The polymerization is usually conducted as a solution polymerization, wherein the formed polymer is substantially soluble in the reaction mixture, or as a suspension/slurry polymerization, wherein the formed polymer is substantially insoluble in the reaction medium. The terms "solution polymerization" and "suspension polymerization" or "slurry polymerization" are use with their conventional meaning in the art of polymerization. More preferably, the polymer is obtained in a solution polymerization. As the polymerization solvent, a hydrocarbon solvent is conventionally used which does not deactivate the initiator, catalyst or active polymer chain. A combination of two or more solvents may be used. Exemplary hydrocarbon solvents include aliphatic and aromatic solvents. Specific examples include (including all conceivable constitutional isomers): propane, butane, pentane, hexane, heptane, butene, propene, pentene, hexane, octane, benzene, toluene, ethylbenzene and xylene.

### f) Chain End-Modifying Agents

One or more chain end-modifying agents may be used in the polymerization reaction of the present invention for further controlling polymer properties by reacting with the terminal ends of the polymer chains in the polymer of the invention. Generally, silane-sulfide omega chain end-modifying agents such as disclosed in WO 2007/047943, WO 2009/148932, US 6,229,036 and US 2013/0131263, can be used for this purpose. Other chain end-modifying agents suitable for use in the present invention are those disclosed in WO 2014/040640 and WO 2015/010710 and the silane sulfide modifiers described in WO 2014/040639.

The chain end-modifying agents may be added intermittently (at regular or irregular intervals) or continuously during the polymerization, but are preferably added at a conversion rate of the polymerization of more than 80 percent and more preferably at a conversion rate of more than 90 percent. Preferably, a substantial amount of the polymer chain ends is not terminated prior to the reaction with the chain end-modifying agent; that is, living polymer chain ends are present and are capable of reacting with the modifying agent.

Other chain end-modifying agents suitable for use in the present invention are those disclosed in WO2014/040639, WO2020179705A1, JP2020015881A and WO2019225824A1.

### g) Coupling Agents

For further controlling polymer molecular weight and polymer properties, a coupling agent ("linking agent") can be used as an optional component in the process of the invention. A coupling agent will reduce hysteresis loss by reducing the number of free chain ends of the elastomeric polymer and/or reduce the polymer solution viscosity, compared with noncoupled essentially linear polymer macromolecules of identical molecular weight. Coupling agents such as tin tetrachloride, tetrachloro silane, tetraethoxy silane, tetramethoxy silane, hexachlorodisilane (HCDS) may functionalize the polymer chain end and react with components of an elastomeric composition, for example with a filler or with unsaturated portions of a polymer. Exemplary coupling agents are described in U.S. 3,281,383, U.S. 3,244,664 and U.S. 3,692,874 (e.g., tetrachlorosilane); U.S. 3,978,103, U.S. 4,048,206, 4,474,908 and U.S. 6,777,569 (blocked mercaptosilanes); U.S. 3,078,254 (multi-halogen-substituted hydrocarbon, such as 1,3,5-tri(bromomethyl) benzene); U.S. 4,616,069 (tin compound and organic amino or amine compound); and U.S. 2005/0124740. Generally, the chain end-modifying agent is added before, during or after the addition of the coupling agent, and the modification reaction is preferably carried out after the addition of the coupling agent. The total amount of coupling agents used will influence the Mooney viscosity of the coupled polymer and is typically in the range of from 0.001 to 4.5 milliequivalents per 100 grams of the elastomeric polymer, for example 0.01 to about 1.5 milliequivalents per 100 grams of polymer.

Further exemplary coupling agents are disclosed in DE102019117685A1, EP3666800A1, WO2016133154A and JP2014055264A.

### h) Randomizer compounds

Randomizer compounds as conventionally known in the art (also known as polar coordinator compounds) may optionally be added to the monomer mixture or polymerization reaction, in order to adjust the microstructure (i.e. the content of vinyl bonds) of the conjugated diene part of the polymer, or to adjust the composition distribution of any aromatic vinyl monomer and of the vinyl bonds in the polymer chain. A combination of two or more randomizer compounds may be used. Randomizer compounds useful in the invention are generally exemplified by Lewis base compounds. Suitable Lewis bases for use in the present invention are, for example, ether compounds such as diethyl ether, di-n-butyl ether, ethylene glycol diethyl ether, ethylene glycol dibutyl ether, diethylene glycol dimethyl ether, propylene glycol dimethyl ether, propylene glycol diethyl ether, propylene glycol dibutyl ether, (C1-C8 alkyl)tetrahydrofurylethers (including methyltetrahydrofurylether, ethyltetrahydrofurylether, propyltetrahydrofurylether, butyltetrahydrofurylether, hexyltetrahydrofurylether and octyltetrahydrofurylether), tetrahydrofuran, 2,2-(bistetrahydrofuryl)propane (DTHFP), bistetrahydrofurfurylformal, methyl ether of tetrahydrofurfuryl alcohol, ethyl ether of tetrahydrofurfuryl alcohol, butyl ether of tetrahydrofurfuryl alcohol, α-methoxytetrahydrofuran, dimethoxybenzene and dimethoxyethane, and tertiary amines such as triethylamine, pyridine, N,N,N',N'-tetramethyl ethylenediamine (TMEDA), dipiperidinoethane, methyl ether of N,N-diethylethanolamine, ethyl ether of N,N-diethylethanolamine, N,N-diethylethanolamine and dimethyl N,N-tetrahydrofurfuryl amine. Examples of preferred randomizer compounds are identified in WO 2009/148932. The randomizer compound will typically be added at a molar ratio of randomizer compound to initiator compound of from 0.012:1 to 10:1, preferably from 0.1:1 to 8:1 and more preferably from 0.25:1 to about 6:1.

### i) Accelerator compounds

The polymerization can optionally include accelerator compounds to increase the reactivity of the initiator (and, thus, to increase the polymerization rate), to randomly arrange aromatic vinyl monomers introduced into the polymer, or to provide a single chain of aromatic vinyl monomers, thus influencing the distribution of aromatic vinyl monomers in a living anionic elastomeric copolymer. Examples of accelerators include sodium alkoxides or sodium phenoxides and potassium alkoxides or potassium phenoxides, preferably potassium alkoxides or potassium phenoxides, such as potassium isopropoxide, potassium t-butoxide, potassium t-amyloxide, potassium n-heptyloxide, potassium benzyloxide, potassium phenoxide; potassium salts of carboxylic acids, such as isovaleric acid, caprylic acid, lauric acid, palmitic acid, stearic acid, oleic acid, linolenic acid, benzoic acid, phthalic acid and 2-ethyl hexanoic acid; potassium salts of organic sulfonic acids, such as dodecyl benzenesulfonic acid, tetradecyl benzenesulfonic acid, hexadecyl benzenesulfonic acid and octadecyl benzenesulfonic acid; and potassium salts of organic phosphorous acids, such as diethyl phosphite, diisopropyl phosphite, diphenyl phosphite, dibutyl phosphite, and dilauryl phosphite. Such accelerator compounds may be added in a total amount of from 0.005 to 0.5 mol per 1.0 gram atom equivalent of initiator. If less than 0.005 mol is added, a sufficient effect is not typically achieved. On the other hand, if the amount of the accelerator compound is more than about 0.5 mol, the productivity and efficiency of the chain end modification reaction can be significantly reduced.

### j) Dosing

The vinyldisiloxane of the present invention can be used in an amount of from 0.2 equivalent per equivalent of initiator compound(s) to 50 wt.% based on the total amount of the resulting elastomeric polymer. When the polymer of the invention is used in tire applications, for example in rubber compound for a tire tread or tire sidewall, it is preferable to use the vinyldisiloxane of the present invention in an amount of from 0.5 equivalent per equivalent of initiator compound(s) to 20 wt.%, more preferably up to 10 wt.%, even more preferably up to 1 wt.% based on the elastomeric polymer. The remaining amount of the elastomeric polymer is derived from the conjugated diene monomer and optional aromatic vinyl monomer as well as further optional components, such as chain end-modifying agents, coupling agents and randomizers.

The mode of addition ("dosing") of the vinyldisiloxane of the present invention in the polymerization relative to the conjugated diene monomer and optional aromatic vinyl monomer, initiator compound and other components will affect the structure of the resulting polymer. Thus, statistical copolymers and block copolymers having blocks of vinyldisiloxane polymer and blocks of other monomers in desired proportions and sequences can be prepared. Without intending any limitation on dosing options generally available, the following dosing options are exemplarily mentioned for the process for preparing the elastomeric polymer of the invention:
(1) Continuous (incremental) addition of the vinyldisiloxane compound of the invention to a mixture comprising the conjugated diene monomer, optionally aromatic vinyl monomer, and the initiator compound, as the polymerization proceeds, which results in the provision of a statistical copolymer.
(2) Dosing of the vinyldisiloxane compound of the invention before addition of the main amount of initiator a) together with main amounts of comonomers (tapered structure) or b) without other comonomers, which can be added after conversion of the vinyldisiloxane to generate a block structure. After quantitative or close to quantitative conversion of monomers, a second addition of vinyldisiloxane may be performed to generate block structure at a polymer end.
(3) Dosing of the vinyldisiloxane compound of the invention before addition of the main amount of initiator a) together with main amounts of comonomers (tapered structure) or b) without other comonomers, which can be added after quantitative or close to quantitative conversion of the vinyldisiloxane to generate block structure. Additionally, several (n) dosing steps of vinyldisiloxane compound of Formula 2 in variable proportions can be made at defined degrees of conversion of total monomer to generate n tapered or block structure elements within the polymer chain. For example, 67% of the vinyldisiloxane may be provided before the addition of the main amount of initiator and 33% of the vinyldisiloxane may be added after 50% conversion of the total monomers. The dosing of vinyldisloxane may also performed by providing 20% of the vinyldisiloxane before the addition of the main amount of initiator and adding 80% of the vinyldisiloxane after 50% conversion of the total monomers.

As another example, 67% of the vinyldisiloxane may be provided before the addition of the main amount of initiator and 33% of the vinyldisiloxane may be added 10-15 minutes before the addition of a coupling agent (as defined above) and/or a chain-end modifying agent (as defined above) for termination of the polymerization.
(4) Several (n) dosing steps of the vinyldisiloxane compound of the invention in variable proportions can be made at defined degrees of conversion of total monomer to generate n (tapered or block) structure elements within the polymer chain. For example, 50% of the vinyldisiloxane may be provided before the addition of the main amount of initiator, and 50% of the vinyldisiloxane may be added after 50% conversion of the total monomers and before the addition of a coupling agent and/or a chain-end modifying agent for termination of the polymerization. As another example, 33% of the vinyldisiloxane may be provided before the addition of the main amount of initiator, 33% of the vinyldisiloxane may be added after 50% conversion of the total monomers, and 33% of the vinyldisiloxane may be added 10-15 min before the addition of a coupling agent and/or a chain-end modifying agent for termination of the polymerization.
(5) Dosing of the vinyldisiloxane compound of the invention only after addition of the main amount of initiator and, e.g., in an amount of 50% after 50% conversion of the comonomers and 50% of the vinyldisiloxane may be added 10-15 min before the addition of a coupling agent and/or a chain-end modifying agent for termination of the polymerization.

### Elastomeric polymer

The elastomeric polymer according to the third aspect of the invention is obtainable by the process of the second aspect of the present invention, namely by a process comprising the polymerization of at least one conjugated diene monomer and optionally one or more aromatic vinyl monomers in the presence of one or more initiator compounds and one or more compounds of the first aspect of the invention. The elasomteric polymer of the invention may be a statistical, block or tapered copolymer, or an alpha- or alpha,omega-modified polymer in which the vinyldisiloxane compound of the invention is incorporated in the polymer chain by means of its vinyl function. The elastomeric polymer may be linear or branched.

For example, the elastomeric polymer may comprise the following repeating unit in the polymer chain: 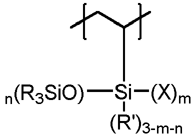 wherein R, R' and X, as well as m and n are defined as above for Formula 2.

In preferred embodiments, the elastomeric polymer of the invention is an SSBR (solution styrene butadiene rubber) with a preferred vinyl content (vinyl content = the content of 1,2-butadiene fraction based on BR fraction of the polymer or copolymer) of 5-80 wt.%, preferred 10-75 wt.%, for example 10-30 wt.%, 30-50 wt.% or 50-70 wt%, a styrene content (depending on the specific application) of 40-70 wt.%, or 15-40 wt.%, or 2-15 wt.%; a PBR (polybutadiene rubber) with a vinyl content of <15 wt.%; or 15-40 wt.%, or 40-80 wt.%; a PIR (polyisoprene rubber); an SSIR (solution styrene isoprene rubber); or an SSIBR (solution styrene isoprene butadiene rubber); more preferably an SSBR or PBR; even more preferably the elastomeric polymer is an SSBR, each being modified by incorporation of the vinyldisiloxane monomer according to the invention. In case of an SSBR, the elastomeric polymer is characterized by a glass transition temperature (Tg, determined by DSC) of -90 to 0°C, preferably -85 to -5°C, more preferably -80 to -15°C. Preferred embodiments for passenger car tire or truck tire applications include -80 to -60°C, -60 to -40°C and -40 to - 15°C.

In preferred embodiments, the polymer of the invention has prior to coupling and chain-end modification a peak molecular weight (measured by GPC) from: 800 to 10,000 g/mol or 10,000 to 50,000 g/mol or 50,000 to 120,000 g/mol or 120,000 to 250,000 g/mol or 250,000 to 400,000 g/mol or 400,000 to 800,000 g/mol.

In the present embodiments, the double bond of the conjugated diene-based polymer obtained in accordance with the above-described production method can be totally or partially converted into a saturated hydrocarbon through further hydrogenation in an inert solvent. This hydrogenation can improve the thermal resistance and weatherability, and prevent degradation of a product in processing at high temperature. Thus, better performance is exerted in various applications including automobile applications.

The ratio of hydrogenation of unsaturated double bonds on the basis of a conjugated diene compound (also referred to as "hydrogenation ratio", simply) is not particularly limited, and can be arbitrarily selected in accordance with the purpose. In the case of use for a vulcanized rubber, it is preferred that the double bonds in the conjugated diene portion be partially remained. From this viewpoint, the hydrogenation ratio of the conjugated diene portion in the polymer is preferably 3.0% or more and 95% or less, more preferably 10.0% or more and 90% or less, and further preferably 20% or more and 85% or less. The hydrogenation ratio of aromatic double bonds on the basis of an aromatic vinyl compound in the copolymer of a conjugated diene compound and an aromatic vinyl compound is not particularly limited. However, the hydrogenation ratio of aromatic double bonds on the basis of an aromatic vinyl compound in the copolymer of a conjugated diene compound and an aromatic vinyl compound is preferably 50% or less, more preferably 30% or less, and further preferably 20% or less. The hydrogenation ratio can be determined by using a nuclear magnetic resonance apparatus (NMR).

The method for hydrogenation is not particularly limited, and known methods can be used. Examples of suitable methods for hydrogenation include a method in which a polymer solution is bubbled with gaseous hydrogen to hydrogenate in the presence of a catalyst. Examples of the catalyst include heterogeneous catalysts such as catalysts including a noble metal supported on a porous inorganic material; and homogeneous catalysts such as catalysts derived by reaction of a solubilized salt of nickel, cobalt, or the like with an organoaluminum or the like, and catalysts with a metallocene such as titanocene. Among them, titanocene catalysts are preferred from the viewpoint that particularly mild hydrogenation conditions can be selected. Hydrogenation of aromatic groups can be performed by using a noble metal-supported catalyst.

Specific examples of hydrogenation catalysts include, but not limited to, (1) supporting-type heterogeneous hydrogenation catalysts with a metal such as Ni, Pt, Pd, and Ru supported on carbon, silica, alumina, diatomaceous earth, or the like; (2) what is called Ziegler hydrogenation catalysts with a transition metal salt such as an organic acid salt or acetylacetone salt of Ni, Co, Fe, Cr, or the like and a reductant such as an organoaluminum; and (3) what is called organometal complexes such as organometal compounds of Ti, Ru, Rh, Zr, or the like. Further examples of hydrogenation catalysts include hydrogenation catalysts described in Japanese Patent Publication No. 42-8704, Japanese Patent Publication No. 43-6636, Japanese Patent Publication No. 63-4841, Japanese Patent Publication No. 1-37970, Japanese Patent Publication No. 1-53851, Japanese Patent Publication No. 2-9041, and Japanese Patent Laid-Open No. 8-109219. Preferred examples of hydrogenation catalysts include a reaction mixture of a titanocene compound and a reducing organometal compound.

### Non-vulcanized polymer composition

The non-vulcanized (non-cured) polymer composition of the fourth aspect of the present invention comprises the elastomeric polymer of the invention and one or more additives. For example, the one or more additives are selected from extender oils, stabilizers and further polymers, including polymer oils (Mₚ (real) = 800 to 50,000 g/mol measured by GPC), (which are not the polymers of the invention). Such further polymers include what is conventionally referred to as oligomers in this field. Liquid polymers or polymer oils or oligomers are eg. homo- or copolymers of styrene, isoprene, butadiene or farnesene.

In one embodiment, the polymer composition comprises one or more fillers. In another embodiment, the polymer composition additionally comprises one or more vulcanizing agents. In a preferred embodiment, the polymer composition comprises one or more fillers and one or more vulcanizing agents.

In one embodiment, the non-vulcanized polymer composition is obtained by conventional work-up of the reaction mixture obtained in the polymerization process. Work-up means the removal of the solvent using steam stripping or vacuum evaporation techniques.

In another embodiment, the non-vulcanized polymer composition of the invention is obtained as a result of a further mechanical mixing process involving the worked-up reaction mixture (including the polymer of the invention), preferably in the form of a rubber bale (i.e. the product of a conventional compounding process in an internal mixer and /or by means of a two-roll mill), and at least one filler.

The following components are conventionally added in non-vulcanized compositions used in tires: extender oils, stabilizers, fillers, further polymers including polymer oils (Mₚ (real) = 800 to 50,000 g/mol measured by GPC).

### a) Extender oils

In one embodiment, the polymer composition of the present invention comprises the elastomeric polymer of the invention in combination with one or more oils, especially mineral oils or extender oils from natural (non-fossil) sources (such as sunflower oil, rapeseed oil, soy oil, tall oil or castor oil). For representative examples and classification of oils see, e.g., WO 2009/148932 and US 2005/0159513.

Such oils include, for instance, conventionally known extender oils such as aromatic, naphthenic and paraffinic extender oils, for example MES (mild extraction solvate), TDAE (treated distillate aromatic extract), rubber-to-liquid (RTL) oils, biomass-to-liquid (BTL) oils, factices, extender resins or liquid polymers (such as liquid BR) having a median molecular weight (determined via GPC according to BS ISO 11344:2004) of from 800 to 50,000 g/mol. When using a mineral oil as the extender oil, it is preferably one or more selected from DAE (Distillated Aromatic Extracts), RAE (Residual Aromatic Extract), TDAE, MES and naphthenic oils. The aforementioned oils comprise different concentrations of polycyclic aromatic compounds, paraffinics, naphthenics and aromatics, and have different glass transition temperatures. The above-mentioned types of oil have been characterized in "Kautschuk, Gummi, Kunststoffe", vol. 52, pages 799-805. In some embodiments, MES, RAE and TDAE are preferred extender oils for rubber.

The one or more oils can be added to the polymer prior to or after the termination of the polymerization process. When the extender oil is added to the polymer solution, the timing of addition should preferably be after modification of the polymer or termination of the polymerization, for example after the addition of the modifying agent or polymerization termination agent. After the addition of extender oil, the oil-extended polymer composition can be obtained by separating any polymerization solvent from the polymer by means of a direct drying method or steam stripping, drying the rubber using a vacuum dryer, hot-air dryer, roller and the like.

The polymer composition may contain one or more oils in a total amount of from 0 to 70 phr, preferably 0.1 to 60 phr, more preferably 0.1 to 50 phr. When liquid polymers are used as extender oils in the polymer composition of the present invention, they are not taken into account when calculating the composition of the polymer matrix.

In another embodiment, the oil is added to the "solvent-free" polymer in a mechanical mixer together with preferably at least one filler, and at least one further polymer.

### b) Stabilizers

One or more stabilizers (also referred to as "antioxidants") can optionally be added to the polymer prior to or after the termination of the polymerization process to prevent the degradation of the elastomeric polymer by molecular oxygen. Antioxidants based on sterically hindered phenols, such as 2,6-di-tert-butyl-4-methylphenol, 6,6'-methylenebis(2-tert-butyl-4-methylphenol), Iso-octyl-3-(3,5-di-tert-butyl-4-hydroxyphenyl) propionate, hexamethylenebis[3-(3,5-di-tert-butyl-4-hydroxyphenyl)propionate], octadecyl-3-(3,5-di-tert-butyl-4-hydroxyphenyl) propionate, isotridecyl-3-(3,5-di-tert-butyl-4-hydroxyphenyl) propionate, 1,3,5-trimethyl-2,4,6-tris(3,5-di-tert-butyl-4-hydroxybenzyl) benzene, 2,2'-ethylidenebis-(4,6-di-tert-butylphenol), tetrakis[methylene-3-(3,5-di-tert-butyl-4-hydroxyphenyl)propionate]methane, 2-[1-(2-hydroxy-3, 5-di-tert-pentylphenyl)ethyl]-4, 6-di-tert-pentylphenyl acrylate and 2-tert-butyl-6-(3-tert-butyl-2-hydroxy-5-methylbenzyl)-4-methylphenyl acrylate, and antioxidants based on thio-esters, such as 4,6-bis(octylthiomethyl)-o-cresol and pentaerythrityl tetrakis(3-laurylthiopropionate), are typically used. Other stabilizers are phosphite based (e.g. tris[4-(1,1-dimethylpropyl)phenyl] phosphite, bis[2,4-bis(2-methylbutan-2-yl)phenyl] [4-(2-methylbutan-2-yl)phenyl] phosphite, bis[4-(2-methylbutan-2-yl)phenyl][2,4-bis(2-methylbutan-2-yl)phenyl] phosphite, tris[2,4-bis(2-methylbutan-2-yl)phenyl] phosphite octadecyl 3-(3,5-di-tert-butyl-4-hydroxyphenyl)propionate). Further examples of suitable stabilizers can be found in F. Röthemeyer, F. Sommer, Kautschuk Technologie, 2nd ed., (Hanser Verlag, 2006) pages 340-344, and references cited therein.

### c) Further polymers

Apart from the polymer of the invention, extender oil(s), filler(s), etc., the polymer composition of the invention may additionally contain further polymers, especially further elastomeric polymers. Further polymers may be added in the form of a solution to a solution of the polymer of the invention prior to work up of the polymer blend or may be added during a mechanical mixing process, e.g. in a Brabender mixer.

Further (elastomeric) polymers as referred to herein are elastomeric polymers which are not in accordance with the polymer of the invention, i.e. which do not contain repeating units derived from the vinyldisiloxane compound of the present invention. Such further polymers include what is conventionally referred to as oligomers in this field.

### d) Fillers

The polymer composition of the present invention, which optionally comprises one or more extender oils as defined above, may further comprise one or more fillers. One or more fillers can be added to the polymer prior to or after the termination of the polymerization process. Examples of suitable fillers include carbon black (including electroconductive carbon black), carbon nanotubes (CNT) (including discrete CNT, hollow carbon fibers (HCF) and modified CNT carrying one or more functional groups, such as hydroxyl, carboxyl and carbonyl groups), graphite, graphene (including discrete graphene platelets), silica, carbon-silica dual-phase filler, clays (layered silicates, including exfoliated nanoclay and organoclay), calcium carbonate, magnesium carbonate, magnesium oxide, titanium dioxide, rubber gels, lignin, amorphous fillers, such as glass particle-based fillers, starch-based fillers, and combinations thereof. Further examples of suitable fillers are described in WO 2009/148932.

In the polymer composition of the present invention, the one or more fillers are preferably selected from carbon black, carbon nanotubes, graphite, graphene, silica, carbon-silica dual-phase filler, clays calcium carbonate, magnesium carbonate, lignin, glass particle-based fillers and starch-based fillers, preferably the filler is carbon black or silica or may be a combination thereof. More preferably, the filler is silica.

As the carbon black, any type of carbon black conventionally known to a person of skill in the art may be used. In one embodiment, the carbon black has an iodine number according to ASTM D 1510 of 20 to 250 mg/g, preferably 30 to 180 mg/g, more preferably 40 to 180 mg/g, and even more preferably 40 to 130 mg/g, and a DBP number according to ASTM D 2414 of 80 to 200 ml/100 g, preferably 100 to 200 ml/100 g, more preferably 115 to 200 ml/100 g (the DBP number determines the specific absorption volume of carbon black or of any bright filler by means of dibutyl phthalate).

Any type of silica conventionally known to a person of skill in the art and suitable as filler for tire rubber blends may be used. It is particularly preferred to use highly dispersed, precipitated silica having an nitrogen surface area (BET surface area; according to DIN ISO 9277 and DIN 66132) of 35 to 350 m²/g, preferably 35 to 260 m²/g, more preferably 100 to 260 m²/g and even more preferably 130 to 235 m²/g, and having a CTAB surface area (according to ASTM D 3765) of 30 to 400 m²/g, preferably 30 to 250 m²/g, more preferably 100 to 250 m²/g and even more preferably 125 to 230 m²/g. Such silica results, e.g. in rubber blends for tire treads, in particularly beneficial physical properties of the vulcanizates. In addition, it may bring about advantages in the processing of the blend, namely by reducing the time required for blending, while maintaining product properties, thus improving productivity. Useful silicas include those of the type Ultrasil^{®} VN3 (trademark of Evonik Industries) as well as highly dispersed types, so-called HD silicas (e.g. Zeosil^{®} 1165 MP of Rhodia).

### e) Vulcanizing agents and vulcanizing accelerators

The polymer composition of the prsent invention may optionally further comprise one or more vulcanizing agents. Any vulcanizing agent conventionally used in the manufacture of rubber products can be used in the invention, and a combination of two or more vulcanizing agents may be used.

Sulfur, sulfur-containing compounds acting as sulfur donors such as dithiols, sulfur accelerator systems and peroxides are the most common vulcanizing agents. Examples of sulfur-containing compounds acting as sulfur donors include dithiodimorpholine (DTDM), tetramethylthiuram disulfide (TMTD), tetraethyl thiuram disulfide (TETD) and dipentamethylene thiuram tetrasulfide (DPTT). Examples of sulfur accelerators include amine derivates, guanidine derivates, aldehydeamine condensation products, thiazoles, xanthogenates, thiuram sulfides, dithiocarbamates and thiophosphates. It is preferably to use one or more sulfonamide accelerators selected from N-cyclohexyl 2-benzothiazol sulfenamide (CBS), N,N-dicyclohexyl benzothiazole 2-sulfenamide (DCBS), benzothiazyl 2-sulfenemorpholide (MBS) and N-tert-butyl 2-benzothiazyl sulfenamide (TBBS). Further crosslinking systems such as available under the trade names Vulkuren^{®} (1,6-bis(N,N-dibenzyl thiocarbamoyldithio)-hexane; Lanxess), Duralink^{®} or Perkalink^{®} (1,3-bis(citraconimidomethyl)benzene; Lanxess) or disclosed in WO 2010/049261 may be added to the polymer composition. Examples of peroxides include di-*tert*-butyl-peroxides, di-(*tert-*butyl-peroxy-trimethyl-cyclohexane), di-(*tert*-butyl-peroxy-isopropyl-)benzene, dichloro-benzoylperoxide, dicumylperoxides, *tert*-butyl-cumyl-peroxide, dimethyl-di(*tert*-butyl-peroxy)hexane, dimethyl-di(*ter*-butyl-peroxy)hexine and butyl-di(tert-butyl-peroxy)valerate (Rubber Handbook, SGF, The Swedish Institution of Rubber Technolgy 2000).

A vulcanizing accelerator of the sulfene amide-type, guanidine-type or thiuram-type can be used together with a vulcanizing agent as required.

In addition, the polymer composition of the invention may contain conventional additives and vulcanization auxiliaries in proportions conventionally used. Such additives include:
a) aging inhibitors such as N-phenyl N'-(1,3-dimethylbutyl)-p-phenylenediamine (6PPD), N,N'-diphenyl-p-phenylenediamine (DPPD), N,N'-ditolyl-p-phenylenediamine (DTPD), N-isopropyl N'-phenyl-p-phenylenediamine (IPPD), 2,2,4-trimethyl 1,2-dihydrochinolin (TMQ),
b) activators such as zinc oxide and fatty acids (e.g. stearic acid);
c) waxes;
d) resins, especially adhesive resins;
e) mastication additives such as 2,2'-dibenzamidodiphenyldisulfide (DBD); and
f) processing additives such as zinc soaps and fatty acid esters and their derivatives.

Zinc oxide (zinc white) is preferably used as a component of the sulfur accelerator system.

Vulcanizing agents are typically added to the polymer composition in a total amount of from 0.5 to 10 parts by weight or, in some embodiments, 1 to 6 parts by weight per 100 parts by weight of the polymer. Examples of vulcanizing accelerators and amounts thereof relative to the polymer are given in WO 2009/148932.

### Vulcanized polymer composition

The vulcanized polymer composition of the fifth aspect of the present invention is obtainable by vulcanizing a polymer composition of the invention comprising one or more vulcanizing agents. The method of making the vulcanized polymer composition of the sixth aspect of the present invention comprises a step of vulcanizing the polymer composition of the invention under conditions and with machinery conventionally known in the art.

### Article

Since the vulcanized polymer compositions of the invention exhibit low rolling resistance and improved wet grip performance, as well as suitable mechanical properties, and can be favorably used, e.g., in a variety of different SBR/BR grades having different microstructures, they are well suited for use in manufacturing, e.g., tires or parts of tires including for example: tire treads, side walls and tire carcasses as well as other industrial products such as belts, hoses, vibration dampers and footwear components. Thus, the article of the seventh aspect of the present invention comprises at least one component formed from the vulcanized polymer composition of the invention. The article may be, for instance, a tire, including parts thereof such as a tire tread, a tire side wall and a tire carcass, a belt, a gasket, a seal, a hose, a vibration damper, a footwear component, such as a shoe sole, a golf ball or a hose. Preferably, the article is a tire (incl. summer, winter, all-weather passenger car tire or truck tires) or a part thereof, more preferably the article is a tire.

### DEFINITIONS AND ABBREVIATIONS

The chemical definitions of substituents or specific moieties, as well as abbreviations as used herein generally correspond to the meaning known in the art.

For example, alkyl groups as used herein and unless specifically defined otherwise, whether as such or in association with other groups, such as alkylaryl, include both straight chain (linear) alkyl groups, such as methyl (Me), ethyl (Et), n-propyl (n-Pr), n-butyl (n-Bu), n-pentyl, n-hexyl, octyl etc., branched alkyl groups, such as isopropyl (i-Pr), tert-butyl (t-Bu), etc., and cyclic alkyl groups, such as cyclohexyl (Cy).

Aryl groups as used herein include phenyl, biphenyl and other benzenoid compounds. Aryl groups preferably contain only one aromatic ring and most preferably contain a C₆ aromatic ring.

Alkylaryl groups as used herein refer to a combination of one or more aryl groups bound to one or more alkyl groups, for example in the form of alkyl-aryl, aryl-alkyl, alkyl-aryl-alkyl and aryl-alkyl-aryl. Alkylaryl groups preferably contain only one aromatic ring and most preferably contain a C₆ aromatic ring. For example, one preferred alkylaryl group is benzyl (Bn).

### EXAMPLES

The present invention will be explained in more detail by way of examples, which are not intended to be limiting the present invention.

### 1) Preparation and characterization of amine-containing vinyldisiloxane modifying monomers

### a) Synthesis of halogen-amine precursors

### Preparation Example 1

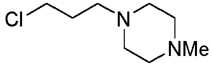

1-Bromo-3-chloropropane (78.7 g, 500 mmol) was dissolved in dioxane (750 ml). Sodium bicarbonate (42.0 g, 500 mmol, 1.0 equiv.) was added followed by 1-methylpiperazine (100 g, 1000 mmol, 2.0 equiv.). The reaction mixture was stirred at 50°C for 16 h. The solids were filtered off and the solvent was removed under reduced pressure and the residue was distilled fractionally to yield **Preparation Example 1** (52.3 g, 296 mmol, 59 %) as colorless oil. Purity 94 % (GC), C₈H₁₇ClN₂, M_{w} = 176.69 g/mol.

**bp** = 81-83°C (1.1 mbar). **¹H NMR** (400 MHz, 20°C, CDCl₃): δ = 3.59 (t, *J* = 6.6 Hz, 2 H), 2.49 (t, *J* = 7.0 Hz, 2 H), 2.52-2.39 (m, br, 8 H), 2.29 (s, 3 H), 1.95 (pent, *J* = 6.9 Hz, 2 H) ppm. **¹³C NMR** (101 MHz, 20°C, CDCl₃): δ = 55.40 (CH₂), 55.13 (2 CH₂), 53.21 (2 CH₂), 46.04 (CH₃), 43.20 (CH₂), 29.89 (CH₂) ppm. **GC-MS** (EI, 70 eV): m/z (%) = 176 (M⁺, 43), 132 (7), 113 (Me-Pip-Me⁺, 100), 106 (8), 98 (25), 85 (14), 70 (85). **IR** (ATR): λ⁻¹ = 2938 (m), 2877 (w), 2793 (s), 2684 (w), 1458 (m), 1448 (m), 1373 (m), 1356 (m), 1296 (m), 1283 (s), 1181 (m), 1162 (s), 1124 (m), 1088 (m), 1013 (vs), 814 (m), 787 (m), 758 (m), 723 (m), 653 (s) cm⁻¹.

### Preparation Example 2

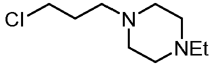

1-Bromo-3-chloropropane (34.5 g, 219 mmol) was added to a solution of triethylamine (26.6 g, 263 mmol, 1.2 equiv.) and 1-ethylpiperazine (25.0 g, 219 mmol, 1.0 equiv.) in DCM (220 ml). The reaction mixture was stirred at 25°C for 16 h. Hexanes (200 ml) were added and the solids were filtered off using Celite. The solvent was removed under reduced pressure and the residue was distilled fractionally to yield **Preparation Example 2** (26.9 g, 141 mmol, 64%) as colorless oil. Purity 95 % (GC), C₉H₁₉ClN₂, M_{w} = 190.72 g/mol.

**bp** = 82-84°C (0.4 mbar). The spectroscopic data was in accordance to the date cited in the literature (J. S. Yadav et al. Synth Commun. 2003, 33, pp. 2483-2486. DOI:10.1081/SCC-120021838).

### Preparation Example 3

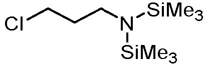

Chloropropylamine hydrochloride (20.0 g, 154 mmol, 1.0 equiv.) was dissolved in DCM (310 ml). Then triethylamine (54.5 g, 538 mmol, 3.5 equiv.) and chlorotrimethylsilane (41.8 g, 385 mmol, 2.5 equiv.) were added. The reaction mixture was stirred at 25°C for 16 h. Cyclohexane (300 ml) was added and the precipitate was filtered off. The solvent was removed under reduced pressure and the residue was distilled *in vacuo* to yield **Preparation Example 3** (30.0 g, 126 mmol, 82 %) as a colorless oil. Purity 96 % (GC), C₉H₂₄ClNSi₂, M_{w} = 237.92 g/mol.

**bp** = 67-69°C (0.6 mbar). **¹H NMR** (400 MHz, 20°C, CDCl₃): δ = 3.47 (t, *J* = 6.4 Hz, 2 H), 2.94-2.89 (m, 2 H), 1.86-1.78 (m, 2 H), 0.10 (s, 18 H) ppm. **¹³C NMR** (101 MHz, 20°C, CDCl₃): δ =43.13 (CH₂), 42.85 (CH₂), 37.66 (CH₂), 2.05 (6 CH₃) ppm. **GC-MS** (El, 70 eV): m/z (%) = 237 (M⁺, 2), 194 (30), 174 (100), 114 (8), 100 (11), 86 (66).

### Preparation Example 4

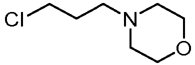

Morpholine (17.4 g, 200 mmol, 1.0 equiv.) was dissolved in DCM (80 ml). Then triethylamine (25.3 g, 250 mmol, 1.25 equiv.) and 1-bromo-3-chloropropane (39.4 g, 250 mmol, 1.25 equiv.) were added. The reaction mixture was stirred under reflux for 2 h and 16 h at 25°C. Cyclohexane (100 ml) was added and the precipitate was filtered off. The solvent was removed under reduced pressure and the residue was distilled *in vacuo* to yield **Preparation Example 4** (27.3 g, 166 mmol, 83 %) as a colorless liquid. C₇H₁₄ClNO, M_{w} = 163.65 g/mol.

**bp** = 70-73°C (0.2 mbar). **GC-MS** (El, 70 eV): m/z (%) = 186 (M⁺, 0.01),100 (CH₂=NC₄H₈O⁺, 100), 70 (0.1). **IR** (ATR): λ⁻¹ = 2958 (m), 2851 (m), 2795 (m), 1455 (m), 1413 (m), 1355 (m), 1281 (m), 1259 (m), 1150 (m), 1112 (s), 1071 (m), 1033 (m), 1017 (m), 928 (w), 905 (m), 866 (s), 797 (m), 639 (m) cm⁻¹.

### Preparation Example 5

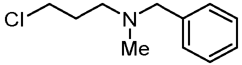

Benzylmethylamine (10.0 g, 82.5 mmol, 1.0 equiv.) was dissolved in DCM (80 ml). Then triethylamine (9.19 g, 91.1 mmol, 1.1 equiv.) and 1-bromo-3-chloropropane (13.0 g, 82.5 mmol, 1.0 equiv.) were added. The reaction mixture was stirred at 25°C for 16 h. Water (100 ml) was added and the pH was adjusted to pH=10. Then the aqueous phase was extracted with MTBE (3 x 100 ml). The combined organic phases were dried over MgSO₄, filtered, the solvent was removed under reduced pressure and the residue was distilled *in vacuo* to yield **Preparation Example 5** (9.97 g, 50.4 mmol, 65 %) as a colorless oil. Purity 92 % (GC), C₁₁H₁₆ClN, M_{w} = 197.71 g/mol.

**bp:** 85-90 °C (1.1 mbar). **¹H NMR** (400 MHz, 20°C, CDCl₃): δ = 7.32-7.29 (m, 4 H), 7.26-7.23 (m, 1 H), 3.60 (t, *J* = 6.8 Hz, 2 H), 3,48 (s, 2), 2.51 (t, *J* = 6.8 Hz, 2 H), 2.10 (s, 3 H), 1.96 (pent, *J* = 6.8 Hz, 2 H) ppm. **¹³C NMR** (101 MHz, 20°C, CDCl₃): δ = 139.05 (C), 128.87 (2 CH), 128.20 (2 CH), 126.94 (CH), 62.42 (CH₂), 54.31 (CH₂), 43.15 (CH₂), 42.10 (CH₃), 30.54 (CH₂), 17.15 (CH₃) ppm. **GC-MS** (El, 70 eV): m/z (%) = 197 (M⁺, 5), 134 (85), 120 (4), 91 (C₇H₇⁺,100). **IR** (ATR): λ⁻¹ = 3062 (w), 3027 (w), 2949 (w, br), 2791 (m, br), 1452 (m), 1025 (m), 733 (s), 693 (s), 653 (m) cm⁻¹.

### Preparation Example 6

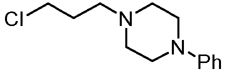

Phenylpiperazine (13.0 g, 80.0 mmol, 1.0 equiv.) was dissolved in THF (80 ml). Then triethylamine (9.31 g, 92.0 mmol, 1.15 equiv.) and 1-bromo-3-chloropropane (12.6 g, 80 mmol, 1.0 equiv.) were added. The reaction mixture was stirred at 25°C for 16 h and at 50°C for 2 d. Water (80 ml) was added and the pH was adjusted to pH=10. Then the aqueous phase was extracted with MTBE (3 x 80 ml). The combined organic phases were dried over MgSO₄, filtered, the solvent was removed under reduced pressure and the residue was distilled *in vacuo* to yield **Preparation Example 6** (9.71 g, 40.7 mmol, 51 %) as a colorless oil. Purity 97 % (GC), C₁₃H₁₉ClN₂, M_{w} = 238.76 g/mol.

**bp:** 143-146 °C (0.7 mbar). **¹H NMR** (400 MHz, 20°C, CDCl₃): δ = 7.29-7.24 (m, 2 H), 6.94-6.92 (m, 2 H), 6.85 (t, *J* = 7.2 Hz, 1 H), 3.63 (t, *J* = 6.6 Hz, 2 H), 3.21-3.18 (m, 4 H), 2.62-2.59 (m, 4 H), 2.55 (t, *J* = 7.0 Hz, 2 H), 1.99 (pent, *J* = 6.8 Hz, 2 H) ppm. **¹³C NMR** (101 MHz, 20°C, CDCl₃): δ = 151.29 (C), 129.09 (2 CH), 119.68 (CH), 116.03 (2 CH), 55.43 (CH₂), 53.27 (2 CH₂), 49.14 (2 CH₂), 48.18 (CH₂), 29.88 (CH₂) ppm. **GC-MS** (El, 70 eV): m/z (%) = 238 (M⁺, 60), 175 (Me-Pip-Ph⁺, 100), 160 (6), 147 (12), 132 (34), 105 (35).

### Preparation Example 7

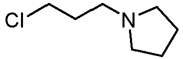

Pyrrolidine (7.11 g, 100 mmol, 0.7 equiv.) was added slowly to a solution of triethylamine (13.7 g, 136 mmol, 0.95 equiv.) and 1-bromo-3-chloropropane (22.5 g, 143 mmol, 1.0 equiv.) in DCM (40 ml). The reaction mixture was refluxed for 3 d. Cyclohexane (50 ml) was added, the solids were filtered oft. The solvent was removed under reduced pressure and the residue was distilled *in vacuo* to yield **Preparation Example 7** (3.83 g, 22.0 mmol, 22 %) as a colorless oil. Purity 95 % (GC), C₇H₁₄ClN, M_{w} = 147.65 g/mol.

**bp:** 25-29 °C (0.023 mbar). **¹H NMR** (400 MHz, 20°C, CDCl₃): δ = 3.33 (t, *J* = 6.6 Hz, 2 H), 2.33 (t, *J* = 6.8 Hz, 2 H), 2.24-2.20 (m, 4 H), 1.66 (pent, *J* = 6.7 Hz, 2 H), 1.56-1.50 (m, 4 H) ppm. **¹³C NMR** (101 MHz, 20°C, CDCl₃): δ = 54.12 (2 CH₂), 53.03 (CH₂), 43.19 (CH₂), 32.26 (CH₂), 23.87 (2 CH₂) ppm. **GC-MS** (El, 70 eV): m/z (%) = 147 (M⁺, 6), 110 (2), 84 (C₅H₁₀N⁺, 100), 70 (4).

### Preparation Example 8

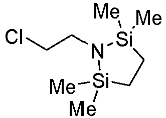

Chloroethylamine hydrochloride (17.4 g, 150 mmol, 1.0 equiv.) was dissolved in DCM (300 ml). Then triethylamine (50.1 g, 495 mmol, 3.3 equiv.) and 1,2-bis(chlorodimethylsilyl)ethane (35.9 g, 150 mmol, 1.0 equiv.) were added. The reaction mixture was stirred at 25°C for 16 h. The mixture was filtered, the solvent was removed under reduced pressure and the residue was distilled fractionally to yield **Preparation Example 8** (28.0 g, 126 mmol, 84%) as a colorless liquid. Purity 95 % (GC), C₈H₂₀ClNiSi₂, M_{w} = 221.88 g/mol.

**Bp:** 39-41 °C (0.01 mbar). **¹H NMR** (400 MHz, 20°C, CDCl₃): δ = 3.19 (t, *J* = 8.4 Hz, 2 H), 3.00 (t, *J* = 8.0 Hz, 2 H), 0.67 (s, 4 H), -0.04 (s, 12 H) ppm. **¹³C NMR** (101 MHz, 20°C, CDCl₃): δ = 45.65 (CH₂), 45.08 (CH₂), 8.14 (2 CH₂), -0.14 (4 CH₃) ppm. **GC-MS** (EI, 70 eV): m/z (%) = 206 (M⁺, 13), 172 (100), 150 (5), 137 (5), 117 (4), 100 (5), 85 (4).

### Preparation Example 9

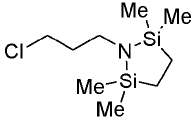

Chloropropylamine hydrochloride (10.0 g, 76.9 mmol) was suspended in DCM (154 ml) at 25°C. Triethylamine (25.7 g, 254 mmol, 3.3 equiv.) and 1,2-bis(chlorodimethylsilyl)ethane (18.4 g, 76.9 mmol, 1 equiv. dissolved in 40 ml DCM) were added and the resulting solution was stirred at 25°C for 16 h. Then cyclohexane (100 ml) was added and the precipitate was filtered off. All volatiles were removed under reduced pressure and the residue was distilled *in vacuo.* **Preparation Example 9** was received as colorless liquid (17.5 g, 74.2 mmol, 96%). C₉H₂₂ClNSi₂, M_{w} = 235.90 g/mol.

**Bp:** 97-100 °C (0.3 mbar). **GC-MS** (El, 70 eV): m/z (%) = 235 (M⁺, 4), 220 (M⁺ -CH₃, 2), 192 (31), 172 (100), 145 (5), 117 (5), 100 (5), 85 (5), 73 (10), 59 (15).

### Preparation Example 10

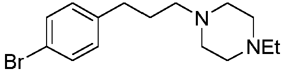

1,4-Dibromobenzene (100 mmol, 23.6g, 1.0 equiv.) was dissolved in THF (80 ml). Then Pd(dppf)Cl₂ (360 mg, 0.005 equiv.) and a Grignard solution of 1-chloro-3-(N-ethylpiperazinyl)propane* (80 mmol, 0.8 equiv. 1M in THF) was added at 25°C. The reaction mixture was stirred for 2 h. Then water (100 ml) was added and the aqueous phase was extracted with MTBE (3 x 100 ml). The organic phase was extracted with diluted HCl aq. and the pH adjusted (pH = 10) aqueous phase was extracted with MTBE (3 x 100 ml), dried over MgSO₄ and filtered. The solvent was removed under reduced pressure to yield **Preparation Example 10** (19.1 g, 61.4 mmol, 61%) as a yellow oil. C₁₅H₂₃BrN₂, M_{w} = 311.27 g/mol.

**¹H NMR** (400 MHz, 20°C, CDCl₃): δ = 7.37 (d, *J* = 8.4 Hz, 2 H), 7.05 (d, *J* = 8.4 Hz, 2 H), 2.58 (t, *J =* 7.8 Hz, 3 H), 2.48 (s, br, 6 H), 2.40 (q, *J* = 7.2 Hz, 2 H), 2.36-2.33 (m, 3 H), 1.78 (pent, *J* = 7.6 Hz, 2 H), 1.08 (t, *J* = 7.2 Hz, 3 H) ppm. **¹³C NMR** (101 MHz, 20°C, CDCl₃): δ = 141.04 (C), 131.24 (2 CH), 130.10 (2 CH), 119.36 (C), 57.74 (CH₂), 53.16 (2 CH₂), 52.79 (2 CH₂), 52.26 (CH₂), 33.02 (CH₂), 28.41 (CH₂), 11.94 (CH₃) ppm. **GC-MS** (El, 70 eV): m/z (%) = 310 (M⁺, 19), 295 (M⁺ -CH₃, 3), 267 (2), 254 (3), 240 (4), 168 (6), 141 (28), 127 (100), 99 (11), 84 (26).

*The Grignard solution of 1-chloro-3-(N-ethylpiperazinyl)propane used in Preparation Example 10 was prepared in the same manner as described in section b) below.

### Preparation Example 11

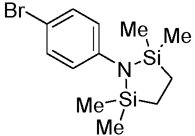

4-Bromoaniline (15.0 g, 87.2 mmol) was dissolved in DCM (300 ml). Then triethylamine (18.5 g, 183 mmol, 2.1 equiv.) and 1,2-bis(chlorodimethylsilyl)ethane (20.9 g, 87.2 mmol, 1.0 eq. dissolved in 30 ml DCM) were added and the mixture was stirred at 25°C for 16 h. Then cyclohexane (200 ml) was added and the precipitate was filtered off. All volatiles were removed under reduced pressure and the residue was distilled *in vacuo.* **Preparation Example 11** was received as yellow liquid (26.7 g, 84.9 mmol, 97%). C₁₂H₂₀BrNSi₂, M_{w} = 314.37 g/mol.

**bp:** 90-95 °C (0.03 mbar). **¹H NMR** (400 MHz, 20°C, C₆D₆): δ = 7.21-7.18 (m, 2 H), 6.64-6.61 (m, 2 H), 0.76 (s, 4 H), 0.08 (s, 12 H) ppm. **¹³C NMR** (101 MHz, 20°C, C₆D₆): δ = 146.61 (C), 132.36 (2 CH), 126.03 (2 CH), 114.17 (C), 8.54 (2 CH₂), -0.14 (4 CH₃) ppm. **²⁹Si{H} NMR** (79.5 MHz, 20°C, C₆D₆): δ = 13.04 (2 Si) ppm.

### b) Synthesis of Grignard reagents

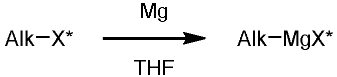

X* = halogen (Cl or Br)

Magnesium turnings (1.3 equiv.) were placed in a nitrogen flushed flask and dried for 5-10 min at elevated temperatures under vacuum. The flask was refilled with nitrogen and then cooled under nitrogen. After the addition of THF (1 ml per 1 mmol of the alkyl chloride or alkyl bromide, respectively) and 1,2-dibromoethane for activation, the alkyl chloride of one of Preparation Examples 1-9 or the alkyl bromide of Preparation Example 10 or 11 was added dropwise. Then, the reaction mixture was refluxed for another 2 h and depending on the conversion stirred for additional time under reflux or at 25°C to obtain the Grignard reagent Alk-MgX*.

### c) Synthesis of chlorosilane precursors

### Preparation Example 12

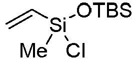

Dichloromethylvinylsilane (28.2 g, 200 mmol) was dissolved in DCM (200 ml) at 25°C. Triethylamine (22.3 g, 220 mmol, 1.1 equiv.) was added. Then TBSOH (26.5 g, 200 mmol, 1.0 equiv.) was added dropwise at 25°C. The reaction mixture was stirred at 25°C for 2.5 h. Then cyclohexane (100 ml) was added and the precipitate was filtered off. All volatiles were removed under reduced pressure and the residue was distilled in vacuo. **Preparation Example 12** was received as colorless liquid (34.2 g, 144 mmol, 72%). Purity 98% (GC). **Bp:** 103-105 °C (50 mbar). C₉H₂₁ClOSi₂, M_{w} = 236.89 g/mol.

The substance was directly submitted to the next conversion.

### Preparation Example 13

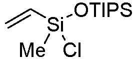

Dichloromethylvinylsilane (5.78 g, 41.0 mmol) was dissolved in DCM (40 ml) at 25°C. Triethylamine (4.56 g, 45.1 mmol, 1.1 equiv.) was added. Then TIPSOH (7.15 g, 41.0 mmol, 1.0 equiv.) was added dropwise at 25°C. The reaction mixture was stirred at 25°C for 16 h. Then cyclohexane (50 ml) was added and the precipitate was filtered off. All volatiles were removed under reduced pressure and the residue was distilled in vacuo. **Preparation Example 13** was received as colorless liquid (9.95 g, 35.7 mmol, 87%). Purity 99% (GC). **Bp:** 66-72 °C (0.83 mbar). C₁₂H₂₇ClOSi₂, M_{w} = 278.97 g/mol.

**GC-MS** (El, 70 eV): m/z (%) = 263 (M⁺-CH₃, 0.2), 235 (M⁺ -C₃H₇, 100), 207 (46), 193 (22), 179 (54), 165 (50), 153 (19), 139 (18), 123 (9), 105 (10).

The substance was directly submitted to the next conversion.

### Preparation Example 14

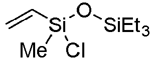

Dichloromethylvinylsilane (6.02 g, 45.5 mmol) was dissolved in DCM (45 ml) at 25°C. Triethylamine (5.06 g, 50.0 mmol, 1.1 equiv.) was added. Then TESOH (6.02 g, 45.5 mmol, 1.0 equiv.) was added dropwise at 25°C. The reaction mixture was stirred at 25°C for 16 h. Then cyclohexane (50 ml) was added and the precipitate was filtered off. All volatiles were removed under reduced pressure and the residue was distilled in vacuo. **Preparation Example 14** was received as colorless liquid (6.63 g, 28.0 mmol, 62%). Purity 96% (GC). C₉H₂₁ClOSi₂, M_{w} = 236,89 g/mol
**Bp:** 40-44 °C (0.6 mbar). **GC-MS** (El, 70 eV): m/z (%) = 221 (M⁺-CH₃, 0.2), 207 (M⁺ -C₂H₅, 100), 179 (45), 151 (41), 125 (17), 107 (7).

The substance was directly submitted to the next conversion.

### Preparation Example 15

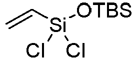

Trichlorovinylsilane (61.0 g, 378 mmol) was dissolved in DCM (300 ml) at 25°C. Triethylamine (38.2 g, 378 mmol, 1.0 equiv.) was added. Then TBSOH (50.0g, 378 mmol, 1.0 equiv.) dissolved in DCM (200 ml) was added dropwise at 0°C. The reaction mixture was stirred at 25°C for 16 h. Then cyclohexane (100 ml) was added and the precipitate was filtered off. All volatiles were removed under reduced pressure and the residue was distilled in vacuo. Preparation **Example 15** was received as colorless liquid (77.3 g, 300 mmol, 80%). Purity 99% (GC). C₈H₁₈Cl₂OSi₂, M_{w} = 257.30 g/mol.

**Bp:** 33-35 °C (0.2 mbar). **¹H NMR** (400 MHz, 20°C, CDCl₃): δ = 6.19 (dd, *J* = 7.6 Hz, *J* = 10.4 Hz, 1 H), 6.15-6.09 (m, 2 H), 0.92 (s, 9 H), 0.17 (s, 6 H) ppm. **¹³C NMR** (101 MHz, 20°C, CDCl₃): δ = 137.31 (CH₂), 131.82 (CH₃), 25.36 (3 CH₃), 17.97 (C), -3.39 (2 CH₃) ppm. **²⁹Si{H} NMR** (79.5 MHz, 20°C, CDCl₃): δ = 18.70 (1 Si), -34.89 (1 Si) ppm.

### d) Synthesis of modifying monomers

### Modifier S1

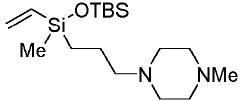

Chloro-tert-butyldimethylsiloxymethylvinylsilane **(Preparation Example 12,** 48.0 g, 203 mmol, 1.0 equiv.) was dissolved in THF (50 ml). A Grignard solution of **Preparation Example 1** (243 mmol, 1.2 equiv. 243 ml) in THF was added dropwise at 25°C. The reaction mixture was stirred for 18 h. After filtration, MTBE (50 ml) was added and the organic phase was washed with water (50 ml). The aqueous phase was extracted with MTBE (3 x 50 ml). The combined organic phases were dried over MgSO₄, the solids were filtered off and all volatiles were removed under reduced pressure. Modifier **S1** (37.7 g, 110 mmol, 54%) was received as a slightly yellow oil. C₁₇H₃₈N₂OSi₂, M_{w} = 342.67 g/mol.

**¹H NMR** (400 MHz, 20°C, CDCl₃): δ = 6.06 (dd, *J* = 20.0 Hz, *J* = 14.8 Hz, 1 H), 5.91 (dd, *J* = 14.8 Hz, *J* = 4.0 Hz, 1 H), 5.69 (dd, *J* = 20.4 Hz, *J* = 4.4 Hz, 1 H), 2.56-2.34 (m, br, 8 H), 2.33-2.29 (m, 2 H), 2.26 (s, 3 H), 1.53-1.45 (m, 2 H), 0.83 (s, 9 H), 0.55-0.51 (m, 2 H), 0.10 (s, 3 H), -0.01 (s, 6 H) ppm. **¹³C NMR** (101 MHz, 20°C, CDCl₃): δ = 138.42 (CH, vinyl), 132.04 (CH₂, vinyl), 62.03 (CH₂), 55.09 (2 CH₃), 53.11 (2 CH₂), 46.01 (CH₃), 25.64 (3 CH₃), 20.36 (CH₂), 18.01 (C), 14.75 (CH₂), -1.48 (CH₃), -2.89 (2 CH₃) ppm. **GC-MS** (El, 70 eV): m/z (%) = 342 (M⁺, 12), 327 (M⁺ -CH₃, 5), 315 (1), 285 (6), 133 (5), 113 (CH₃-C₂N₂H₇-CH₃⁺, 100). **IR** (ATR): λ⁻¹ = 3049 (w), 2931 (m), 2857 (m), 2793 (m), 1460 (m), 1252 (m), 1174 (m), 1052 (s), 1005 (s), 954 (m), 834 (s), 776 (vs), 686 (m) cm⁻¹.

### Modifier S2

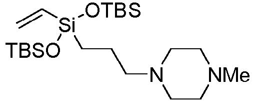

Chlorovinylsiloxysilane (27.3 g, 77.3 mmol, 1.0 equiv.) was dissolved in THF (50 ml). Then a solution of a freshly prepared Grignard solution of **Preparation Example 1** (85 mmol, 1.1 equiv. 85 ml) was added dropwise. The mixture was stirred at room temperature for 18 h. After filtration the solvent was removed under reduced pressure. **Modifier S2** (17.6 g, 38.4 mmol, 50%) was received as a slightly yellow oil. C₂₂H₅₀N₂O₂Si₃, M_{w} = 458.91 g/mol.

**¹H NMR** (400 MHz, 20°C, CDCl₃): δ = 6.01-5.92 (m, 2 H), 5.77 (dd, *J* = 16.0 Hz, *J* = 8.4 Hz, 1 H), 2.61-2.36 (m, br, 8 H), 2.35-2.31 (m, 2 H), 2.20 (s, 3 H), 1.55-1.51 (m, 2 H), 0.87 (s, 18 H), 0.55-0.51 (m, 2 H), 0.05 (s, 12 H) ppm. **¹³C NMR** (101 MHz, 20°C, CDCl₃): δ = 136.55 (CH, vinyl), 132.96 (CH₂, vinyl), 62.06 (CH₂), 55.18 (2 CH₂), 53.14 (2 CH₂), 46.10 (CH₃), 25.71 (6 CH₃), 20.10 (CH₂), 18.11 (2 C), 13.86 (CH₂), -2.85 (4 CH₃) ppm. **GC-MS** (El, 70 eV): m/z (%) = 458 (M⁺, 30), 443 (M⁺ -CH₃, 10), 401 (15), 388 (5), 205 (6), 191 (8), 113 (CH₂=Pip-CH₃⁺, 100), 98 (6), 70 (22). IR (ATR): λ⁻¹ = 3053 (w), 2952 (m), 2930 (m), 2857 (m), 2794 (m), 1461 (m), 1284 (m), 1252 (m), 1046 (s), 1004 (s), 957 (m), 831 (vs), 777 (vs), 717 (m), 677 (m) cm⁻¹.

### Modifier S3

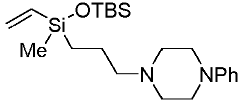

Chloro-tert-butyldimethylsiloxymethylvinylsilane **(Preparation Example 12,** 13.0 g, 55.0 mmol, 1.0 equiv.) was dissolved in THF (50 ml). A Grignard solution of **Preparation Example** 6 (55.0 mmol, 1.0 equiv. 55 ml) in THF was added dropwise at 25°C. The reaction mixture was stirred for 3 d. Water (50 ml) was added and the aqueous phase was extracted with MTBE (3 x 50 ml). The combined organic phases were dried over MgSO₄, the solids were filtered off and all volatiles were removed under reduced pressure. **Modifier S3** (18.9 g, 46.6 mmol, 85%) was received as a brown solid. C₂₂H₄₀N₂OSi₂, M_{w} = 404.75 g/mol.

**¹H NMR** (400 MHz, 20°C, CDCl₃): δ = 7.27-7.23 (m, 2 H), 6.93-6.91 (m, 2 H), 6.86-6.83 (m, 1 H), 6.10 (dd, *J* = 20.0 Hz, *J* = 14.8 Hz, 1 H), 5.96 (dd, *J* = 14.8 Hz, *J* = 4.0 Hz, 1 H), 5.74 (dd, *J =* 20.0 Hz, *J =* 4.4 Hz, 1 H), 3.23 (t, *J* = 5.2 Hz, 4 H), 2.63 (t, *J* = 5.0 Hz, 4 H), 2.44-2.40 (m, 2 H), 1.63-1.55 (m, 2 H), 0.87 (s, 9 H), 0.62-0.57 (m, 2 H), 0.15 (s, 3 H), 0.03 (s, 6 H) ppm. **¹³C NMR** (101 MHz, 20°C, CDCl₃): δ = 151.22 (C, Ar), 138.34 (CH, vinyl), 132.18 (CH₂, vinyl), 129.04 (2 CH), 119.67 (CH), 116.02 (2 CH), 61.91 (CH₂), 53.10 (2 CH₂), 48.94 (2 CH₂), 25.66 (3 CH₃), 20.23 (CH₂), 18.02 (C), 14.73 (CH₂), -1.44 (CH₃), -2.86 (2 CH₃) ppm. **²⁹Si{H} NMR** (79.5 MHz, 20°C, CDCl₃): δ = 10.53 (1 Si), -4.98 (1 Si) ppm. GC-MS (El, 70 eV): m/z (%) = 403 (M⁺, 0.8), 388 (M⁺ -CH₃, 0.5), 246 (0.8), 230 (2), 201 (2), 174 (CH₂-Pip-Bn⁺,100), 159 (7), 133 (7), 73 (14). IR (ATR): λ⁻¹ = 3050 (w), 2952 (m), 2929 (m), 2882 (w), 2856 (w), 2816 (w), 2772 (w), 1600 (m), 1501 (m), 1304 (w), 1252 (m), 1233 (m), 1056 (s), 1004 (m), 954 (m), 953 (m), 834 (s), 777 (vs), 689 (s) cm⁻¹.

### Modifier S4

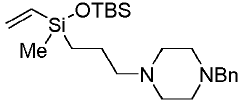

Chloro-tert-butyldimethylsiloxymethylvinylsilane **(Preparation Example 12,** 10.7 g, 45.0 mmol, 1.0 equiv.) was dissolved in THF (50 ml). A Grignard solution of 1-benzyl-4-(3-chloropropyl)piperazine ( CAS: 23145-99-5, commercial available @ eg. Chemieliva Pharmaceuticals) (45.0 mmol, 1.0 equiv. 45 ml) in THF was added dropwise at 25°C. The reaction mixture was stirred for 3 d. Water (50 ml) was added and the aqueous phase was extracted with MTBE (3 x 50 ml). The combined organic phases were dried over MgSO₄, the solids were filtered off and all volatiles were removed under reduced pressure. Modifier S4 (14.5 g, 34.6 mmol, 77%) was received as a brown oil which solidified after some time at 25°C. C₂₃H₄₂N₂OSi₂, M_{w} = 418.77 g/mol.

**¹H NMR** (400 MHz, 20°C, CDCl₃): δ = 7.31-7.23 (m, 5 H), 6.08 (dd, *J* = 20.0 Hz, *J* = 14.8 Hz, 1 H), 5.94 (dd, *J* = 14.8 Hz, *J =* 4.0 Hz, 1 H), 5.72 (dd, *J* = 20.4 Hz, *J* = 4.0 Hz, 1 H), 3.52 (s, 2 H), 2.62-2.42 (s, br, 8 H), 2.39-2.35 (m, 2 H), 1.58-1.49 (m, 2 H), 0.85 (s, 9 H), 0.57-0.53 (m, 2 H), 0.12 (s, 3 H), 0.01 (s, 6 H) ppm. **¹³C NMR** (101 MHz, 20°C, CDCl₃): δ = 138.39 (CH, vinyl), 137.96 (C, Ar), 132.15 (CH₂, vinyl), 129.23 (2 CH), 128.19 (2 CH), 127.03 (CH), 63.01 (2 CH₂), 61.95 (CH₂), 53.06 (2 CH₂), 52.80 (CH₂), 25.66 (3 CH₃), 20.21 (CH₂), 18.04 (C), 14.76 (CH₂), -1.55 (CH₃), -2.86 (2 CH₃) ppm. **²⁹Si{H} NMR** (79.5 MHz, 20°C, CDCl₃): δ = 10.46 (1 Si), -5.01 (1 Si) ppm. **GC-MS** (El, 70 eV): m/z (%) = 418 (M⁺, 10), 403 (M⁺ -CH₃, 5), 361 (5), 327 (1), 244 (3), 189 (C₁₂H₁₇N₂⁺, 100), 161 (3), 133 (4), 119 (4), 91 (C₇H₇⁺, 22). **IR** (ATR): λ⁻¹ = 3028 (w), 2952 (m), 2930 (m), 2806 (m), 1460 (m), 1406 (w), 1252 (m), 1057 (s), 1006 (s), 954 (m), 833 (s), 777 (vs), 736 (s), 697 (s) cm⁻¹.

### Modifier S5

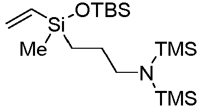

Chloro-tert-butyldimethylsiloxymethylvinylsilane **(Preparation Example 12,** 25 g, 106 mmol, 1.0 equiv.) was dissolved in THF (50 ml). A Grignard solution of **Preparation Example 3** (127 mmol, 1.2 equiv. 127 ml) in THF was added dropwise at 25°C. The reaction mixture was stirred for 16 h at 60°C. The solids were filtered off and all volatiles were removed under reduced pressure. **Modifier S5** (33.6 g, 83.1 mmol, 79%) was received after distillation in vacuo as a colorless liquid. C₁₈H₄₅NOSi₄, M_{w} = 403.90 g/mol.

**bp** = 85-95°C (0.2 mbar). **¹H NMR** (400 MHz, 20°C, C₆D₆): δ = 6.17 (dd, *J* = 20.4 Hz, *J* = 14.8 Hz, 1 H), 5.93 (dd, *J* = 14.9 Hz, *J =* 4.0 Hz, 1 H), 5.77 (dd, *J* = 20.4 Hz, *J* = 4.0 Hz, 1 H), 2.83-2.78 (m, 2 H), 1.59-1.51 (m, 2 H), 1.40 (s, 3 H), 0.96 (s, 9 H), 0.54-0.49 (m, 2 H), 0.19 (s, 18 H), 0.10 (s, 6 H) ppm. **¹³C NMR** (101 MHz, 20°C, C₆D₆): δ = 138.69 (CH, vinyl), 132.40 (CH₂, vinyl), 49.62 (CH₂), 29.45 (CH₂), 25.90 (3 CH₃), 18.34 (C), 14.86 (CH₂), 2.31 (6 CH₃), - 1.24 (CH₃), -2.61 (2 CH₃) ppm. **²⁹Si{H} NMR** (79.5 MHz, 20°C, CDCl₃): δ = 10.43 (1 Si), 4.46 (2 Si), -4.99 (1 Si) ppm. **GC-MS** (EI, 70 eV): m/z (%) = 403 (M⁺, 0.8), 388 (M⁺ -CH₃, 0.5), 246 (0.8), 230 (2), 201 (2), 174 (100), 159 (7), 133 (7), 73 (14). **IR** (ATR): λ⁻¹ = 3051 (w), 2954 (m), 2931 (m), 2895 (w), 2858 (w), 1595 (w), 1406 (w), 1250 (s), 1184 (m), 1065 (s), 1005 (m), 955 (m), 907 (s), 875 (m), 833 (vs), 777 (s), 678 (m) cm⁻¹.

### Modifier S6

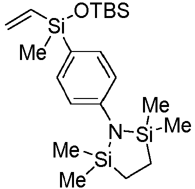

Chloro-tert-butyldimethylsiloxymethylvinylsilane **(Preparation Example 12,** 15.0 g, 63.8 mmol, 1.0 equiv.) was dissolved in THF (30 ml). A Grignard solution of **Preparation Example 11** (76.5 mmol, 1.2 equiv., 76.5 ml) in THF was added dropwise at 25°C. The reaction mixture was stirred for 16 h at 50°C. Cyclohexan was added (50 ml). The solids were filtered off and all volatiles were removed under reduced pressure. **Modifier S6** (18.6 g,42.7 mmol, 67%) was received after distillation in vacuo as a brownish oil. C₂₁H₄₁NOSi₄.

**¹H NMR** (400 MHz, 20°C, C₆D₆): δ = 7.58 (d, *J* = 8.4 Hz, 2 H), 7.04 (d, *J* = 8.4 Hz, 2 H), 6.33 (dd, *J* = 20.0 Hz, *J* = 14.8 Hz, 1 H), 6.00 (dd, *J* = 14.8 Hz, *J* = 3.6 Hz, 1 H), 5.88 (dd, *J* = 20.4 Hz, *J* = 4.0 Hz, 1 H), 0.95 (s, 9 H), 0.79 (s, 4 H), 0.20 (s, 12 H), 0.17 (s, 3 H), 0.08 (s, 6 H) ppm. **¹³C NMR** (101 MHz, 20°C, C₆D₆): δ = 149.40 (C, Ar), 138.72 (CH, vinyl), 135.13 (2 CH, Ar), 133.22 (CH₂, vinyl), 128.84 (C, Ar), 122.92 (2 CH, Ar), 25.97 (3 CH₃), 18.42 (C), 8.74 (2 CH₂), 0.07 (4 CH₃), -0.58 (CH₃), -2.62 (CH₃), -2.68 (CH₃) ppm. **²⁹Si{H} NMR** (79.5 MHz, 20°C, C₆D₆): δ = 12.61 (2 Si), 11.14 (1 Si), -14.08 (1 Si) ppm. **GC-MS** (El, 70 eV): m/z (%) = 435 (M⁺, 83), 420 (M⁺ -CH₃, 5), 378 (65), 350 (4), 304 (17), 290 (100), 278 (23), 262 (5), 230 (40), 218 (10), 192 (5), 181 (37), 168 (10), 147 (22), 133 (40), 73 (55). **IR** (ATR): λ⁻¹ = 3013 (w), 2955 (m), 2928 (w), 2856 (w), 1593 (m), 1502 (m), 1405 (w), 1251 (m), 1117 (m), 1051 (m, br), 1005 (m), 955 (m), 922 (m), 834 (m), 777 (vs), 687 (m), 627 (m) cm⁻¹.

### Modifier S7

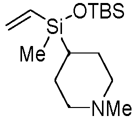

Chloro-tert-butyldimethylsiloxymethylvinylsilane **(Preparation Example 12,** 9.11 g, 38.5 mmol, 1.0 equiv.) was dissolved in THF (20 ml). A Grignard solution of 4-chloro-1-mehyl-piperidine (CAS 5570-77-4, commercially available @ e.g. abcr GmbH, Karlsruhe)(50 mmol, 1.3 equiv. 50 ml) in THF was added dropwise at 25°C. The reaction mixture was stirred for 16 h at 25°C. Water (50 ml) was added and the aqueous phase was extracted with MTBE (2 x 50 ml). The combined organic phases were dried over MgSO₄, the solids were filtered off and all volatiles were removed under reduced pressure. **Modifier S7** (7.81 g, 26.1 mmol, 68%) was received after distillation in vacuo as a yellow oil.C₁₅H₃₃NOSi₂, M_{w} = 299.61 g/mol.

**¹H NMR** (400 MHz, 20°C, C₆D₆): δ = 6.12 (dd, *J* = 20.4 Hz, *J* = 15.2 Hz, 1 H), 5.95 (dd, *J =* 14.8 Hz, *J* = 4.0 Hz, 1 H), 5.76 (dd, *J* = 20.4 Hz, *J =* 4.0 Hz, 1 H), 2.84-2.81 (m, 2 H), 2.16 (s, 3 H), 1.78-1.70 (m, 2 H), 1.67-1.60 (m, 4 H), 0.95 (s, 9 H), 0.59-0.51 (m, 1 H), 0.14 (s, 3 H), 0.00 (s, 6 H) ppm. **¹³C NMR** (101 MHz, 20°C, C₆D₆): δ = 137.60 (CH, vinyl), 132.89 (CH₂, vinyl), 57.73 (2 CH₂), 47.21 (CH₃), 26.70 (CH₂), 26.65 (CH₂), 25.96 (3 CH₃), 24.65 (CH), 18.36 (C), -2.61 (2 CH₃), -3.36 (CH₃) ppm. **GC-MS** (El, 70 eV): m/z (%) = 299 (M⁺, 19), 284 (M⁺ -CH₃, 58), 270 (6), 256 (2), 242 (33), 188 (8), 161 (14), 147 (20), 133 (29), 119 (13), 96 (C₅H₇NCH₃⁺, 100), 73 (12). **IR** (ATR): λ⁻¹ = 3050 (w), 2954 (m), 2929 (m), 2777 (w), 2675 (w), 1463 (w), 1376 (w), 1252 (m), 1202 (w), 1142 (w), 1050 (s), 1005 (s), 954 (m), 888 (m), 834 (s), 776 (vs), 685 (m) cm⁻¹.

### Modifier S8

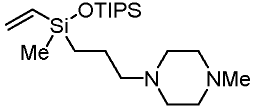

Chloro-triisopropylsiloxymethylvinylsilane **(Preparation Example 13,** 9.39 g, 35.6 mmol, 1.0 equiv.) was dissolved in THF (10 ml). A Grignard solution of **Preparation Example 1** (39 mmol, 1.1 equiv., 40 ml) in THF was added dropwise at 25°C. The reaction mixture was stirred for 16 h at 25°C. Water (50 ml) was added and the aqueous phase was extracted with MTBE (2 x 50 ml). The combined organic phases were dried over MgSO₄, the solids were filtered off and all volatiles were removed under reduced pressure. **Modifier S8** (6.65 g, 25.6 mmol, 72%) was received after distillation in vacuo as a brownish solid. C₂₀H₄₄N₂OSi₂, M_{w} = 384.76 g/mol.

**¹H NMR** (400 MHz, 20°C, C₆D₆): δ = 6.23 (dd, *J* = 20.4 Hz, *J* = 15.2 Hz, 1 H), 5.96 (dd, *J =* 14.8 Hz, *J* = 3.6 Hz, 1 H), 5.80 (dd, *J* = 20.0 Hz, *J* = 3.6 Hz, 1 H), 2.46 (s, br, 4 H), 2.36 (s, br, 4 H), 2.31-2.28 (m, 2 H), 2.11 (s, 3 H), 1.63 (pent, *J* = 6.4 Hz, 2 H), 1.09 (d, *J* = 6.4 Hz, 18 H), 1.02 (hept, *J* = 6.4 Hz, 3 H ), 0.76-0.71 (m, 2 H), 0.26 (s, 3 H) ppm. **¹³C NMR** (101 MHz, 20°C, C₆D₆): δ = 138.67 (CH, vinyl), 131.98 (CH₂, vinyl), 61.54 (CH₂), 54.94 (2 CH₂), 52.84 (2 CH₂), 45.56 (CH₃), 20.71 (CH₂), 17.81 (6 CH₃), 14.82 (CH₂), 12.78 (3 CH), -1.51 (CH₃) ppm. **GC-MS** (El, 70 eV): m/z (%) = 384 (M⁺, 31), 341 (M⁺ -C₃H₇, 38), 314 (4), 284 (2), 157 (5), 145 (8), 131 (8), 113 (C₆H₁₃N₂⁺, 100). **IR** (ATR): λ⁻¹ = 3049 (w), 2940 (m), 2865 (m), 2794 (w), 1461 (m), 1253 (m), 1082 (s), 1055 (vs), 1010 (s), 862 (m), 806 (m), 788 (m), 676 (s) cm⁻¹.

### Modifier S9

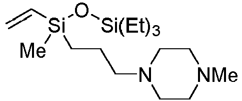

Chloro-triethylsiloxymethylvinylsilane **(Preparation Example 14,** 6.61 g, 27.9 mmol, 1.0 equiv.) was dissolved in THF (10 ml). A Grignard solution of **Preparation Example 1** (31 mmol, 1.1 equiv., 31 ml) in THF was added dropwise at 25°C. The reaction mixture was stirred for 16 h at 25°C. Water (30 ml) was added and the aqueous phase was extracted with MTBE (3 x 30 ml). The combined organic phases were dried over MgSO₄, the solids were filtered off and all volatiles were removed under reduced pressure. **Modifier S9** (9.13 g, 24.8 mmol, 89%) was received after distillation in vacuo as an orange oil. C₁₇H₃₈N₂OSi₂, M_{w} = 342.67 g/mol.

**¹H NMR** (400 MHz, 20°C, CDCl₃): δ = 6.09 (dd, *J* = 20.4 Hz, *J* = 15.2 Hz, 1 H), 5.94 (dd, *J =* 14.8 Hz, *J* = 4.4 Hz, 1 H), 5.72 (dd, *J* = 20.4 Hz, *J* = 4.0 Hz, 1 H), 2.55-2.36 (m, br, 8 H), 2.35-2.31 (m, 2H), 2.28 (s, 3 H), 1.56-1.47 (m, 2 H), 0.92 (t, *J* = 8.0 Hz, 9 H), 0.58-0.54 (m, 2 H), 0.51 (q, *J* = 8.0 Hz, 6 H), 0.13 (s, 3 H) ppm. **¹³C NMR** (101 MHz, 20°C, CDCl₃) δ = 138.56 (CH, vinyl), 131.97 (CH₂, vinyl), 62.08 (CH₂), 55.15 (2 CH₂), 53.17 (2 CH₂), 46.06 (CH₃), 20.42 (CH₂), 14.84 (CH₂), 6.74 (3 CH₃), 6.28 (3 CH₂), -1.45 (CH₃) ppm. **²⁹Si{H} NMR** (79.5 MHz, 20°C, CDCl₃): δ = 10.10 (1 Si), -5.41 (1 Si) ppm. **GC-MS** (El, 70 eV): m/z (%) = 342 (M⁺, 11), 313 (M⁺ -CH₃, 14), 145 (4), 113 (C₆H₁₃N₂⁺, 100), 70 (17). **IR** (ATR): λ⁻¹ = 3050 (w), 2952 (m), 2937 (m), 2875 (m), 2793 (m), 2765 (w), 1594 (w), 1458 (m), 1406 (w), 1296 (m), 1252 (m), 1174 (m), 1120 (m), 1060 (s), 1008 (s), 954 (m), 807 (m), 790 (m), 740 (s), 725 (vs), 671 (m) cm⁻¹.

### Modifier S10

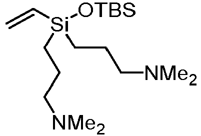

Dichloro-tert-butyldimethylsiloxyvinylsilane **(Preparation Example 15,** 12.3 g, 47.6 mmol, 1.0 equiv.) was dissolved in THF (25 ml). A Grignard solution of 3-chloro-N,N-dimethylpropan-1-amine (100 mmol, 2.1 equiv., 100 ml) in THF was added dropwise at 25°C. The reaction mixture was stirred for 1 h at 25°C. Water (100 ml) was added and the aqueous phase was extracted with MTBE (3 x 50 ml). The combined organic phases were dried over MgSO₄, the solids were filtered off and all volatiles were removed under reduced pressure. **Modifier S10** (14.4 g, 40.0 mmol, 61%) was received after distillation in vacuo as a colorless oil.

**bp** = 129-131°C (0.02 mbar). **¹H NMR** (400 MHz, 20°C, C₆D₆): δ = 6.16 (dd, *J* = 20.4 Hz, *J* = 15.2 Hz, 1 H), 5.96 (dd, *J* = 15.2 Hz, *J* = 4.0 Hz, 1 H), 5.82 (dd, *J* = 20.4 Hz, *J* = 4.0 Hz, 1 H), 2.22 (t, *J* = 7.2 Hz, 4 H), 2.13 (s, 12 H), 1.66-1.58 (m, 4 H), 0.96 (s, 9 H), 0.76-0.72 (m, 4 H), 0.11 (s, 6 H) ppm. **¹³C NMR** (101 MHz, 20°C, C₆D₆): δ = 138.00 (CH, vinyl), 132.70 (CH₂, vinyl), 63.33 (2 CH₂), 45.58 (4 CH₃), 25.99 (3 CH₃), 21.85 (2 CH₂), 18.42 (C), 13.41(2 CH₂), - 2.54 (2 CH₃) ppm **GC-MS** (El, 70 eV): m/z (%) = 358 (M⁺, 0.4), 343 (M⁺ -CH₃, 3), 314 (25), 301 (M⁺-C₄H₉, 14), 274 (65), 230 (5), 145 (7), 119 (5), 103 (4), 84 (10), 58 (C₃H₈N⁺, 100). **IR** (ATR): λ⁻¹ = 3050 (w), 2930 (m), 2856 (m), 2814 (m), 2763 (m), 1463 (m), 1405 (m), 1252 (m), 1181 (m), 1059 (s), 1041 (s), 1005 (m), 953 (m), 833 (s), 777 (vs), 676 (m) cm⁻¹.

### Modifier S11

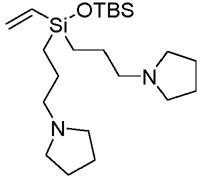

Dichloro-tert-butyldimethylsiloxyvinylsilane **(Preparation Example 15, 3.44** g, 20.0 mmol, 1.0 equiv.) was dissolved in THF (13 ml). A Grignard solution of **Preparation Example 7 (46.0** mmol, 2.3 equiv., 46 ml) in THF was added dropwise at 25°C. The reaction mixture was stirred for 16 h at 25°C. Cyclohexane (50 ml) was added and the solids were filtered off. All volatiles were removed under reduced pressure. **Modifier S11** (4.15 g, 10.1 mmol, 51%) was received as a colorless oil. C₂₂H₄₆N₂OSi₂, M_{w} = 410.79 g/mol.

**¹H NMR** (400 MHz, 20°C, CDCl₃): δ = 6.19 (dd, *J* = 20.0 Hz, *J* = 14.8 Hz, 1 H), 5.97 (dd, *J* = 15.0 Hz, *J* = 4.0 Hz, 1 H), 5.84 (dd, *J* = 20.4 Hz, *J* = 4.4 Hz, 1 H), 2.48-2.43 (m, 12 H), 1.75-1.67 (m, 4 H), 1.65-1.58 (m, 8 H), 0.96 (s, 9 H), 0.82-0.78 (m, 4 H), 0.12 (s, 6 H) ppm. **¹³C NMR** (101 MHz, 20°C, CDCl₃): δ = 138.03 (CH, vinyl), 132.72 (CH₂, vinyl), 59.95 (2 CH₂), 54.30 (4 CH₂), 26.02 (3 CH₃), 23.93 (4 CH₂), 23.14 (2 CH₂), 18.43 (C), 13.70(2 CH₂), -2.51 (2 CH₃) ppm. **²⁹Si{H} NMR** (79.5 MHz, 20°C, CDCl₃): δ = 10.01 (1 Si), -4.52 (1 Si) ppm. **GC-MS** (El, 70 eV): m/z (%) = 409 (M⁺, 0.1), 395 (M⁺ -CH₃, 4), 383 (2), 353 (7), 340 (16), 326 (4), 300 (60), 298 (57), 270 (3), 242 (2), 110 (9), 84 (C₅H₁₀N⁺, 100). **IR** (ATR): λ⁻¹ = 3049 (w), 2954 (m), 2928 (m), 2780 (m), 2736 (w), 1594(w), 1461 (m), 1405 (m), 1351 (m), 1251 (m), 1139 (m), 1065 (s), 1005 (s), 953 (m), 833 (s), 776 (vs), 676 (m) cm⁻¹.

### Modifier S12

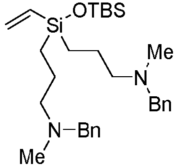

Dichloro-tert-butyldimethylsiloxyvinylsilane **(Preparation Example 15,** 9.00 g, 35.0 mmol, 1.0 equiv.) was dissolved in THF (30 ml). A Grignard solution of **Preparation Example 5** (75 mmol, 2.14 equiv., 75 ml) in THF was added dropwise at 25°C. The reaction mixture was stirred for 1 h at 25°C. Water (50 ml) was added and the aqueous phase was extracted with MTBE (3 x 50 ml). The combined organic phases were dried over MgSO₄, the solids were filtered off and all volatiles were removed under reduced pressure. **Modifier S12** (16.4 g, 32.2 mmol, 92%) was received as a yellow oil. C₃₀H₅₀N₂OSi₂, M_{w} = 510.91 g/mol.

**¹H NMR** (400 MHz, 20°C, CDCl₃): δ = 7.34-7.24 (m, 10 H), 6.06-5.95 (m, 2 H), 5.73 (dd, *J* = 19.6 Hz, *J =* 4.8 Hz, 1 H), 3.52 (s, 4 H), 2.41-2.37 (m, 4 H), 2.21 (s, 6 H), 1.61-1.52 (m, 4 H), 0.87 (s, 9 H), 0.60-0.56 (m, 4 H), 0.03 (s, 6 H) ppm. **¹³C NMR** (101 MHz, 20°C, CDCl₃): δ = 138.42 (2 C, Ar), 137.18 (CH, vinyl), 132.73 (CH₂, vinyl), 129.17 (4 CH, Ar), 128.20 (4 CH, Ar), 127.03 (2 CH, Ar), 62.04 (2 CH₂), 60.60 (2 CH₂), 41.94 (2 CH₃), 25.71 (3 CH₃), 20.60 (2 CH₂), 18.11 (C), 12.98 (2 CH₂), -2.77 (2 CH₃) ppm. **²⁹Si{H} NMR** (79.5 MHz, 20°C, CDCl₃) δ = 10.37 (1 Si), -5.28 (1 Si) ppm. **GC-MS** (El, 70 eV): m/z (%) = 510 (M⁺, 0.5), 495 (M⁺ -CH₃, 2), 453 (2), 419 (8), 390 (5), 350 (18), 287 (5), 200 (3), 134 (C₉H₁₂N⁺, 100), 91 (C₇H₇⁺, 72). **IR** (ATR): λ⁻¹ = 3062 (w), 3028 (w), 2951 (m), 2928 (m), 2856 (m), 2785 (m), 1454 (m), 1252 (m), 1182 (w), 1053 (s, br), 1005 (s), 955 (s), 833 (s), 777 (s), 697 (vs) cm⁻¹.

### Modifier S13

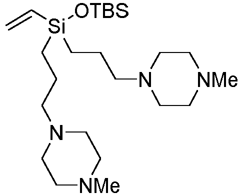

Dichloro-tert-butyldimethylsiloxyvinylsilane **(Preparation Example 15,** 28.1 g, 109 mmol, 1.0 equiv.) was dissolved in THF (75 ml). A Grignard solution of **Preparation Example 1** (230 mmol, 2.11 equiv., 230 ml) in THF was added dropwise at 25°C. The reaction mixture was stirred for 16 h at 25°C. Water (100 ml) was added and the aqueous phase was extracted with MTBE (3 x 100 ml). The combined organic phases were dried over MgSO₄, the solids were filtered off and all volatiles were removed under reduced pressure. **Modifier S13** (38.2 g, 81 mmol, 74%) was received as a yellow oil. C₂₄H₅₂N₄OSi₂, M_{w} = 468.88 g/mol.

**¹H NMR** (400 MHz, 20°C, CDCl₃): δ = 6.03 (dd, *J* = 19.6 Hz, *J* = 14.2 Hz, 1 H), 5.94 (dd, *J* = 15.2 Hz, *J* = 4.4 Hz, 1 H), 5.71 (dd, *J* = 19.6 Hz, *J* = 4.8 Hz, 1 H), 2.58-2.31 (m, 16 H), 2.32-2.28 (m, 4 H), 2.26 (s, 6 H), 1.53-1.44 (m, 4 H), 0.83 (s, 9 H), 0.57-0.53 (m, 4 H), 0.00 (s, 6 H) ppm. **¹³C NMR** (101 MHz, 20°C, CDCl₃): δ = 137.16 (CH, vinyl), 132.69 (CH₂, vinyl), 62.14 (2 CH₂), 55.12 (4 CH₂), 53.16 (4 CH₂), 46.05 (2 CH₃), 25.69 (3 CH₃), 20.31 (2 CH₂), 18.09 (C), 13.13 (2 CH₂), -2.78 (2 CH₃) ppm. **GC-MS** (EI, 70 eV): m/z (%) = 468 (M⁺, 59), 453 (M⁺ -CH₃, 16), 438 (5), 424 (21), 396 (16), 329 (16), 270 (10), 145 (6), 113 (CH₃-NC₄H₇N-CH₃⁺, 100), 98 (22), 70 (55). **IR** (ATR): λ⁻¹ = 3049 (w), 2933 (m), 2876 (m), 2792 (s), 1458 (m), 1283 (m), 1252 (m), 1174 (m), 1163 (m), 1052 (s), 1011 (s), 833 (s), 776 (vs), 675 (m) cm⁻¹.

### Modifier S14

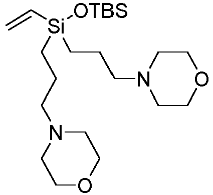

Dichloro-tert-butyldimethylsiloxyvinylsilane **(Preparation Example 15,** 40.8 g, 159 mmol, 1.0 equiv.) was dissolved in THF (110 ml). A Grignard solution of **Preparation Example 4** (333 mmol, 2.1 equiv., 333 ml) in THF was added dropwise at 25°C. The reaction mixture was stirred for 16 h at 25°C. Hexanes (300 ml) were added, the solids were filtered off and all volatiles were removed under reduced pressure. **Modifier S14** (44.7 g, 101 mmol, 64%) was received as a yellow oil. C₂₂H₄₆N₂O₃Si₂, M_{w} = 442.79 g/mol.

**¹H NMR** (400 MHz, 20°C, CDCl₃): δ = 6.06 (dd, *J* = 19.6 Hz, *J* = 15.2 Hz, 1 H), 5.97 (dd, *J* = 15.2 Hz, *J* = 4.8 Hz, 1 H), 5.74 (dd, *J* = 19.6 Hz, *J* = 4.8 Hz, 1 H), 3.72-3.70 (m, 8 H), 2.43-2.40 (m, 8H), 2.33-2.29 (m, 4 H), 1.56-1.47 (m, 4 H), 0.86 (s, 9 H), 0.61-0.57 (m, 4 H), 0.03 (s, 6 H) ppm. **¹³C NMR** (101 MHz, 20°C, CDCl₃): δ = 137.09 (CH, vinyl), 132.79 (CH₂, vinyl), 66.96 (4 CH₂), 62.52 (2 CH₂), 53.71 (4 CH₂), 25.69 (3 CH₃), 20.05 (2 CH₂), 18.11 (C), 13.06 (2 CH₂), -2.77 (2 CH₃) ppm. **²⁹Si{H} NMR** (79.5 MHz, 20°C, CDCl₃): δ = 10.40 (1 Si), -5.44 (1 Si) ppm. **GC-MS** (El, 70 eV): m/z (%) = 442 (M⁺, 46), 427 (M⁺ -CH₃, 5), 412 (17), 385 (-C₄H₉, 10), 316 (30), 314 (30), 286 (25), 230 (4), 184 (4), 145 (11), 100 (100). **IR** (ATR): λ⁻¹ = 3049 (w), 2953 (m), 2928 (m), 2855 (m), 2804 (m), 1462 (m), 1359 (w), 1252 (m), 1118 (s), 1056 (s), 1004 (s), 868 (m), 833 (s), 777 (vs), 675 (m) cm⁻¹.

### e) Preparation of Reference Compounds

### Reference Compound C1

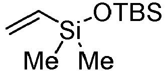

Chlorodimethylvinylsilane (11.2 g, 92.4 mmol, 1.0 equiv.) was added dropwise to a solution of tertbutyldimethylsilanol (12.2 g, 92.4 mmol, 1.0 equiv.) and triethylamine (10.3 g, 102 mmol, 1.1 equiv.) in cyclohexane (100 ml) at room temperature. The mixture was stirred at room temperature for 18 h. After filtration the solvent was removed under reduced pressure and distillation at 15 mbar furnished the vinylsilane compound of **Reference Compound C1** (14.6 g, 67.4 mmol, 73%) as a colorless liquid. C₁₀H₂₄OSi₂, M_{w} = 216.47 g/mol.

**bp** = 49-51°C (15 mbar). **¹H NMR** (400 MHz, 20°C, C₆D₆): δ = 6.17 (dd, *J* = 20.3 Hz, *J* = 14.8 Hz, 1 H), 5.89 (dd, *J* = 14.9 Hz, *J* = 4.0 Hz, 1 H), 5.75 (dd, *J* = 20.3 Hz, *J* = 4.0 Hz, 1 H), 0.94 (s, 9 H), 0.17 (s, 6 H), 0.06 (s, 6 H) ppm. **¹³C NMR** (101 MHz, 20°C, C₆D₆): δ = 139.75 (CH, vinyl), 131.79 (CH₂, vinyl), 25.89 (3 CH₃), 18.28 (C), 0.55 (2 CH₃), -2.71 (2 CH₃) ppm. GC-**MS** (El, 70 eV): m/z (%) = 216 (M⁺, 0.1), 201 (M⁺ -CH₃, 3), 159 (M⁺ *-tert*Bu, 100), 131 (11), 145 (100), 73 (12).

### Reference Compound C2

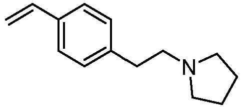

An isomeric mixture of divinylbenzene in ethylstyrene (122 g, 750 mmol, 1.0 equiv. 63%) was dissolved in cyclohexane (375 ml). Pyrrolidine (750 mmol, 1.0 equiv.) was added at 25°C. Then TMEDA (4.36 g, 37.5 mmol, 0.05 equiv.) and n-butyllithium solution in hexanes (15 ml, 37.5 mmol, 0.05 equiv.) were added. The reaction mixture was stirred for 16 h at 25°C. The reaction mixture was washed with water (400 ml) three times. After drying over MgSO₄, the solids were filtered off and the solvent was removed under reduced pressure. An isomeric mixture of the vinyl compound of **Reference Compound C2** (40.5 g, 201 mmol, 27%) was received after distillation *in vacuo* as a colorless oil. C₁₄H₁₉N, M_{w} = 201.31 g/mol.

### bp = 105-106°C (0.25 mbar).

### Reference Compound C3

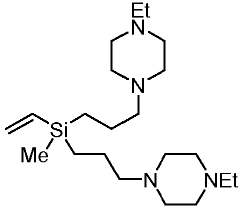

Dichloromethylvinylsilane (4.23 g, 30 mmol, 1.0 equiv.) was dissolved in THF (30 ml). Grignard solution (60 mmol, 2.0 equiv. 60 ml) in THF was added dropwise at 25°C. The reaction mixture was stirred for 18 h. After filtration, cyclohexane (50 ml) was added and the organic phase was washed with water (50 ml, pH=14). The organic phase was dried over MgSO₄, the solids were filtered off and all volatiles were removed under reduced pressure. The compound of **Reference Compound C3** (10.3 g, 27.0 mmol, 90%) was received as a slightly yellow oil. C₂₁H₄₄N₄Si, M_{w} = 380.70 g/mol.

**¹H NMR** (400 MHz, 20°C, CDCl₃): δ = 6.10 (dd, *J* = 20.0 Hz, *J* = 14.8 Hz, 1 H), 5.96 (dd, *J* = 14.8 Hz, *J* = 4.0 Hz, 1 H), 5.67 (dd, *J* = 20.0 Hz, *J* = 4.0 Hz, 1 H), 2.70-2.35 (m, br, 16 H), 2.41 (q, *J* = 7.2 Hz, 4 H), 2.34-2.30 (m, 4 H), 1.53-1.43 (m, 4 H), 1.08 (t, *J* = 7.2 Hz, 6 H), 0.56-0.52 (m, 4 H), 0.05 (s, 3 H) ppm. **¹³C NMR** (101 MHz, 20°C, CDCl₃): δ = 137.61 (CH, vinyl), 132.26 (CH₂, vinyl), 62.31 (2 CH₂), 53.22 (4 CH₂), 52.78 (4 CH₂), 52.31 (2 CH₂), 21.10 (2 CH₂), 11.95 (2 CH₃), 11.48 (2 CH₂), -5.51 (CH₃) ppm. **²⁹Si{H} NMR** (79.5 MHz, 20°C, CDCl₃): δ = -4.90 (1 Si) ppm. **GC-MS** (El, 70 eV): m/z (%) = 380 (M⁺, 51), 365 (M⁺ -CH₃, 10), 351 (3), 336 (2), 322 (12), 310 (5), 296 (13), 267 (2), 225 (13), 154 (9), 140 (8), 127 (C₂H₅-C₄N₂H₇-CH₃⁺, 100), 112 (15), 98 (10), 84 (31). **IR** (ATR): λ⁻¹ = 3045 (w), 2967 (m), 2934 (m), 2874 (m), 2805 (s), 2765 (m), 1449 (m), 1346 (m), 1304 (m), 1252 (m), 1170 (s), 1162 (s), 1119 (s), 1009 (s), 946 (m), 797 (s), 775 (s), 745 (s), 706 (m) cm⁻¹.

### 2. Syntheses of Polymers

### General polymerization procedure:

Cyclohexane, butadiene and styrene (amounts given in the tables) were charged to an air-free 10 L reactor and the stirred mixture was heated up to 40 °C. Then TMEDA (amount is given in the tables) and a modifying monomer (amount and type of modifier is given in tables) were added and n-butyllithium (n-BuLi) was charged dropwise to react the impurities until the color of the reaction mixture changed to yellowish (titration). Subsequently, the desired amount of initiator in cyclohexane corresponding to the target molecular weight of the polymer was charged immediately to start the polymerization. The start time of the charge of the initiator was used as the start time of the polymerization. In parallel, the temperature was increased by heating or cooling in the wall of the reactors beginning with the charge of the initiator to the final polymerization temperature. The final temperature was hold till full conversion was detected. Then butadiene (1.7-2% of amount given in tables) was charged. If indicated in the table, the chain-end modifier dimethoxydimethylsilane (DMDM, amount) was added after 5 minutes. The reaction was terminated after 20 min by addition of methanol. Then the polymer solution was stabilized with Irganox 1520D, the polymer recovered by steam stripping and dried until a content of residual volatiles < 0.6% was obtained. The complete data set of the sample is given in the tables below.

In the following, the polymers which are in accordance with the present invention are referred to as Examples (abbreviated as "Ex."). Polymers which are not in accordance with the present invention, because they were prepared in absence of a modifying monomer are referred to as Reference Examples (abbreviated as "REx."), and polymers which are not in accordance with the present invention, because they were prepared in presence of a modifying monomer not in accordance with the present invention are referred to as Comparative Examples (abbreviated as "CEx.").

**Table 1: Polymerization 1.**

| | **Ex. A** | **CEx. A** | **Ex. B** | **Ex. C** | **Ex. D** | **Ex. E** |
|---|---|---|---|---|---|---|
| Cyclohexane /g | 4569 | 4566 | 4374 | 4373 | 4374 | 4373 |
| Butadiene total/g | 609.5 | 610.9 | 693.9 | 693.8 | 693.9 | 693.8 |
| Butadiene initial /g | 456.7 | 457.8 | 202.0 | 201.9 | 201.9 | 201.9 |
| Butadiene incremental /g | 152.8 | 153.1 | 478.8 | 478.8 | 478.8 | 478.8 |
| Flow rate g/min | 7.4 | 7.5 | 8.0 | 8.0 | 8.0 | 8.0 |
| Styrene total /g | 0 | 0 | 122.2 | 122.3 | 122.3 | 122.2 |
| Styrene initial /g | 0 | 0 | 122.2 | 122.3 | 122.3 | 122.2 |
| Styrene increm /g | 0 | 0 | 0 | 0 | 0 | 0 |
| Flow rate g/min | 0 | 0 | 0 | 0 | 0 | 0 |
| TMEDA /mmol | 0.18 | 0.18 | 3.08 | 3.08 | 3.08 | 3.08 |
| Initiator/ mmol | **NB** 4.77 | **NB** 4.60 | **NB** 6.67 | **NB** 6.69 | **NB** 5.82 | **NB** 7.20 |
| Final Temperature /°C | 85 | 85 | 78 | 78 | 78 | 78 |
| Modifier /mmol | **S13** 7.16 | **C2** 7.18 | **S1** 9.20 | **S10** 9.20 | **S7** 9.19 | **S9** 9.19 |
| Coupling agent/mmol | **TMOS** 0.25 | **TMOS** 0.25 | **TMOS** 0.37 | **TMOS** 0.37 | **TMOS** 0.37 | **TMOS** 0.37 |
| Chainend Mod /mmol | **DMDM** 3.04 | **DMDM** 3.05 | **DMDM** 3.68 | **DMDM** 3.68 | **DMDM** 3.68 | **DMDM** 3.68 |

| | | | | | | |
|---|---|---|---|---|---|---|
| NB = nBuLi, TMOS = Tetramethoxysilane, DMDM = Dimethoxydimethylsilane | | | | | | |

**Table 2: Polymerization 2.**

| | **Ex. F** | **CEx. F** | **Ex. G** | **Ex. H** | **REx. H** | **Ex. I** |
|---|---|---|---|---|---|---|
| Cyclohexane /g | 4377 | 4373 | 4372 | 4372 | 4368 | 4647 |
| Butadiene total /g | 693.8 | 694.0 | 693.7 | 693.8 | 696.8 | 585.3 |
| Butadiene initial /g | 201.9 | 201.9 | 201.3 | 201.3 | 202.8 | 571.8 |
| Butadiene incremental /g | 478.8 | 478.8 | 478.8 | 478.8 | 480.0 | 0 |
| Flow rate g/min | 8.0 | 8.0 | 8.0 | 8.0 | 8.0 | 0 |
| Styrene total /g | 122.2 | 122.3 | 122.2 | 122.2 | 122.8 | 155.6 |
| Styrene initial /g | 122.2 | 122.3 | 122.2 | 122.2 | 122.8 | 155.6 |
| Styrene increm /g | 0 | 0 | 0 | 0 | 0 | 0 |
| Flow rate g/min | 0 | 0 | 0 | 0 | 0 | 0 |
| TMEDA /mmol | 3.08 | 3.08 | 3.08 | 3.08 | 3.10 | 5.92 |
| Initiator/ mmol | **NB** 8.30 | **NB** 5.92 | **NB** 5.96 | **NB** 6.16 | **NB** 5.23 | **NB** 3.97 |
| Final Temp /°C | 78 | 78 | 78 | 78 | 78 | 60 |
| Modifier /mmol | **S8** 9.20 | **C1** 9.20 | **S5** 9.20 | **S2** 9.20 | **-** | **S1** 11.8 |
| Coupling agent /mmol | **TMOS** 0.37 | **TMOS** 0.37 | **TMOS** 0.37 | **TMOS** 0.37 | **TMOS** 0.40 | **DVB** 0.89 |
| Chainend Mod /mmol | **DMDM** 3.68 | **DMDM** 3.67 | **DMDM** 3.68 | **DMDM** 3.68 | **DMDM** 3.70 | **DMDM** 3.55 |

| | | | | | | |
|---|---|---|---|---|---|---|
| NB = nBuLi, TMOS = Tetramethoxysilane, DMDM=Dimethoxydimethylsilane, DVB=Divinylbenzene | | | | | | |

**Table 3: Polymerization 3.**

| | **Ex. J** | **Ex. K** | **CEx. K** | **Ex. L** | **REx. L** |
|---|---|---|---|---|---|
| Cyclohexane /g | 4646 | 4634 | 4648 | 4646 | 4648 |
| Butadiene total /g | 585.3 | 584.9 | 585.3 | 585.0 | 587.5 |
| Butadiene initial /g | 571.6 | 571.5 | 571.9 | 571.6 | 572.5 |
| Butadiene incremental /g | 0 | 0 | 0 | 0 | 0 |
| Flow rate g/min | | | | | |
| | 0 | 0 | 0 | 0 | 0 |
| Styrene total /g | 155.6 | 155.6 | 155.7 | 155.6 | 156.2 |
| Styrene initial /g | 155.6 | 155.6 | 155.7 | 155.6 | 156.2 |
| Styrene increm /g | 0 | 0 | 0 | 0 | 0 |
| Flow rate g/min | 0 | 0 | 0 | 0 | 0 |
| TMEDA /mmol | 5.92 | 5.92 | 5.93 | 5.92 | 5.95 |
| Initiator/ mmol | **NB** | **NB** | **NB** | **NB** | **NB** |
| | 4.04 | 4.12 | 3.00 | 4.07 | 3.31 |
| Final Temperature /°C | **60** | **60** | **60** | **60** | **60** |
| Modifier /mmol | **S14** 11.8 | **S13** 11.8 | **C2** 11.9 | **S13** 11.8 | - |
| Coupling agent/mmol | **DVB** 0.89 | **DVB** 0.89 | **DVB** 0.89 | **V2** 1.18 | **DVB** 0.89 |
| Chainend Mod /mmol | **DMDM** 3.55 | **DMDM** 3.55 | **DMDM** 3.56 | **DMDM** 3.55 | **DMDM** 3.57 |

| | | | | | |
|---|---|---|---|---|---|
| V2 = 1,4-bis(ethenyldimethylsilyl)piperazine | | | | | |

**Table 4: Polymerization 4.**

| | **Ex. M** | **Ex. N** | **CEx. O** | **Ex. P** | **Ex. Q** | **REx. R** |
|---|---|---|---|---|---|---|
| Cyclohexane /g | 4361 | 4374 | 4373 | 4440 | 4398 | 4396 |
| Butadiene total /g | 691.7 | 689.1 | 694.0 | 696.6 | 690.0 | 692.6 |
| Butadiene initial /g | 201.3 | 201.1 | 202.0 | 202.7 | 200.7 | 201.6 |
| Butadiene incremental /g | 478.6 | 476.8 | 478.8 | 476.2 | 476.1 | 489.4 |
| Flow rate g/min | 8.0 | 7.9 | 8.0 | 7.9 | 7.9 | 8.2 |
| Styrene total /g | 122.2 | 121.6 | 122.3 | 122.8 | 121.5 | 122.0 |
| Styrene initial /g | 122.2 | 121.6 | 122.3 | 122.8 | 121.5 | 122.0 |
| Styrene increm /g | 0 | 0 | 0 | 0 | 0 | 0 |
| Flow rate g/min | 0 | 0 | 0 | 0 | 0 | 0 |
| TMEDA /mmol | 3.08 | 3.07 | 3.08 | 3.07 | 3.06 | 4.13 |
| Initiator/ mmol | **NB** 6.00 | **NB** 6.30 | **NB** 5.31 | **LD1** 5.40 | **LD1** 5.61 | **LD2** 5.64 |
| Final Temperature /°C | **78** | **78** | **78** | **78** | **78** | **78** |
| Modifier /mmol | **S13** 9.20 | **S13** 4.58 | **C2** 9.20 | **S13** 9.15 | **S13** 9.15 | - |
| Coupling agent/mmol | **HCDS** 0.37 | **HCDS** 0.37 | **HCDS** 0.37 | **HCDS** 0.37 | **HCDS** 0.37 | **DVB** 1.38 |
| Chainend Mod /mmol | **DMDM** 3.68 | **DMDM** 3.66 | **DMDM** 3.68 | **DMDM** 3.66 | **DMDM** 3.66 | **DMDM** 11.0 |

| | | | | | | |
|---|---|---|---|---|---|---|
| LD1 = [[(dibutylamino)dimethylsilyl]methyl] lithium CAS: 1582266-07-6, LD2= [µ-[1,3-phenylenebis[1-[4-(1,1-dimethylethyl)phenyl]hexylidene]]]dilithium CAS: 10167774-63-5, HCDS = Hexachlorodisilane | | | | | | |

**Table 5: Polymerization 5.**

| | **Ex. S** | **CEx. T** | **REx. U** |
|---|---|---|---|
| Cyclohexane /g | 4396 | 4397 | 4370 |
| Butadiene total /g | 689.4 | 689.8 | 696.9 |
| Butadiene initial /g | 200.7 | 200.8 | 202.9 |
| Butadiene incremental /g | 487.1 | 487.4 | 480.8 |
| Flow rate g/min | | | |
| | 8.1 | 8.1 | 8.0 |
| Styrene total /g | 121.5 | 121.5 | 122.7 |
| Styrene initial /g | 121.5 | 121.5 | 155.7 |
| Styrene increm /g | 0 | 0 | 0 |
| Flow rate g/min | 0 | 0 | 0 |
| TMEDA /mmol | 4.11 | 4.11 | 3.09 |
| Initiator/ mmol | **LD1** 6.31 | **LD2** 5.44 | **NB** 5.30 |
| Final Temperature /°C | **78** | **78** | **78** |
| Modifier /mmol | **S13** 9.58 | **C2** 9.73 | - |
| Coupling agent/mmol | **DVB** 1.37 | **DVB** 1.37 | **TMOS** 0.37 |
| Chainend Mod /mmol | **DMDM** 11.5 | **DMDM** 11.0 | **DMDM** 3.70 |

**Table 6: Polymerization 6 (TDAE blends)**

| | **REx. V** | **Ex. W** | **Ex. X** | **Ex. Y** | **CEx. Z** |
|---|---|---|---|---|---|
| Cyclohexane /g | 4762 | 4762 | 4763 | 4765 | 4767 |
| Butadiene total /g | 403.5 | 402.2 | 403.0 | 399.6 | 399.8 |
| Butadiene initial /g | 151.8 | 151.2 | 151.2 | 150.7 | 150.7 |
| Butadiene incremental /g | 241.0 | 240.2 | 240.3 | 239.3 | 239.4 |
| Flow rate g/min | | | | | |
| | 4.8 | 4.8 | 4.8 | 4.8 | 4.8 |
| Styrene total /g | 248.4 | 247.5 | 247.6 | 245.6 | 245.8 |
| Styrene initial /g | 93.1 | 92.7 | 92.7 | 92.4 | 92.4 |
| Styrene increm /g | 151.5 | 151.0 | 151.0 | 150.5 | 150.5 |
| Flow rate g/min | 3.0 | 3.0 | 3.0 | 3.0 | 3.0 |
| DTHFP /mmol | 2.21 | 2.29 | 2.29 | 2.28 | 2.28 |
| Initiator/ mmol | **NB** 2.49 | **NB** 2.98 | **NB** 2.49 | **NB** 2.73 | **NB** 2.26 |
| Final Temperature /°C | **68** | **68** | **68** | **68** | **68** |
| Modifier /mmol | - | **S13** 5.50 | **S5** 5.50 | **S3** 5.48 | **C2** 5.48 |
| Coupling agent/mmol | **TMOS** 0.20 | **TMOS** 0.22 | **TMOS** 0.22 | **TMOS** 0.21 | **TMOS** 0.21 |
| Chainend Mod /mmol | - | - | - | - | - |

**Table 7: Polymerization 7. LP-SP Blends**

| | **CEx. SP1** | **Ex. SP2** | **REx. LP1** | **Ex. LP2** |
|---|---|---|---|---|
| Cyclohexane /g | 4581 | 4579 | 4679 | 4828 |
| Butadiene total /g | 621.3 | 621.4 | 869.6 | 643.1 |
| Butadiene initial /g | 310.1 | 310.3 | 0 | 0 |
| Butadiene incremental /g | 310.5 | 310.7 | 869.6 | 641.6 |
| Flow rate g/min | | | | |
| | 5.2 | 5.2 | 28.9 | 21.4 |
| Styrene total /g | 109.5 | 109.6 | 0 | 0 |
| Styrene initial /g | 109.5 | 109.6 | 0 | 0 |
| Styrene increm /g | 0 | 0 | 0 | 0 |
| Flow rate g/min | 0 | 0 | 0 | 0 |
| TMEDA /mmol | 1.84 | 1.83 | DTHFP:6.71 | 0 |
| Initiator/ mmol | **NB** 2.22 | **NB** 2.54 | **NB** 125 | **NB** 214 |
| Final Temperature /°C | **78** | **78** | **85** | **85** |
| Modifier /mmol | **C2** 4.61 | **S13** 4.62 | - | **S13** 171 |
| Coupling agent/mmol | **TMOS** 0.17 | **TMOS** 0.16 | - | - |
| Chainend Mod /mmol | **DMDM** 2.31 | **DMDM** 2.28 | **DMDM** 108 | **DMDM** 256 |

**Table 8: Polymerization 8. 15/30 vs 8/40 Microstructure**

| | **REx. AA** | **Ex. AB** | **Ex. AC** | **Ex. AD** | **CEx. AE** | **Ex. AF** | **REx. AG** |
|---|---|---|---|---|---|---|---|
| Cyclohexane /g | 4310 | 4374 | 4375 | 4351 | 4377 | 4374 | 4371 |
| Butadiene total /g | 696.6 | 693.4 | 692.2 | 742.6 | 688.0 | 690.3 | 696.0 |
| Butadiene initial /g | 203.7 | 202.1 | 202.1 | 261.8 | 200.6 | 201.4 | 245.7 |
| Butadiene | 492.9 | 479.7 | 479.6 | 467.3 | 487.4 | 489.0 | 437.3 |
| incremental /g | | | | | | | |
| Flow rate g/min | 8.1 | 7.8 | 7.8 | 7.8 | 8.1 | 8.1 | 7.3 |
| Styrene total /g | 124.8 | 122.2 | 122.2 | 69.0 | 124.2 | 124.7 | 123.1 |
| Styrene initial /g | 124.8 | 122.2 | 122.2 | 69.0 | 124.2 | 124.7 | 81.9 |
| Styrene increm /g | 0 | 0 | 0 | 0 | 0 | 0 | 41.2 |
| Flow rate g/min | 0 | 0 | 0 | 0 | 0 | 0 | 0.7 |
| TMEDA /mmol | 2.77 | 2.70 | 2.70 | 3.45 | 2.70 | 2.70 | 2.92 |
| Initiator/ mmol | **NB** 4.79 | **NB** 8.23 | **NB** 8.45 | **NB** 5.81 | **NB** 4.98 | **NB** 6.71 | **NB** 4.86 |
| Final Temperature /°C | **78** | **78** | **78** | **78** | **78** | **78** | **78** |
| Modifier /mmol | - | **S13 8.31** | **S13** 8.31 | **S13** | C3 | **S13** | - |
| | | | | 12.4 | 12.6 | 12.9 | |
| Coupling agent /mmol | **SnCl4** 0.36 | **TMOS** 0.25 | **TMOS** 0.25 | **TMOS** 0.25 | **DVB** 1.66 | **DVB** 1.66 | **SnCl4** 0.33 |
| Chainend Mod /mmol | **2f** 3.33 | **2f** 3.53 | **2f** 3.53 | **2f** 3.51 | **2f** 5.07 | **2f** 5.10 | **2f** 3.60 |

| | | | | | | | |
|---|---|---|---|---|---|---|---|
| 2f = 3-Methoxy-3,8,8,9,9-pentamethyl-2-oxa-7-thia-3,8-disiladecane | | | | | | | |

### Polymer characterization

In the following tables the polymer characteristics of the prepared polymers after steam stripping are shown.

**Table 9: Polymer data 1**

| | **Ex. A** | **CEx. A** | **Ex. B** | **Ex. C** | **Ex. D** | **Ex. E** |
|---|---|---|---|---|---|---|
| Staudinger Index /ml/g | 229.299 | - | 248.55 | 246.720 | 232.985 | - |
| Mooney | | | | | | |
| Viscosity ML1+4+1 /MU | 25.9 | 26.3 | 54.7 | 48.6 | 41.7 | 101.5 |
| Mooney Slope /MU/s Mp /kg/mol | -0.7668 | -0.743 | -0.7386 | -0.8968 | -0.6528 | -0.5997 |
| | 245 | 244 | 288 | 284 | 268 | 349 |
| Mn /kg/mol | 223 | 238 | 276 | 233 | 241 | 323 |
| Mw /kg/mol | 375 | 380 | 441 | 385 | 399 | 542 |
| Vinyl /% | 11.7 | 10.7 | 32.8 | 34.0 | 28.8 | 37.9 |
| Styrene /% | 0 | 0 | 14.6 | 14.7 | 15.0 | 14.4 |
| CR /% | 24.4 | 25.6 | 26.4 | 37.6 | 23.8 | 28.4 |
| Shrinking Factor | 1.182 | 1.194 | 1.128 | 0.993 | 1.164 | 1.033 |
| Tg /°C | -90.8 | -91.6 | -69.5 | -68.3 | -73.4 | -64.2 |

**Table 10. Polymer data 2**

| | **Ex. F** | **CEx. F** | **Ex. G** | **Ex. H** | **REx. H** | **Ex. I** |
|---|---|---|---|---|---|---|
| Staudinger Index /ml/g | | 230.14 | 235.83 | 229.64 | 245.88 | 258.943 |
| Mooney | | | | | | |
| Viscosity ML1+4+1 /MU | 19.8 | 41.7 | 38.1 | 35.6 | 53.0 | 90.4 |
| Mooney Slope /MU/s | -1.63 | -0.8270 | -0.7692 | -0.7377 | -0.8302 | -0.6529 |
| Mp /kg/mol | | | | | | |
| | 202 | 273 | 267 | 256 | 299 | 387 |
| Mn /kg/mol | 185 | 290 | 276 | 242 | 312 | 361 |
| Mw /kg/mol | 312 | 421 | 409 | 390 | 473 | 527 |
| Vinyl /% | 27.1 | 27.1 | 27.0 | 27.6 | 27.9 | 61.7 |
| Styrene /% | 14.6 | 14.2 | 14.3 | 14.5 | 14.7 | 20.4 |
| CR /% | 26.0 | 24.9 | 23.8 | 24.0 | 26.0 | 27.6 |
| Shrinking Factor | nd | 1.066 | 0.977 | 1.077 | 0.994 | 1.202 |
| Tg /°C | -67.7 | -72.9 | -72.6 | -72.4 | -73.8 | -24.2 |

**Table 11. Polymer data 3**

| | **Ex. J** | **Ex. K** | **CEx. K** | **Ex. L** | **REx. L** |
|---|---|---|---|---|---|
| Staudinger Index /ml/g | 244.225 | 247.71 | 296.40 | 221.95 | 239.01 |
| Mooney | | | | | |
| Viscosity ML1+4+1 /MU | 79.3 | 78.3 | 123.4 | 83.4 | 75.5 |
| Mooney Slope /MU/s | -0.7362 | -0.7948 | -0.5207 | -0.6218 | -0.6334 |
| Mp /kg/mol | | | | | |
| | 403 | 373 | 431 | 373 | 355 |
| Mn /kg/mol | 351 | 284 | 372 | 288 | 350 |
| Mw /kg/mol | 505 | 439 | 586 | 457 | 457 |
| Vinyl /% | 61.5 | 61.5 | 61.5 | 61.5 | 61.1 |
| Styrene /% | 20.6 | 20.6 | 20.5 | 20.5 | 20.7 |
| CR /% | 21.3 | 18.5 | 25.8 | 19.6 | 21.7 |
| Shrinking Factor | 1.170 | 0.963 | 1.368 | 1.272 | 1.447 |
| Tg /°C | -24.3 | -24.6 | -24.7 | -24.5 | -24.6 |

**Table 12. Polymer data 4**

| | **Ex. M** | **Ex. N** | **CEx. O** | **Ex. P** | **Ex. Q** | **REx. R** |
|---|---|---|---|---|---|---|
| Staudinger Index /ml/g | 261.57 | 300.97 | 294.11 | 348.93 | 267.39 | 219.74 |
| Mooney | | | | | | |
| Viscosity ML1+4+1 /MU | 60.6 | 79.7 | 74.8 | 130.7 | 58.8 | 33.7 |
| Mooney Slope /MU/s | -0.5889 | -0.4456 | -0.4729 | -0.4111 | -0.5231 | -0.7827 |
| Mp /kg/mol | | | | | | |
| | 276 | 300 | 285 | 370 | 260 | 303 |
| Mn /kg/mol | 262 | 282 | 309 | 287 | 254 | 246 |
| Mw /kg/mol | 553 | 685 | 642 | 752 | 554 | 335 |
| Vinyl /% | 34.4 | 31.9 | 28.1 | 38.0 | 32.9 | 28.1 |
| Styrene /% | 14.3 | 14.5 | 14.4 | 14.5 | 14.5 | 14.6 |
| CR /% | 34.6 | 51.5 | 37.2 | 39.3 | 34.7 | 17.7 |
| Shrinking Factor | nd | 0.921 | 0.992 | 0.690 | 1.048 | 1.600 |
| Tg /°C | -68.1 | -65.9 | -73.7 | -63.7 | -69.5 | -70.6 |

| | | | | | | |
|---|---|---|---|---|---|---|
| nd = not determined | | | | | | |

**Table 13. Polymer data 5**

| | **Ex. S** | **CEx. T** | **REx. U** |
|---|---|---|---|
| Staudinger Index /ml/g | 207.32 | 199.72 | 231.33 |
| Mooney | | | |
| Viscosity ML1+4+1 /MU | 31.0 | 35.9 | 40.8 |
| Mooney Slope /MU/s | -0.6366 | -0.8391 | -0.9233 |
| Mp /kg/mol | | | |
| | 293 | 308 | 267 |
| Mn /kg/mol | 188 | 235 | 279 |
| Mw /kg/mol | 308 | 334 | 432 |
| Vinyl /% | 30.8 | 28.0 | 27.0 |
| Styrene /% | 14.8 | 14.8 | 14.4 |
| CR /% | 16.5 | 16.9 | 24.6 |
| Shrinking Factor | nd | 1.431 | nd |
| Tg /°C | -67.0 | -71.7 | -74.5 |

**Table 14. Polymer data 6 (TDAE blends)**

| | **REx. V** | **Ex. W** | **Ex. X** | **Ex. Y** | **CEx. Z** |
|---|---|---|---|---|---|
| Mooney | | | | | |
| Viscosity ML1+4+1 /MU | 48.0 | 44.4 | 47.1 | 44.4 | 50.8 |
| Mooney Slope /MU/s | -0.8348 | -0.8026 | -0.8062 | -0.8751 | -0.7317 |
| Mp /kg/mol | | | | | |
| | 516 | 522 | 504 | 462 | 492 |
| Mn /kg/mol | 493 | 348 | 489 | 434 | 476 |
| Mw /kg/mol | 841 | 722 | 820 | 777 | 846 |
| Vinyl /% | 49.3 | 51.8 | 49.8 | 49.8 | 49.5 |
| Styrene /% | 37.7 | 37.5 | 37.1 | 37.9 | 38.1 |
| CR /% | 32.8 | 29.2 | 32.9 | 34.0 | 36.9 |
| Shrinking Factor | **nd** | **nd** | **nd** | **nd** | **nd** |
| Tg /°C | -25.9 | -24.5 | -25.3 | -24.8 | -25.1 |
| TDAE /phr | 37.5 | 37.5 | 37.5 | 37.5 | 37.5 |

**Table 15. Polymer data 7 LP(25 phr)/SP Blends**

| | **Blend A SP1/LP1** | **Blend B SP1/LP2** | **Blend C SP2/LP1** | **Blend D SP2/LP2M** |
|---|---|---|---|---|
| Mooney | | | | |
| Viscosity | 68.4 | 71.3 | 69.5 | 71.1 |
| ML1+4+1 /MU | | | | |
| Mooney Slope /MU/s | -0.4093 | -0.4453 | -0.5724 | -0.6037 |
| Mp /kg/mol | 613 | 634 | 593 | 588 |
| Mn /kg/mol | 75 | 94 | 77 | 90 |
| Mw /kg/mol | 777 | 833 | 701 | 714 |
| Vinyl /% | 30.9 | 32.1 | 32.2 | 33.4 |
| Styrene /% | 11.5 | 11.9 | 11.7 | 11.9 |
| CR /% | 24.2 | 24.3 | 21.5 | 21.3 |
| Shrinking Factor | 0.713 | nd | 0.668 | nd |
| Tg /°C | -64.2 | -62.6 | -63.7 | -61.3 |
| LP /phr | 25 | 25 | 25 | 25 |

**Table 16. Polymer Data 8 15/30 vs 8/40 Microstructure**

| | **REx. AA** | **Ex. AB/AC-blend*** | **Ex. AD** | **CEx. AE** | **Ex. AF** | **REx. AG** |
|---|---|---|---|---|---|---|
| Mooney Viscosity ML1+4+1 /MU | 68.3 | 56.5 | 46.3 | 68.4 | 77.9 | 53.6 |
| Mooney Slope /MU/s | -0.9011 | -0.8298 | -0.7345 | -0.6456 | -0.5913 | -0.9534 |
| Mp /kg/mol | 300 | 319 | 312 | 313 | 339 | 310 |
| Mn /kg/mol | 309 | 253 | 236 | 292 | 302 | 296 |
| Mw /kg/mol | 490 | 419 | 399 | 417 | 452 | 459 |
| Vinyl /% | 29.0 | 32.4 | 40.4 | 29.8 | 28.8 | 30.3 |
| Styrene /% | 14.7 | 14.4 | 8.6 | 14.8 | 14.8 | 15.5 |
| CR /% | 27.5 | 19.4 | 18.1 | 24.1 | 23.3 | 24.4 |
| Shrinking Factor | nd | nd | 1.83 | nd | nd | nd |
| Tg /°C | -71.8 | -68.6 | -59.0 | -72.0 | -70.2 | -59.6 |
| Staudinger Index /ml/g | | | 241.35 | 162.66 | | 249.10 |

| | | | | | | |
|---|---|---|---|---|---|---|
| *Polymer AB and AC were homogenated together and worked up. | | | | | | |

### Vulcanized polymer compositions (vulcanized rubber compounds)

After workup of the polymers, the samples were compounded in a two-step mixing process according to different mixing regimes ("recipes") and the properties of the vulcanized polymer compositions (vulcanized rubber) were determined. The corresponding mixing recipes (incl. further additives) are listed in the table below with the amounts of the formulation ingredients given in phr ("parts per hundred rubber"):

**Table 17: Formulation recipes**

| **Mixing stage** | **Formulation** | **A** | **B** | **C** | **D** | **E** | **F** | **G** |
|---|---|---|---|---|---|---|---|---|
| 1^{st} mixing step | polymer | 80 | 80 | 100 | 100 | 80 | 80 | 100 |
| | BUNA^{™} cis 132¹ | 20 | 20 | - | - | 20 | 20 | 20 |
| | Ultrasil^{®} 7000 GR² | 80 | 80 | 80 | - | 80 | 80 | 80 |
| | Si 75^{®3} | 6.9 | 6.9 | 6.9 | - | 6.9 | 6.9 | 6.9 |
| | IRBB9⁴ | - | - | - | 52.5 | - | - | - |
| | ZnO | 2.5 | 2.5 | 2.5 | 3 | 2.5 | 2.5 | 2.5 |
| | Dusantox^{®} 6PPD⁵ | 2 | 2 | 2 | - | 2 | 2 | 2 |
| | Wax Antilux^{®} 654⁶ | 1.5 | 1.5 | 1.5 | - | 1.5 | 1.5 | 1.5 |
| | Stearic Acid | 1.0 | 1.0 | 1.0 | 1.0 | 1.0 | 1.0 | 1.0 |
| | TDAE VivaTec500⁷ | 20 | 20 | 20 | 5 | 30 | 6 | - |
| 2^{nd} mixing step | sulfur | 1.4 | 1.4 | 1.4 | 1.75 | 1.4 | 1.4 | 1.4 |
| | TBBS⁸ | 1.5 | 1.5 | 1.5 | 1.05 | 1.5 | 1.7 | 1.7 |
| | DPG⁹ | 1.5 | 1.5 | 1.5 | - | 1.5 | 2 | 2 |

| | | | | | | | | |
|---|---|---|---|---|---|---|---|---|
| ¹: Trinseo Deutschland GmbH ²: Evonik Industries; BET surface area ca. 170 m²/g ³: Bis(triethoxysilylpropyl)disulfide; Evonik Industries ⁴: Industry Reference Black 9, N330 ⁵: N-1,3-dimethylbutyl-N'-phenyl-p-phenylenediamine; Duslo ⁶: Antisun and antiozonant wax; Rheinchemie (Lanxess) ⁷: Treated distilled aromatic extract; extender oil by Hansen & Rosenthal ⁸: N-tert-butyl-2-benzothiazole sulfenamide ⁹: 1,3-diphenylguanidine | | | | | | | | |

In the following tables, the performance data of the vulcanized polymer compositions ("vulcanizates") is listed. It is to be noted that vulcanized rubbers of one series can be compared only. Therefore, only entries within one table can be compared to each other except for Tables 18 and 19 which were evaluated in one run and therefore can be compared to each other.

**Table 18: Performance data 1 (Recipe A)**

| | **C1 (Polymer Ex. B)** | **C2 (Polymer Ex. C)** | **C4 (Polymer Ex. E)** |
|---|---|---|---|
| CML calc /MU | 109.4 | 138.2 | 128.5 |
| CML-ML /MU | 52.1 | 88.1 | 29.9 |
| slope | -0.248 | -0.214 | -0.231 |
| TS /MPa | 21.8 | 22.0 | 20.5 |
| EB /% | 528 | 529 | 501 |
| Mod 100 /MPa | 2.2 | 2.1 | 2.2 |
| Mod 300 /MPa | 9.1 | 8.9 | 9.0 |
| RB@0°C | 37.3 | 36.4 | 34.5 |
| RB@25°C | 54.8 | 55.9 | 54.2 |
| RB@60°C | 58 | 61.1 | 58.9 |
| HBU /°C | 144.5 | 143.1 | 137.2 |
| E' (-10°C)MPa | 14.3 | 12.5 | 14.3 |
| E' (60°C) MPa | 7.8 | 7.4 | 7.7 |
| tan d @ -10°C | 0.340 | 0.329 | 0.355 |
| tan d @ 0°C | 0.288 | 0.262 | 0.294 |
| tan d @ 60°C | 0.156 | 0.130 | 0.148 |
| Tan d @0°C/tan d @ 60°C | 1.846 | 2.015 | 1.986 |
| DIN Abrasion /mm³ | 89 | 84 | 92 |
| Bound Rubber /% | 52.8 | 60.4 | 53.8 |

**Table 19: Performance data 2 (Recipe A)**

| | **C6 (Polymer CEx. F)** | **C7 (Polymer Ex. G)** | **C8 (Polymer Ex. H)** | **C9 (Polymer REx. H)** |
|---|---|---|---|---|
| CML calc /MU | 94.0 | 163.3 | 105.5 | 106.4 |
| CML-ML /MU | 51.5 | 121.5 | 68.5 | 49.5 |
| slope | -0.239 | -0.214 | -0.251 | -0.226 |
| TS /MPa | 20.1 | 21.6 | 22.3 | 21.5 |
| EB /% | 492 | 500 | 536 | 524 |
| Mod 100 /MPa | 2.1 | 2.3 | 2.2 | 2.0 |
| Mod 300 /MPa | 9.1 | 9.7 | 9.0 | 8.8 |
| RB@0°C | 40.5 | 40.7 | 41.1 | 40.2 |
| RB@25°C | 51.9 | 52 | 52.6 | 52.4 |
| RB@60°C | 58.1 | 59.7 | 59.7 | 59.5 |
| HBU /°C | 146.9 | 140.9 | 145.8 | 143.0 |
| E' (-10°C)MPa | 11.5 | 12.4 | 13.6 | 11.2 |
| E' (60°C) MPa | 6.8 | 7.2 | 7.8 | 6.5 |
| tan d @ -10°C | 0.303 | 0.299 | 0.309 | 0.299 |
| tan d @ 0°C | 0.255 | 0.252 | 0.259 | 0.256 |
| tan d @ 60°C | 0.142 | 0.134 | 0.147 | 0.145 |
| Tan d @0°C/tan d @ 60°C | 1.796 | 1.881 | 1.762 | 1.766 |
| DIN Abrasion /mm³ | 71 | 75 | 82 | 76 |
| Bound Rubber /% | 64.4 | 67.3 | 58.1 | 64.4 |

The use of the inventive substances lead to an improved performance balance (reflected by tan d 0°C/ tan d 60°C) or to an improvement of the mechanical properties (EB and TS) while keeping the performance balance constant (C8). The inventive vinylsiloxanes can be chosen according to the respective performance priorities of the cured rubber compounds.

**Table 20: Performance data 3 CV751 p2); Recipe C**

| | **C10 (Polymer Ex. A)** | **C11 (Polymer CEx. A)** | **C12 (Ref.polymer BUNA^{™} cis 132)** |
|---|---|---|---|
| CML calc /MU | 131.4 | 170.3 | 64.3 |
| CML-ML /MU | 104.9 | 143.2 | 14.0 |
| slope | -0.208 | -0.212 | 0.207 |
| TS /MPa | 17.5 | 16.6 | 16.1 |
| EB /% | 470 | 471 | 588 |
| Mod 100 /MPa | 2.2 | 2 | 1.7 |
| Mod 300 /MPa | 8 | 7.3 | 6.4 |
| RB@0°C | 59.3 | 58.8 | 43.1 |
| RB@25°C | 64.1 | 62.8 | 46.0 |
| RB@60°C | 65.4 | 62.9 | 48.4 |
| HBU /°C | 173.2 | 174.0 | 181.4 |
| E' (-10°C)MPa | 11.7 | 11.0 | 7.0 |
| E' (60°C) MPa | 8.6 | 7.6 | 4.7 |
| tan d @ -10°C | 0.183 | 0.176 | 0.324 |
| tan d @ 0°C | 0.164 | 0.158 | 0.299 |
| tan d @ 60°C | 0.117 | 0.130 | 0.190 |
| tan d @0°C/tan d @ 60°C | 1.402 | 1.215 | 1.574 |
| DIN Abrasion /mm³ | 45 | 46 | 55 |
| Bound Rubber /% | 67.3 | 60.5 | 39.7 |

The performance balance between WG and RR of compound C10 was improved compared to comparative example C11. Furthermore, the absolute value of RR predictor tan d @60°C , RB and HBU were significantly improved compared to commercial BUNA cis 132. A higher polymer-filler interaction degree can be noted in the bound rubber value which was increased from 39.7% to 67.3%.

**Table 21: Performance data 4 (Recipe D)**

| | **C13 (Polymer Ex. G)** | **C14 (Polymer REx. H)** |
|---|---|---|
| CML calc /MU | 247.1 | 104.6 |
| CML-ML /MU | 220.6 | 77.5 |
| slope | -0.164 | -0.430 |
| TS /MPa | 16.4 | 20.1 |
| EB /% | 261 | 350 |
| Mod 100 /MPa | 3.2 | 3.2 |
| Mod 300 /MPa | - | 15.9 |
| RB@0°C | 49.4 | 46.3 |
| RB@25°C | 61.4 | 58.2 |
| RB@60°C | 68.6 | 65.1 |
| HBU /°C | 118.8 | 130.4 |
| E' (-10°C)MPa | 9.3 | 11.1 |
| E' (60°C) MPa | 6.5 | 7.1 |
| tan d @ -10°C | 0.196 | 0.237 |
| tan d @ 0°C | 0.168 | 0.210 |
| tan d @ 60°C | 0.097 | 0.133 |
| tan d @0°C/tan d @ 60°C | 1.732 | 1.579 |
| DIN Abrasion /mm³ | 92 | 98 |
| Bound Rubber /% | 57.3 | 52.1 |

Significant performance benefits of the inventive technology can also be observed with carbon black as filler. C13 exhibits better RB values, the HBU, RR predictor tan d @ 60°C as well as the performance balance are improved compared to the reference example C14.

**Table 22: Performance data 5 (Recipe B)**

| | **C15 (Polymer Ex. M)** | **C16 (Polymer Ex. N)** | **C17 (Polymer REx. U)** | **C18 (Polymer CEx. O)** |
|---|---|---|---|---|
| CML calc /MU | 156.6 | 150.5 | 93.6 | 130.1 |
| CML-ML /MU | 95.8 | 72.6 | 51.9 | 55.2 |
| slope | -0.171 | -0.165 | -0.225 | -0.173 |
| TS /MPa | 20.6 | 19.1 | 20.9 | 21.7 |
| EB /% | 473 | 442 | 517 | 512 |
| Mod 100 /MPa | 2.4 | 2.3 | 2.0 | 2.5 |
| Mod 300 /MPa | 10.3 | 10.7 | 8.7 | 10.4 |
| RB@0°C | 38.4 | 42.9 | 42.9 | 41.3 |
| RB@25°C | 51.6 | 54.8 | 53.5 | 52.5 |
| RB@60°C | 60.3 | 61.3 | 59.2 | 59.3 |
| HBU /°C | 144.0 | 147.6 | 147.4 | 151.0 |
| E' (-10°C)MPa | 14.4 | 13.2 | 11.4 | 14.6 |
| E' (60°C) MPa | 8.1 | 7.6 | 6.6 | 8.2 |
| tan d @ -10°C | 0.335 | 0.304 | 0.292 | 0.312 |
| tan d @ 0°C | 0.278 | 0.247 | 0.253 | 0.270 |
| tan d @ 60°C | 0.138 | 0.133 | 0.144 | 0.155 |
| Tan d @ 0°C/tan d @60°C | 2.014 | 1.857 | 1.757 | 1.742 |
| DIN Abrasion /mm³ | 84 | 80 | 73 | 75 |
| Bound Rubber /% | 54.6 | 58.7 | 60.4 | 52.3 |

Both vulcanizates (C15 and C16) show improved WG/RR-Performance balance compared to the reference and comparative example C17 and C18, respectively.

**Table 23: Performance data 7 (Recipe E)**

| | **C20 (Polymer Ex. S)** | **C21 (Polymer CEx. T)** | **C22 (Polymer REx. R)** |
|---|---|---|---|
| CML /MU | 96.8 | 94.6 | 118.8 |
| CML-ML /MU | 65.8 | 58.7 | 85.1 |
| slope | -0.225 | -0.243 | -0.212 |
| TS /MPa | 18.0 | 18.0 | 19.0 |
| EB /% | 450 | 521 | 524 |
| Mod 100 /MPa | 1.9 | 1.7 | 1.6 |
| Mod 300 /MPa | 9.2 | 7.4 | 7.5 |
| RB@0°C | 44.1 | 42.0 | 42.2 |
| RB@25°C | 55.9 | 53.3 | 53.4 |
| RB@60°C | 62.3 | 60.5 | 60.8 |
| HBU /°C | 141.2 | 142.6 | 140.0 |
| E' (-10°C)MPa | 8.3 | 8.6 | 7.7 |
| E' (60°C) MPa | 4.7 | 4.8 | 4.4 |
| tan d @ -10°C | 0.263 | 0.278 | 0.259 |
| tan d @ 0°C | 0.217 | 0.236 | 0.219 |
| tan d @ 60°C | 0.118 | 0.137 | 0.124 |
| Tan d @ 0°C/tan d @60°C | 1.839 | 1.723 | 1.766 |
| Bound Rubber /% | 71.0 | 63.4 | 76.3 |

Inventive Compound C20 exhibits improved WG/RR performance balance as reflected by th quotient 1.839 vs 1.723 or 1.766 of the reference and comparative examples, respectively.As the data in Table 23 shows,

**Table 24: Performance data 8 (Recipe B)**

| | **C23 (Polymer Ex. I)** | **C24 (Polymer Ex. J)** | **C25 (Polymer Ex. K)** | **C26 (Polymer CEx. K)** | **C27 (Polymer Ex. L)** | **C28 (Polymer REx. L)** |
|---|---|---|---|---|---|---|
| CML calc /MU | 109.4 | 108.2 | 112.7 | 141.1 | 101.9 | 122.9 |
| CML-ML /MU | 15.2 | 20.3 | 30.8 | 13.0 | 30.2 | 44.4 |
| slope | -0.236 | -0.240 | -0.228 | -0.200 | -0.247 | -0.215 |
| TS /MPa | 20.0 | 19.6 | 17.4 | 18.2 | 16.5 | 18.1 |
| EB /% | 415 | 417 | 385 | 385 | 371 | 407 |
| Mod 100 /MPa | 2.5 | 2.0 | 2.5 | 2.4 | 2.7 | 2.3 |
| Mod 300 /MPa | 12.4 | 11.8 | 12.2 | 12.1 | 12.6 | 11.3 |
| RB@0°C | 15.0 | 15.1 | 16.1 | 15.7 | 16.0 | 15.0 |
| RB@25°C | 35.8 | 35.9 | 40.1 | 35.5 | 41.3 | 34.4 |
| RB@60°C | 59.9 | 59.1 | 64.0 | 60.1 | 65.1 | 59.6 |
| HBU /°C | 125.1 | 122.8 | 122.4 | 119.1 | 120.8 | 124.9 |
| E' (-10°C)MPa | 17.6 | 15.8 | 14.7 | 17.6 | 14.7 | 15.5 |
| E' (60°C) MPa | 5.6 | 5.0 | 4.9 | 6.0 | 4.8 | 5.1 |
| tan d @ -10°C | 0.651 | 0.642 | 0.657 | 0.637 | 0.667 | 0.622 |
| tan d @ 0°C | 0.416 | 0.423 | 0.386 | 0.431 | 0.363 | 0.458 |
| tan d @ 60°C | 0.127 | 0.135 | 0.098 | 0.127 | 0.091 | 0.131 |
| Tan d @ 0°C/tan d @60°C | 3.276 | 3.133 | 3.939 | 3.394 | 3.989 | 3.496 |
| DIN Abrasion /mm³ | 132 | 131 | 124 | 134 | 121 | 130 |
| Bound Rubber /% | 79.1 | 74.9 | 73.8 | 79.1 | 72.4 | 78.3 |

Depending on the choice of vinyl disiloxane either the RR-indicators, the WG/RR balance or the mechanical properties reflected by EB and TS can be improved vs reference or comparative example. C25 and C27 show improved RR and WG/RR balance whereas C23 and C24 show an improved level of mechanical properties while keeping other characteristics constant.

**Table 25: Performance data 9 (Recipe F)**

| | **C29 (Polymer REx. V)** | **C30 (Polymer Ex. W)** | **C32 (Polymer Ex. Y)** | **C33 (Polymer CEx. Z)** |
|---|---|---|---|---|
| CML calc /MU | 81.4 | 84.2 | 77.0 | 83.2 |
| CML-ML /MU | 33.0 | 40.1 | 31.5 | 33.2 |
| slope | -0.312 | -0.275 | -0.303 | -0.295 |
| TS /MPa | 21.0 | 22.2 | 21.3 | 20.8 |
| EB /% | 486 | 509 | 501 | 475 |
| Mod 100 /MPa | 2.4 | 2.2 | 2.4 | 2.6 |
| Mod 300 /MPa | 11.1 | 11.0 | 10.8 | 11.5 |
| RB@0°C | 8.4 | 7.7 | 7.7 | 8.4 |
| RB@25°C | 24.0 | 23.8 | 23.0 | 23.6 |
| RB@60°C | 53.0 | 55.9 | 52.1 | 53.0 |
| HBU /°C | 118.9 | 116.8 | 121.6 | 118.6 |
| E' (-10°C)MPa | 29.7 | 41.9 | 37.6 | 42.4 |
| E' (60°C) MPa | 5.0 | 5.5 | 5.5 | 5.9 |
| tan d @ -10°C | 0.899 | 0.915 | 0.886 | 0.899 |
| tan d @ 0°C | 0.620 | 0.666 | 0.621 | 0.634 |
| tan d @ 60°C | 0.141 | 0.141 | 0.156 | 0.152 |
| Tan d @ 0°C/tan d @60°C | 4.397 | 4.723 | 3.981 | 4.171 |
| DIN Abrasion /mm³ | - | 116 | 140 | 140 |
| Bound Rubber /% | 52.5 | 55.1 | 45.5 | 51.8 |

C30 exhibits improved WG/RR balance compared to references.

**Table 26: Performance data 10 (Recipe G)**

| | **C34 Comp** | **C36** |
|---|---|---|
| | **(Blend A)** | **(Blend C)** |
| CML calc /MU | 167.8 | 166.3 |
| CML-ML /MU | 98.2 | 96.4 |
| slope | -0.195 | -0.176 |
| TS /MPa | 22.0 | 19.8 |
| EB /% | 444 | 413 |
| Mod 100 /MPa | 2.6 | 2.5 |
| Mod 300 /MPa | 11.5 | 11.0 |
| RB@0°C | 43.3 | 44.1 |
| RB@25°C | 51.2 | 52.1 |
| RB@60°C | 57.5 | 58.6 |
| HBU /°C | 138.2 | 135.6 |
| E' (-10°C)MPa | 13.1 | 12.2 |
| E' (60°C) MPa | 8.0 | 7.6 |
| tan d @ -10°C | 0.269 | 0.266 |
| tan d @ 0°C | 0.247 | 0.242 |
| tan d @ 60°C | 0.138 | 0.126 |
| Tan d @ 0°C/tan d @60°C | 1.790 | 1.920 |

C36 including the inventively modified polymer and a DMDM modified low molecular polymer component show significant improvements compared to blend C (C36) with a state-of-the art modified polymer and the same DMDM-modified low molecular polymer component. RR predictor tan d @ 60°C as well as the WG/RR balance were both improved. Other characteristics (RB, HBU) support this trend.

**Table 27: 15/30 vs 8/40 Microstructure (Recipe A)**

| | **C38 (Polymer REx. AA)** | **C39 (Polymer Ex. AB/AC-blend)** | **C40 (Polymer Ex. AD)** | **C41 (Polymer REx. AA)** | **C42 (Polymer CEx. AE)** | **C43 (Polymer Ex. AF)** | **C44 (Polymer Ex. AG)** |
|---|---|---|---|---|---|---|---|
| CML calc /MU | | | | 114.3 | 163.4 | 186.3 | 110.3 |
| CML-ML /MU | | | | 44.3 | 92.8 | 106.5 | 40.7 |
| slope | | | | -0.267 | -0.141 | -0.078 | -0.285 |
| TS /MPa | | | | 22.4 | 21.5 | 22.1 | 22.0 |
| EB /% | 1.8 | 2.1 | 2.2 | 529 | 433 | 420 | 527 |
| Mod 100 /MPa | | | | 2.1 | 2.6 | 2.5 | 1.9 |
| Mod 300 /MPa | 7.8 | 9.7 | 9.8 | 8.9 | 12.2 | 12.7 | 8.4 |
| RB@0°C | 43.8 | 42.8 | 43.5 | 42.8 | 41.5 | 42.4 | 41.3 |
| RB@25°C | 54.1 | 55.4 | 55.1 | 54.9 | 55.9 | 55.6 | 53.5 |
| RB@60°C | 61.4 | 64.1 | 63.9 | 61.1 | 64.0 | 64.4 | 60.5 |
| HBU /°C | 142.2 | 130.2 | 130.5 | 138.1 | 133.6 | 129.3 | 139.2 |
| E' (-10°C)MPa | 10.1 | 10.7 | 11.0 | 13.0 | 13.8 | 11.6 | 9.8 |
| E' (60°C) MPa | 5.8 | 6.9 | 6.8 | 7.7 | 8.3 | 7.5 | 5.7 |
| tan d @ -10°C | 0.288 | 0.292 | 0.285 | 0.282 | 0.295 | 0.285 | 0.283 |
| tan d @ 0°C | 0.247 | 0.234 | 0.234 | 0.242 | 0.244 | 0.234 | 0.230 |
| tan d @ 60°C | 0.142 | 0.112 | 0.112 | 0.132 | 0.113 | 0.105 | 0.127 |
| Tan d @ 0°C/tan d | 1.739 | 2.089 | 2.089 | 1.833 | 2.159 | 2.229 | 1.811 |
| @60°C | | | | | | | |
| DIN Abrasion /mm³ | 85 | 93 | 97 | 94 | 85 | 76 | 83 |
| Bound Rubber /% | 60.4 | 64.5 | 57.0 | 60.6 | 63.4 | 68.0 | 61.1 |

The first part in table 27 (C38-40) shows the influence of a different microstructure with same Tg on the performance of the inventive technology. C39 (15 ST/30 V) and C40 (8 ST / 40 V) have the same impressive performance improvement of RR and WG/RR balance compared to the reference C38. The inventive modifiers can be applied independently of the chosen microstructure.

Inventive C43 is the best cured rubber compound in the series of C41-C44. C42 was prepared by applying the closest state of the art (vinyl silane with aminoalkyl groups w/o disiloxane unit). C42 shows improved performance vs reference compounds but inferior performance compared to inventive example C43.

As demonstrated above, the modifying monomer in accordance with the invention allows for the preparation of vulcanized polymer compositions having superior properties, and in particular an improved performance balance which is represented by a balance of rolling resistance and wet grip performance paired with acceptable or even improved processing and mechanical properties, such as DIN abrasion (abrasion resistance) and handling characteristics.

### Test Methods

The molecular weight analyses were carried out via size exclusion chromatography SEC/RI using a HEWLETT PACKARD HP 1100. The eluent THF was degassed on-line. The solvent flow rate was 1.0 ml/min. 100 µL of polymer solution were injected per analysis. The analyses were carried out at 40°C. The molecular weights were initially calculated based on a polystyrene calibration and given in the tables as polystyrene. The real molecular weights (SSBR molecular weights) were determined by division by a factor derived from an earlier comparison between molecular weights from SEC/RI and SEC/MALLS. The value of the factor depends on the polymer composition (styrene and butadiene content). A factor of 1.52 was used for SSBR with 21% and 25% styrene. Mp (as SSBR) was used for the calculation of TMEDA molar ratios.

Gas chromatography (GC) was used to monitor the polymerization reactions and the conversion of the monomers.

NMR-spectroscopy was performed on a BRUKER Avance 400 in a 5 mm BBO probe.

FTIR-spectroscopy measured in attenuated total reflection was used to determine the vinyl content and styrene content.

The glass transition temperature T_{g} was determined using the DSC Q2000 under the following conditions:

| | |
|---|---|
| Weight | : ca. 10 - 12 mg |
| Sample container | : Alu/S |
| Temperature range | : (-140...80) °C |
| Heating rate | : 20 K/min or 5 K/min |
| Cooling rate | : free cooling |
| Purge gas | : 20 ml Ar/min |
| Cooling agent | : liquid nitrogen |

Each sample was measured at least once. The measurements contain two heating runs. The 2nd heating run was used to determine the glass transition temperature.

Measurements of non-vulcanized rheological properties according to ASTM D 5289-95 were made using a rotorless shear rheometer (MDR 2000 E) to characterize cure characteristics. Test pieces were vulcanized to t₉₅ at 160°C. For rebound resilience (RB) tests the specimen were vulcanized to t₉₅₊₅ at 160°C. Tensile strength (TS) and moduli were measured according to ASTM D 412 on a Zwick Z010. DIN abrasion was measured according to DIN 53516 (1987-06-01). Rebound resilience (ISO 4662) was measured at 0°C, RT (20°C) and 60°. Dynamic properties in terms of tan δ and E' at specified temperatures were measured using a dynamic spectrometer Eplexor 150N/500N manufactured by Gabo Qualimeter Testanlagen GmbH (Germany) applying a compression dynamic strain of 1 % at a frequency of 2 Hz in strain measuring mode.

### Shrinking Factor:

The shrinking factor is obtained analogous to a method established by Asahi (EP 3 536 720 A1). The shrinking factor is obtained by measuring an elugram using a gel chromatography with a triple detector system with a light-scattering, viscometer and RI detector. Particularly, using a GPC-light scattering measurement apparatus provided with a light scattering detector in which one mixed bed column filled with a polystyrene-based gel as a filler are connected and a viscometer, an absolute molecular weight and intrinsic viscosity corresponding to each absolute molecular weight are obtained, intrinsic viscosity of a linear polymer corresponding to the absolute molecular weight is calculated, and a shrinking factor is obtained as a ratio of the intrinsic viscosity corresponding to each absolute molecular weight. Also, the RI signal was detected as a concentration specific value by the RI area. For example, the shrinking factor was obtained as follows. 50 µl of a THF soluted sample was injected to a GPC-Triple detector system (Viscotek TDAmax, Malvern Co.) provided with a light scattering detector, a viscometer and an RI-detector. An absolute molecular weight was obtained from the light scattering detector, intrinsic viscosity [µ] with respect to the absolute molecular weight per peak was obtained from the light scattering detector and the viscometer and intrinsic viscosity [µ]0 of a linear polymer with respect to the absolute molecular weight M was calculated by Formula 1: ${\left[μ\right]}_{0} = {10}^{- 3.883} {M}^{0.771}$

The RI area was obtained via the RI detector.

The Shrinking Factor SF (g') is an average value of the ratio of intrinsic viscosity ${SF}_{x} = \frac{μ}{{μ}_{0}}$ with SFx representing the shrinking factor over all peaks in the elugram. Measurements were performed using THF as eluent and Mixed B (Agilent Co.) as column at 40°C and with a flow rate of 0.5 ml/min.

A specimen was prepared by dissolving 10 mg of a polymer in 10 ml of THF.

The procedure for determination of the Staudinger Index (Intrinsic Viscosity) is as follows:
1. Preparation of 4 different concentrations of synthetic rubber in toluene
   a. The concentrations are as follows:
      i. 0.7 g/dL
      ii. 0.6 g/dL
      iii. 0.5 g/dL
      iv. 0.4 g/dL
   b. An Erlenmeyer Flask is placed on an analytical balance (accuracy <0.1 mg) whereas the weight is zeroed
   c. The polymer is crumbled in particles, filled Erlenmeyer flasks and weight is noted
      i. ~ 0.28 g
      ii. ~ 0.24 g
      iii. ~ 0.20 g
      iv. ~ 0.16 g
   d. ~ 40 ml Toluene is added to the polymer crumbles and the resulting weight is noted
   e. The Erlenmeyer flasks are closed and placed on a shaker for ~ 24h
2. Measurement of dynamic viscosity of polymer solutions in a capillary viscosimeter (Rheotest LK 2.2)
   a. Transfer of polymer solution into measurement vessels → check for complete solubility, non-soluble fractions of polymer void measurement!
   b. Measurement at 25°C
   c. 10 measurement runs
   d. Mean value of last 5 runs is taken for evaluation
3. Calculation of reduced viscosity or "Staudinger Function" according to formula 1.1. $\frac{1}{c} ∗ \frac{η - {η}_{s}}{{η}_{s}}$
   ηₛ ... Viscosity of solvent
   η ... viscosity of solution
   c ... concentration
4. Plotting of Staudinger Function in a graph with Staudinger Function on y-axis and concentration on x-axis
5. Calculate a linear regression through the plot and extrapolate to c=0
6. Staudinger function/reduced viscosity at c=0 is called the "Staudinger Index" or "Intrinsic Viscosity".

## Claims

1. A vinyldisiloxane compound of the following Formula 2: 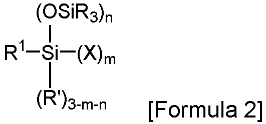 wherein
R and R' are independently selected from C₁₋₁₈ hydrocarbyl;
R¹ is optionally substituted vinyl, the one or more optional substituents being selected from methyl, ethyl and vinyl;
X is independently selected from one of the following moieties (**X-1** to **X-5**): 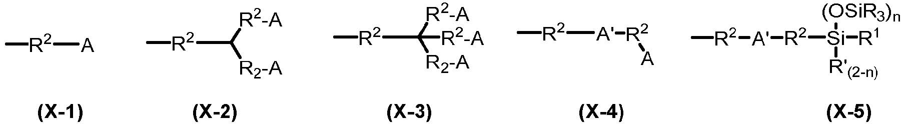
preferably X is (**X-1**),
R² is a C₁₋₁₈ hydrocarbylene;
A is NR³R⁴,
R³ and R⁴ being independently selected from linear, branched and cyclic C₁₋₈ alkyl, C₆₋₁₂ aryl, C₇-₁₅ aralkyl, SiR"₃ or linear, branched and cyclic C₂₋₈ alkyl-O-SiR"₃, wherein each R" is independently selected from C₁₋₆ alkyl, C₆₋₁₂ aryl and C₇₋₁₈ alkylaryl,
and R³ may be connected with R² or R⁴ to form a ring and the ring may contain, further to the R²-bonded nitrogen atom, one or more selected from an oxygen atom, a nitrogen atom and a sulfur atom, with the proviso that, in case R³ and R⁴ are both SiR"₃, two R" may be connected with each other to form a ring together with the Si-atoms to which they are bonded;
A' is NR⁵ with R⁵ being selected from linear, branched and cyclic C₁₋₈ alkylene, C₆₋₁₂ arylene, C₇-C₁₅ aralkylene, SiR"₃ or R²A; or A' is a cyclic diaminediyl group such as piperazinyl;
n is 1 or 2;
m is 1 or 2, and
m + n is 2 or 3.

2. The compound according to claim 1,
wherein R² is linear or branched C₁₋₈ alkylene, C₆₋₁₂ arylene or C₇-C₁₅ aralkylene; and/or
wherein R and R' are independently selected from methyl, ethyl, n-propyl, i-propyl, t-butyl, phenyl and benzyl; and/or
wherein R¹ is vinyl or 1,3-butadienyl, preferably vinyl.

3. The compound according to claim 1 or 2,
wherein A is represented by one of the following moieties: 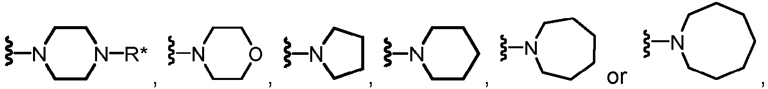
preferably 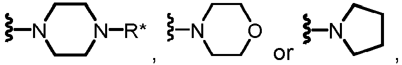
R* being selected from C₁₋₆ alkyl, C₆₋₁₂ aryl and C₇₋₁₈ alkylaryl, SiR"₃ or linear, branched and cyclic C₂₋₈ alkyl-O-SiR"₃;
more preferably A is represented by 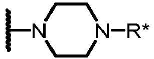
and R* is methyl, ethyl, butyl, phenyl, benzyl or SiR"₃ or C₂₋₈ alkyl-O-SiR"₃, and R" is independently selected from methyl, ethyl, n-propyl, i-propyl, t-butyl, phenyl and benzyl.

4. The compound according to claim 1 or 2,
wherein R³ and R⁴ are independently selected from methyl, ethyl, phenyl and benzyl.

5. The compound according to any of claims 1-4,
wherein R² independently is phenylene or linear C₁₋₈ alkylene, preferably linear C₃-₆ alkylene, more preferably C₃ alkylene; and/or
wherein R is (methyl, methyl, methyl), (ethyl, ethyl, ethyl), (i-propyl, i-propyl, i-propyl), (phenyl, phenyl, phenyl), (methyl, methyl, t-butyl), (methyl, methyl, i-propyl), (ethyl, ethyl, i-propyl) or (phenyl, phenyl, t-butyl), preferably (ethyl, ethyl, ethyl), (i-propyl, i-propyl, i-propyl) or (methyl, methyl, t-butyl); and/or
wherein
n is 1 and m is 2,
n is 2 and m is 1, or
n is 1 and m is 1,
preferably n is 1 and m is 2 or 1.

6. A process for preparing an elastomeric polymer,
wherein the process comprises the polymerization of at least one conjugated diene monomer and optionally one or more aromatic vinyl monomers in the presence of one or more initiator compounds and one or more compounds as defined in any of claims 1-5.

7. The process according to claim 6,
wherein the diene monomer is 1,3-butadiene and the aromatic vinyl monomer is styrene and/or
wherein the polymerization is a solution polymerization.

8. An elastomeric polymer obtainable by the process of claim 6 or 7.

9. The elastomeric polymer according to claim 8 which comprises the following repeating unit:

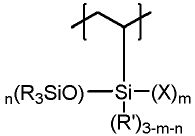

10. A non-vulcanized polymer composition comprising the elastomeric polymer as defined in claim 8 or 9 and one or more additives which are preferably selected from extender oils, stabilizers and further polymers.

11. The polymer composition according to claim 10, further comprising one or more fillers which are preferably selected from carbon black, carbon nanotubes, graphite, graphene, silica, carbon-silica dual-phase filler, clays calcium carbonate, magnesium carbonate, lignin, glass particle-based fillers and starch-based fillers, preferably the filler is silica.

12. The polymer composition according to claim 10 or 11, further comprising one or more vulcanizing agents.

13. A vulcanized polymer composition obtainable by vulcanizing the polymer composition as defined in claim 12.

14. A method of making a vulcanized polymer composition, wherein the method comprises the step of vulcanizing the polymer composition as defined in claim 12.

15. An article comprising at least one component formed from the vulcanized polymer composition as defined in claim 13 which is preferably a tire, a tire tread, a tire side wall, a tire carcass, a belt, a gasket, a seal, a hose, a vibration damper, a footwear component, a golf ball or a hose, more preferably a tire.

## Patentansprüche

1. Vinyldisiloxan-Verbindung der folgenden Formel 2: 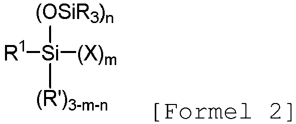 worin
R und R' unabhängig ausgewählt sind aus C₁₋₁₈-Hydrocarbyl;
R¹ gegebenenfalls substituiertes Vinyl ist, wobei die ein oder mehreren Substituenten ausgewählt sind aus Methyl, Ethyl und Vinyl;
X unabhängig ausgewählt ist aus einer der folgenden Einheiten (**X-1** bis **X-5**): 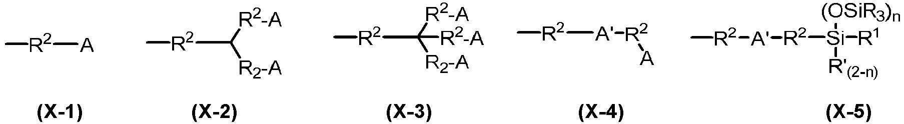
vorzugsweise ist X (**X-1**),
R² ein C₁₋₁₈-Hydrocarbylen ist;
A NR³R⁴ ist,
R³ und R⁴ unabhängig ausgewählt sind aus linearem, verzweigtem und cyclischem C₁₋₈-Alkyl, C₆₋₁₂-Aryl, C₇₋₁₅-Aralkyl, SiR''₃ oder linearem, verzweigtem und cyclischem C₂₋₈-Alkyl-O-SiR''₃, worin jedes R'' unabhängig ausgewählt ist aus C₁₋₆-Alkyl, C₆₋₁₂-Aryl und C-₇₋₁₈-Alkylaryl,
und R³ mit R² oder R⁴ verbunden sein kann, so dass ein Ring gebildet wird, und der Ring kann ferner zum R²-gebundenen Stickstoffatom eines oder mehrere enthalten, ausgewählt aus einem Sauerstoffatom, einem Stickstoffatom und einem Schwefelatom, mit der Maßgabe, dass, wenn R³ und R⁴ beides SiR''₃ sind, zwei R'' miteinander verbunden sein können, so dass sie zusammen mit den Si-Atomen, an die sie gebunden sind, einen Ring bilden;
A' NR⁵ ist, wobei R⁵ ausgewählt ist aus linearem, verzweigtem und cyclischem C₁₋₈₋Alkylen, C₆₋₁₂-Arylen, C₇₋C₁₅-Aralkylene, SiR''₃ oder R²A; oder A' eine cyclische Diamindiylgruppe wie Piperazinyl ist;
n 1 oder 2 ist;
m 1 oder 2 ist und
m + n 2 oder 3 ist.

2. Verbindung gemäß Anspruch 1,
worin R² lineares oder verzweigtes C₁₋₈-Alkylene, C₆₋₁₂-Arylene oder C₇-C₁₅-Aralkylen ist; und/oder
worin R und R' jeweils unabhängig ausgewählt sind aus Methyl, Ethyl, n-Propyl, i-Propyl, t-Butyl, Phenyl und Benzyl; und/oder
worin R¹ Vinyl oder 1,3-Butadienyl, vorzugsweise Vinyl, ist.

3. Verbindung gemäß Anspruch 1 oder 2,
worin A durch eine der folgenden Einheiten dargestellt ist: 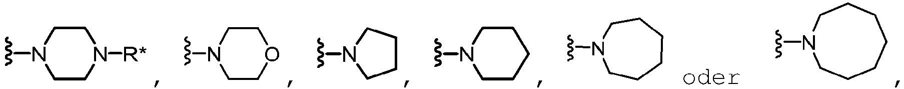
vorzugsweise 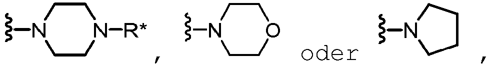
wobei R* ausgewählt ist aus C₁₋₆-Alkyl, C₆₋₁₂-Aryl und C₇₋₁₈-Alkylaryl, SiR''₃ oder linearem, verzweigtem und cyclischem C₂₋₈-Alkyl-O-SiR"₃;
bevorzugter ist A durch 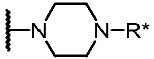
dargestellt und R* ist Methyl, Ethyl, Butyl, Phenyl, Benzyl oder SiR"₃ oder C₂₋₈-Alkyl-O-SiR''₃, und R'' ist unabhängig ausgewählt aus Methyl, Ethyl, n-Propyl, i-Propyl, t-Butyl, Phenyl und Benzyl.

4. Verbindung gemäß Anspruch 1 oder 2,
worin R³ und R⁴ unabhängig ausgewählt sind aus Methyl, Ethyl, Phenyl und Benzyl.

5. Verbindung gemäß einem der Ansprüche 1-4,
worin R² unabhängig Phenylen oder lineares C₁₋₈-Alkylen ist, vorzugsweise lineares C₃₋₆-Alkylen, bevorzugter C₃-Alkylen; und/oder
worin R (Methyl, Methyl, Methyl), (Ethyl, Ethyl, Ethyl), (i-Propyl, i-Propyl, i-Propyl), (Phenyl, Phenyl, Phenyl), (Methyl, Methyl, t-Butyl), (Methyl, Methyl, i-Propyl), (Ethyl, Ethyl, i-Propyl) oder (Phenyl, Phenyl, t-Butyl), vorzugsweise (Ethyl, Ethyl, Ethyl), (i-Propyl, i-Propyl, i-Propyl) oder (Methyl, Methyl, t-Butyl), ist; und/oder
worin
n 1 ist und m 2 ist,
n 2 ist und m 1 ist oder
n 1 ist und m 1 ist,
vorzugsweise ist n 1 und m ist 2 oder 1.

6. Verfahren zur Herstelleng eines Elastomerpolymers, worin das Verfahren die Polymerisation mindestens eines konjugierten Dienmonomers und gegebenenfalls einer oder mehrerer aromatischen Vinylmonomeren in Gegenwart einer oder mehrerer Initiatorverbindungen und einer oder mehrerer wie in einem der Ansprüche 1-5 definierten Verbindungen umfasst.

7. Verfahren gemäß Anspruch 6,
worin das Dienmonomer 1,3-Butadien und das aromatische Vinylmonomer Styrol ist und/oder
worin die Polymerisation eine Lösungspolymerisation ist.

8. Elastomerpolymer, erhältlich durch das Verfahren gemäß Anspruch 6 oder 7.

9. Elastomerpolymer gemäß Anspruch 8, das die folgende Wiederholungseinheit umfasst:

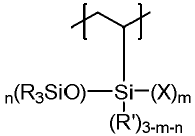

10. Unvulkanisierte Polymerzusammensetzung, die das wie in Anspruch 8 oder 9 definierte Elastomerpolymer und ein oder mehrere Additive, die vorzugsweise aus Streckölen, Stabilisatoren und weiteren Polymeren ausgewählt sind, umfasst.

11. Polymerzusammensetzung gemäß Anspruch 10, ferner umfassend einen oder mehrere Füllstoffe, die vorzugsweise ausgewählt sind aus Ruß, Kohlenstoffnanoröhren, Graphit, Graphen, Siliciumdioxid, Kohlenstoff-Siliciumdioxid-Zweiphasenfüllstoffen, Tonen, Calciumcarbonat, Magnesiumcarbonat, Lignin, auf Glaspartikeln basierenden Füllstoffen und auf Stärke basierenden Füllstoffen, vorzugsweise ist der Füllstoff Siliciumdioxid.

12. Polymerzusammensetzung gemäß Anspruch 10 oder 11, ferner umfassend ein oder mehrere Vulkanisierungsmittel.

13. Vulkanisierte Polymerzusammensetzung, erhältlich durch Vulkanisieren der wie in Anspruch 12 definierten Polymerzusammensetzung.

14. Verfahren zur Herstellung einer vulkanisierten Polymerzusammensetzung, worin das Verfahren den Schritt des Vulkanisierens der wie in Anspruch 12 definierten Polymerzusammensetzung umfasst.

15. Gegenstand, umfassend mindestens einen aus der wie in Anspruch 13 definierten vulkanisierten Polymerzusammensetzung gebildeten Bestandteil, der vorzugsweise ein Reifen, eine Reifenlauffläche, eine Reifenflanke, eine Reifenkarkasse, ein Gürtel, eine Dichtung, eine Abdichtung, ein Schlauch, ein Schwingungsdämpfer, eine Schuhkomponente, ein Golfball oder ein Schlauch, bevorzugter ein Reifen, ist.

## Revendications

1. Composé vinyldisiloxane de la Formule 2 suivante : 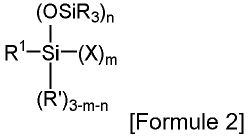 dans laquelle
R et R' sont indépendamment sélectionnés parmi un hydrocarbyle en C₁₋₁₈ ;
R¹ est un vinyle facultativement substitué, les un ou plusieurs substituants facultatifs étant sélectionnés parmi méthyl, éthyl et vinyle ;
X est indépendamment sélectionné parmi l'un des motifs suivants **(X-1** à **X-5)** : 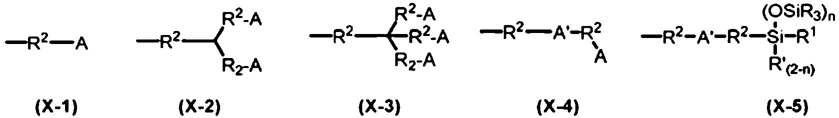
de préférence X est **(X-1),**
R² est un hydrocarbylène en C₁₋₁₈ ;
A est NR³R⁴,
R³ et R⁴ étant indépendamment sélectionnés parmi un alkyle en C₁₋₈ linéaire, ramifié et cyclique, un aryle en C₆₋₁₂, un aralkyle en C₇₋₁₅, SiR"₃ ou un alkyle-O-SiR"₃ en C₂₋₈ linéaire, ramifié et cyclique, dans lequel chaque R" est indépendamment sélectionné parmi un alkyle en C₁₋₆, un aryle en C₆₋₁₂ et un alkylaryle en C₇₋₁₈,
et R³ peut être relié à R² ou R⁴ pour former un cycle et le cycle peut contenir, en plus de l'atome d'azote lié à R², un ou plusieurs atomes sélectionnés parmi un atome d'oxygène, un atome d'azote et un atome de soufre, avec la réserve que, dans le cas où R³ et R⁴ sont à la fois SiR"3, deux R" peuvent être reliés l'un à l'autre pour former un cycle avec les atomes de Si auxquels ils sont liés ;
A' est NR⁵ avec R⁵ étant sélectionné parmi un alkylène en C₁₋₈ linéaire, ramifié et cyclique, un arylène en C₆₋₁₂, un aralkylène en C₇-C₁₅, SiR"₃ ou R²A ; ou A' est un groupe diaminediyl cyclique tel que pipérazinyle ;
n est 1 ou 2 ;
m est 1 ou 2, et
m + n est 2 ou 3.

2. Composé selon la revendication 1,
dans lequel R² est un alkylène en C₁₋₈ linéaire ou ramifié, un arylène en C₆₋₁₂ ou un aralkylène en C₇-C₁₅ ; et/ou
dans lequel R et R' sont indépendamment sélectionnés parmi méthyl, éthyl, n-propyl, i-propyl, t-butyl, phényl et benzyl ; et/ou
dans lequel R¹ est vinyle ou 1,3-butadiényl, de préférence vinyle.

3. Composé selon la revendication 1 ou la revendication 2,
dans lequel A est représenté par l'un des motifs suivants : 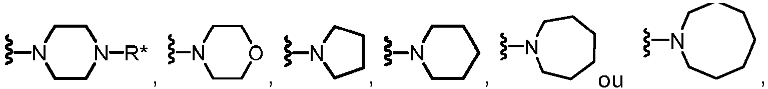
de préférence 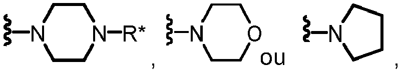 R* étant sélectionné parmi un alkyle en C₁₋₆, un aryle en C₆₋₁₂ et un alkylaryle en C₇₋₁₈, SiR"₃ ou un alkyle-O-SiR"₃ en C₂₋₈ linéaire, ramifié et cyclique ;
plus préférablement A est représenté par 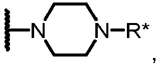
et R* est méthyl, éthyl, butyl, phényle, benzyle ou SiR"₃ ou un alkyle-O-SiR"₃ en C₂₋₈, et R" est indépendamment sélectionné parmi méthyl, éthyl, n-propyl, i-propyl, t-butyl, phényle et benzyle.

4. Composé selon la revendication 1 ou la revendication 2,
dans lequel R³ et R⁴ sont indépendamment sélectionnés parmi méthyl, éthyl, phényl et benzyl.

5. Composé selon l'une quelconque des revendications 1 à 4,
dans lequel R² est indépendamment un phénylène ou un alkylène en C₁₋₈ linéaire, de préférence un alkylène en C₃₋₆ linéaire, plus préférablement un alkylène en C₃ ; et/ou dans lequel R est (méthyl, méthyl, méthyl), (éthyl, éthyl, éthyl), (i-propyl, i-propyl, i-propyl), (phényl, phényl, phényl), (méthyl, méthyl, t-butyl), (méthyl, méthyl, i-propyl), (éthyl, éthyl, i-propyl) ou (phényl, phényl, t-butyl), de préférence (éthyl, éthyl, éthyl), (i-propyl, i-propyl, i-propyl) ou (méthyl, méthyl, t-butyl) ; et/ou
dans lequel
n est 1 et m est 2,
n est 2 et m est 1, ou
n est 1 et m est 1,
de préférence n est 1 et m est 2 ou 1.

6. Procédé de préparation d'un polymère élastomère,
dans lequel le procédé comprend la polymérisation d'au moins un monomère diénique conjugué et facultativement un ou plusieurs monomères vinylaromatiques en présence d'un ou plusieurs composés initiateurs et d'un ou plusieurs composés tels que définis dans l'une quelconque des revendications 1 à 5.

7. Procédé selon la revendication 6,
dans lequel le monomère diénique est le 1,3-butadiène et le monomère vinylaromatique est le styrène et/ou
dans lequel la polymérisation est une polymérisation en solution.

8. Polymère élastomère pouvant être obtenu par le procédé de la revendication 6 ou de la revendication 7.

9. Polymère élastomère selon la revendication 8 qui comprend l'unité répétitive suivante :

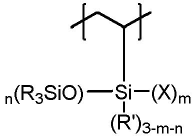

10. Composition polymère non vulcanisée comprenant le polymère élastomère tel que défini dans la revendication 8 ou la revendication 9 et un ou plusieurs additifs qui sont de préférence sélectionnés parmi des huiles d'extension, des stabilisants et des polymères supplémentaires.

11. Composition polymère selon la revendication 10, comprenant en outre une ou plusieurs charges qui sont de préférence sélectionnées parmi du noir de carbone, des nanotubes de carbone, du graphite, du graphène, de la silice, une charge biphasée carbone-silice, des argiles, du carbonate de calcium, du carbonate de magnésium, de la lignine, des charges à base de particules de verre et des charges à base d'amidon, de préférence la charge est de la silice.

12. Composition polymère selon la revendication 10 ou la revendication 11, comprenant en outre un ou plusieurs agents de vulcanisation.

13. Composition polymère vulcanisée pouvant être obtenue par vulcanisation de la composition polymère telle que définie dans la revendication 12.

14. Procédé de fabrication d'une composition polymère vulcanisée, dans lequel le procédé comprend l'étape de vulcanisation de la composition polymère telle que définie dans la revendication 12.

15. Article comprenant au moins un composant formé à partir de la composition polymère vulcanisée telle que définie dans la revendication 13 qui est de préférence un pneu, une bande de roulement de pneu, un flanc de pneu, une carcasse de pneu, une courroie, un joint d'étanchéité, un joint, un tuyau, un amortisseur de vibrations, un composant de chaussure, une balle de golf ou un tuyau, plus préférablement un pneu.
